(19) Europäisches Patentamt
European Patent Office
Office européen des brevets

(11) EP 0 515 240 B1

(12) # FASCICULE DE BREVET EUROPEEN

(45) Date de publication et mention de la délivrance du brevet:
**24.09.1997 Bulletin 1997/39**

(21) Numéro de dépôt: **92401237.0**

(22) Date de dépôt: **30.04.1992**

(51) Int Cl.[6]: **C07D 211/46**, C07D 211/54, C07D 401/12, C07D 211/58, C07D 409/12, C07D 401/14, A61K 31/445

(54) **Composés N-(aminoalkyl)pipéridine et leurs énantiomères comme antagonistes des récepteurs des neurokinines, procédés pour leur préparation et compositions pharmaceutiques les contenant**

N-(Aminoalkyl)piperidin-Verbindungen und ihre Enantiomere als Neurokinine-Receptoren Antagonisten, Verfahren zu ihrer Herstellung und diese enthaltende pharmazeutische Zusammensetzungen

N-(aminoalkyl)piperidine compounds and their enantiomers as neurokinines receptors antagonists, processes for their preparation and pharmaceutical compositions containing them

(84) Etats contractants désignés:
**AT BE CH DE DK ES FR GB GR IT LI LU NL PT SE**

(30) Priorité: **03.05.1991 FR 9105486**

(43) Date de publication de la demande:
**25.11.1992 Bulletin 1992/48**

(73) Titulaire: **SANOFI**
**75008 Paris (FR)**

(72) Inventeurs:
- **Emonds-Alt, Xavier**
  **F-34980 Combaillaux (FR)**
- **Martinez, Serge**
  **F-34000 Montpellier (FR)**
- **Proietto, Vincenzo**
  **F-34680 Saint Georges d'Orques (FR)**
- **Van Broeck, Didier**
  **F-34570 Murviel Les Montpellier (FR)**

(74) Mandataire: **Gillard, Marie-Louise et al**
**Cabinet Beau de Loménie**
**158, rue de l'Université**
**75340 Paris Cédex 07 (FR)**

(56) Documents cités:
**EP-A- 0 428 434**
**DE-A- 2 610 228**
**DE-A- 2 719 211**
**DE-A- 2 840 302**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 515 240 B1

## Description

La présente invention a pour objet de nouveaux dérivés aromatiques substitués par un groupement amino et par diverses fonctions amines ou amides, ainsi que leurs énantiomères.

La présente invention concerne également le procédé d'obtention des composés, qui peut être énantiosélectif et l'utilisation des composés selon l'invention dans des compositions à usage thérapeutique et plus particulièrement dans les phénomènes pathologiques qui impliquent le système des neurokinines comme : la douleur (D. Regoli et al., Life Sciences, 1987, 40, 109-117), l'allergie et l'inflammation (J.E. Morlay et al., Life Sciences, 1987, 41, 527-544), l'insuffisance circulatoire (J. Losay et al., 1977, Substance P, Von Euler, U.S. and Pernow ed., 287-293, Raven Press, New York), les troubles gastro-intestinaux (D. Regoli et al., Trends Pharmacol. Sci., 1985, 6, 481-484), les troubles respiratoires (J. MIZRAHI et al., Pharmacology, 1982, 25; 39-50).

Des ligands endogènes aux récepteurs des neurokinines ont été décrits, telles la substance P (SP), la neurokinine A (NKA) (S.J. Bailey et al., 1983, Substance P, P. Skrabanck ed., 16-17 Boole Press, Dublin) et la neurokinine B (NKB) (S.P. Watson, Life Sciences, 1983, 25, 797-808).

Les récepteurs aux neurokinines ont été reconnus sur de nombreuses préparations et sont actuellement classés en trois types : $NK_1$, $NK_2$ et $NK_3$. Alors que la plupart des préparations étudiées jusqu'à maintenant, présentent plusieurs types de récepteurs, tel l'iléon de cobaye ($NK_1$, $NK_2$ et $NK_3$), certaines d'entre elles n'en possèderaient qu'un seul, telles l'artère carotide de chien ($NK_1$), l'artère pulmonaire de lapin dépourvue d'endothélium ($NK_2$), et la veine porte de rat ($NK_3$) (D. Regoli et al., Trends Pharmacol. Sci., 1988, 9 290-295 et Pharmacology, 1989, 38, 1-15).

Une caractérisation plus précise des différents récepteurs est rendue possible par la synthèse récente d'agonistes sélectifs. Ainsi, la [$Sar^9$, $Met\text{-}(O2)^{11}$] SP, la [$Nle^{10}$] $NKA_{4\text{-}10}$, et la [Me $Phe^7$] -NKB présenteraient une sélectivité respective pour les récepteurs $NK_1$, $NK_2$ et $NK_3$ (cf D. Regoli, 1988 et 1989 précédemment cité).

On a maintenant trouvé que certains composés aromatiques aminés possèdent des propriétés pharmacologiques intéressantes, en tant qu'antagonistes des récepteurs des neurokinines et sont notamment utiles pour la traitement de toute pathologie substance P et neurokinine dépendante.

Ainsi selon un de ses aspects, la présente invention concerne des dérivés aromatiques aminés de formule :

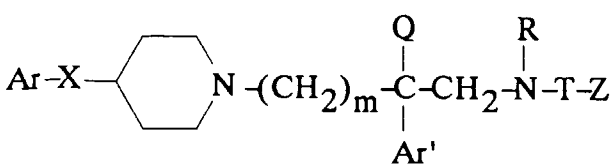 (I)

dans laquelle :

- m est égal à 2 ou 3 ;
- Ar représente un phényle non substitué ou substitué une ou plusieurs fois par un atome d'halogène, de préférence un atome de chlore ou de fluor, par un alkyle en $C_1$-$C_3$, par un trifluorométhyle, par un alcoxy dans lequel l'alkyle est en $C_1$-$C_3$, par un hydroxyle ou par un méthylènedioxy ; un groupe thiényle, pyridyle, imidazolyle substitué ou non par un alkyle en $C_1$-$C_3$ ;
- Ar' représente un groupe phényle non substitué, mono ou di-substitué par un atome d'halogène, de préférence un atome de chlore ou de fluor, par un alkyle en $C_1$-$C_3$, par un trifluorométhyle, par un alcoxy dans lequel l'alkyle est en $C_1$-$C_3$, par hydroxyle ou par un méthylènedioxy ; un groupe thiényle ; un groupe imidazolyle ou un groupe benzothiényle non substitués ou substitués par un halogène de préférence par un atome de chlore ou de fluor ; un groupe naphtyle non substitué ou substitué par un halogène de préférence par un atome de fluor ; un groupe biphényle ; un indolyle non substitué ou substitué sur l'azote par un groupe benzyle;
- X représente un atome d'oxygène, un atome de soufre, un groupe -NH- , un groupe

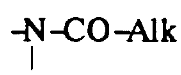

ou un groupe

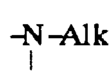

dans lesquels Alk est un groupe alkyle en $C_1$-$C_3$;

- Q représente l'hydrogène, un groupe alkyle en $C_1$-$C_4$ ou un groupe aminoalkyle de formule $-(CH_2)_q$-Am', où q est 2 ou 3 et Am' est un groupe pipéridino, benzyl-4 pipéridino ou dialkylamino, chaque alkyle pouvant contenir de 1 à 4 atomes de carbone ;
- R représente l'hydrogène, un groupe méthyle ou un groupe $(CH_2)_n$-L, où n est un nombre entier de 2 à 6 et L est l'hydrogène ou un groupe amino ;
- T représente un groupe choisi parmi :

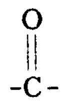 et 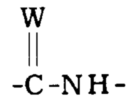

W étant un atome d'oxygène ou de soufre, et

- Z représente soit M ou OM lorsque T représente :

le groupe

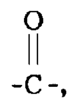

soit M lorsque T représente le groupe

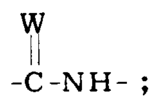

M représente l'hydrogène ou un alkyle droit ou ramifié en $C_1$-$C_6$ ; un α-hydroxybenzyle, un α-alkylbenzyle ou un phénylalkyle dans lesquels le groupe alkyle comprend de 1 à 3 atomes de carbone, non substitué, mono ou polysubstitué sur le cycle aromatique par un halogène, un hydroxy, un alcoxy de 1 à 4 atomes de carbone, un alkyle de 1 à 4 atomes de carbone ; un pyridylalkyle dans lequel le groupe alkyle comprend de 1 à 3 atomes de carbone ; un naphtylalkyle dans lequel le groupe alkyle comprend de 1 à 3 atomes de carbone ; un pyridylthioalkyle dans lequel le groupe alkyle comprend de 1 à 3 atomes de carbone ; un styryle ; un (méthyl-1) imidazolyl-2 thioalkyle dans lequel le groupe alkyle comprend de 1 à 3 atomes de carbone ; un oxo-1 phénylindan-3 yle-2 ; un groupe aromatique ou hétéroaromatique non substitué, mono ou polysubstitué tel que défini ci-après ;

ou un de leurs sels avec des acides minéraux ou organiques ou leurs sels d'ammonium quaternaire

Les sels d'ammonium quaternaire des composés de formule (I) sont formés à partir de l'azote de la pipéridine. Le groupe

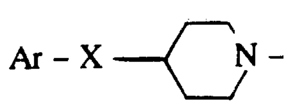

est alors représenté par le groupe :

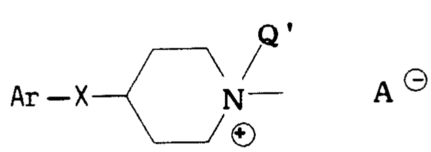

dans laquelle

- Q' représente un groupe alkyle en $C_1$-$C_6$ ou un groupe benzyle et
- $A^{\ominus}$ représente un anion choisi parmi chlorure, bromure, iodure, acétate, méthanesulfonate ou paratoluènesulfonate.

Les sels de composés de formule (I) selon la présente invention comprennent aussi bien ceux avec des acides minéraux ou organiques qui permettent une séparation ou une cristallisation convenable des composés de formule (I), tels que l'acide picrique ou l'acide oxalique ou un acide optiquement actif, par exemple un acide mandélique ou camphosulfonique, que ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le 2-naphtalènesulfonate, le glycolate, le gluconate, le citrate, l'iséthionate.

De façon grossière, dans la formule (I), Z représente un groupe aromatique ou hétéroaromatique mono-, di- ou tricyclique, pouvant porter un ou plusieurs substituants, dont un atome de carbone du carbocycle aromatique ou de l'hétérocycle aromatique est directement lié au groupe T.

Plus particulièrement, le radical Z peut être un groupe phényle, qui peut être non substitué ou éventuellement contenir un ou plusieurs substituants.

Lorsque Z est un groupe phényle, celui-ci peut être de préférence mono- ou disubstitué notamment en position 2,4, mais aussi par exemple en position 2,3, 4,5, 3,4 ou 3,5 ; il peut aussi être trisubstitué, notamment en position 2,4,6 mais aussi par exemple en 2,3,4, 2,3,5 ou 2,4,5 3,4,5 ; tétrasubstitué, par exemple en 2,3,4,5 ; ou pentasubstitué. Les substituants du groupe phényle peuvent être : F ; Cl ; Br ; I ; CN ; OH ; $NH_2$ ; NH-CO-$NH_2$; $NO_2$; $CONH_2$ ; $CF_3$ ; alkyle en $C_1$-$C_{10}$, de préférence en $C_1$-$C_4$, méthyle ou éthyle étant préférés, ainsi que par exemple n-propyle, isopropyle, n-butyle, isobutyle, sec-butyle, tert.-butyle, pentyle ou n-pentyle, hexyle ou n-hexyle, heptyle ou n-heptyle, octyle ou n-octyle, nonyle ou n-nonyle ainsi que décyle ou n-décyle ; alcényle contenant 2 à 10, de préférence 2-4 atomes de carbone, par exemple vinyle, allyle, 1-propényle, isopropényle, butényle ou 1-butèn-1-, -2-, -3- ou -4-yle, 2-butèn-1-yle, 2-butèn-2-yle, pentènyle, hexényle ou décényle ; alcynyle contenant 2 à 10, de préférence 2-4 atomes de carbone, par exemple éthynyle, 1-propyn-1-yle, propargyle, butynyle ou 2-butyn-1-yle, pentynyle, décynyle ; cycloalkyle contenant 3 à 8, de préférence 5 ou 6 atomes de carbone, cyclopentyle ou cyclohexyle étant préférés, ainsi que par exemple cyclopropyle, cyclobutyle, 1-, 2- ou 3-méthylcyclopentyle, 1-, 2-, 3- ou 4-méthylcyclohexyle, cycloheptyle ou cyclooctyle ; bicycloalkyle contenant 4 à 11, de préférence 7 atomes de carbone, exo ou endo 2-norbornyle étant préférés, ainsi que par exemple 2-isobornyle ou 5-camphyle ; hydroxyalkyle contenant 1 à 5, de préférence 1-2 atomes de carbone, hydroxyméthyle et 1- ou 2-hydroxyéthyle étant préférés, ainsi que par exemple 1-hydroxyprop-1-yle, 2-hydroxyprop-1-yle, 3-hydroxyprop-1-yle, 1-hydroxyprop-2-yle, 1-hydroxybut-1-yle, 1-hydroxypent-1-yle ; alcoxy contenant 1 à 10, de préférence 1-4 atomes de carbone, méthoxy ou éthoxy étant préférés, ainsi que par exemple n-propoxy, isopropoxy, n-butoxy, isobutoxy, sec-butoxy, tert-butoxy, pentyloxy, hexyloxy, heptyloxy, octyloxy, nonyloxy ou décyloxy ; alcoxyalkyle contenant 2 à 10, de préférence de 2 à 6 atomes de carbone, par exemple alcoxyméthyle ou alcoxyéthyle, tel que méthoxyméthyle ou 1- ou 2-méthoxyéthyle, 1- ou 2-n-butoxyéthyle, 1- ou 2-n-octyloxyéthyle ; alcoxyalcoxyalkyle contenant de 3 à 10, de préférence de 4 à 7 atomes de carbone, par exemple alcoxyalcoxyméthyle, par exemple 2-méthoxyéthoxyméthyle, 2-éthoxyéthoxyméthyle ou 2-isopropoxyéthoxyméthyle, alcoxyalcoxyéthyle par exemple 2-(2-méthoxyéthoxy)éthyle ou 2-(2-éthoxyéthoxy)éthyle ; alcoxyalcoxy contenant de 2 à 10, de préférence de 3 à 6 atomes de carbone, par exemple 2-méthoxyéthoxy, 2-éthoxyéthoxy ou 2-n-butoxyéthoxy ; alcényloxy contenant 2 à 10, de préférence 2 à 4 atomes de carbone, allyloxy étant préféré, ainsi que par exemple vinyloxy, propényloxy, isopropényloxy, buténylowy tel que 1-butèn-1-, -2-, -3- ou -4-yloxy, 2-butèn-1-yloxy, 2-butèn-2-yloxy, pentènyloxy, hexényloxy ou décényloxy ; alcényloxyalkyle contenant de 3 à 10, de préférence 3-6 atomes de carbone, par exemple allyloxyméthyle ; alcynyloxy contenant de 2 à 10, de préférence 2 à 4 atomes de carbone, propargyloxy étant préférés, ainsi que par exemple éthynyloxy, 1-propyn-1-yloxy, butynyloxy ou 2-butyn-1-yloxy, pentynyloxy ou décynyloxy ; alcynyloxyalkyle contenant de 3 à 10 de préférence 3 à 6 atomes de carbone, par exemple éthylnyloxyméthyle, propargyloxyméthyle ou 2-(2-butyn-1-yloxy)éthyle ; cycloalcoxy contenant 3 à 8, de préférence 5 ou 6 atomes de carbone, cyclopentyloxy ou cyclohexyloxy étant préférés, ainsi que par exemple cyclopropyloxy, cyclobutyloxy, 1-, 2- ou 3-méthylcyclopentyloxy, 1-, 2-, 3- ou 4-méthylcyclohexyloxy, cycloheptyloxy ou cyclooctyloxy ; alkylthio contenant de 1 à 10, de préférence 1 à 4 atomes de carbone, méthylthio ou éthylthio étant préférés, ainsi que par exemple n-propylthio, isopropylthio, n-butylthio, isobutylthio, sec-butylthio, tert-butylthio, pentylthio, hexylthio, octylthio, nonylthio ou décylthio ; alkylthioalkyle contenant de 2 à 10, de préférence 2 à 6 atomes de carbone, par exemple méthylthiométhyle, 2-méthylthioéthyle ou 2-n-butylthioéthyle ; acylamino, à savoir alcanoylamino contenant de 1 à 7, de préférence 1 à 4 atomes de carbone, formylamino et acétylamino étant préférés, ainsi que propionylamino, butyrylamino, isobutyrylamino, valérylamino, caproylamino, heptanoylamino, ainsi que benzoylamino ; acylaminoalkyle, à savoir alcanoylaminoalkyle contenant de 2 à 8, de préférence 3 à 6 atomes de carbone, tel que formylaminoéthyle, acétylaminoéthyle, propionylaminoéthyle, n-butyrylaminoéthyle, formylaminopropyle, acétylaminopropyle, propionylaminopropyle, formylaminobutyle, acétylaminobutyle, ainsi que propionylaminobutyle, butyrylaminobutyle ; acyloxy contenant de 1 à 6, de préférence 2 à 4 atomes de carbone, acétyloxy, propionyloxy ou butyryloxy étant préférés, ainsi que par exemple formyloxy, valéryloxy, caproyloxy ; alcoxycarbonyle contenant de 2 à 5, de préférence 2 et 3 atomes de carbone, méthoxycarbonyle et éthoxycarbonyle étant préférés, ainsi que par exemple n-propoxycarbonyle, isopropoxycarbonyle, n-butoxycarbonyle, isobutoxycarbonyle, sec-butoxycarbonyle ou tert-butoxycarbonyle ; cycloalcoxycarbonyle contenant de 4 à 8, de préférence 6 ou 7 atomes de carbone, cyclopentyloxycarbonyle, cyclohexyloxycarbonyle étant préférés, ainsi que cyclopropyloxycarbonyle, cyclobutyloxycarbonyle ou cycloheptyloxycarbonyle ; alkylaminocarbonylamino contenant de 2 à 4 atomes de carbone, tel que méthylaminocarbonylamino, éthylaminocarbonylamino, propylaminocarbonylamino ; dialkylaminocarbonylamino contenant de 3 à 7, de préférence 3 à 5 atomes de carbone, de préférence diméthylaminocarbonylamino, ainsi que di-n-propylaminocarbonylamino, diisopropylaminocarbonylamino ; (1-pyrrolidino)carbonylamino (1-pipéridino)carbonylamino ; cycloalkylaminocarbonylamino contenant de 4 à 8, de préférence 6 ou 7 atomes de carbone, cyclopentylaminocarbonylamino, cyclohexylaminocarbonylamino étant préférés, ainsi que cyclopropylaminocarbonylamino, cyclobutylaminocarbonylamino, cycloheptylaminocarbonylamino; alkylaminocarbonylaminoalkyle contenant de 3 à 9, de préférence 4 à 7 atomes de carbone, méthylaminocarbonylaminoéthyle, éthylaminocarbonylaminoéthyle, éthylaminocarbonylaminopropyle, éthylaminocarbonylaminobutyle étant préférés, ainsi que par exemple méthylaminocarbonylaminométhyle, n-propylaminocarbonylaminobutyle, n-butylaminocarbonylaminobutyle ; dialkylaminocarbonylaminoalkyle contenant de 4 à 11 atomes de carbone, par exemple diméthylaminocarbonylaminométhyle, diéthylaminocarbonylaminoéthyle, diéthylaminocarbonylaminopropyle, diéthylaminocarbonylaminobutyle, (1-pyrrolidino) carbonylaminoéthyle, (1-pipéridino)- carbonylaminoéthyle, cycloalkylaminocarbonylaminoalkyle contenant de 5 à 12, de préférence 8 à 11 atomes de carbone, cyclopentylaminocarbonylaminoéthyle, cyclopentylaminocarbonylaminopropyle, cyclohexylaminocarbonylaminopropyle cyclohexylaminocarbonylaminoéthyle, cyclohexylaminocarbonylaminopropyle et cyclohexylaminocarbonylaminobutyle étant préférés, ainsi que par exemple cyclopropylaminocarbonylaminométhyle, cycloheptylaminocarbonylaminoéthyle ; alcoxycarbonylaminoalkyle contenant de 3 à 12, de préférence 4 à 9 atomes de carbone, méthoxycarbonylaminoéthyle, éthoxycarbonylaminoéthyle, n-propoxycarbonyleaminoéthyle, isopropoxycarbonylaminoéthyle, n-butoxycarbonylaminoéthyle, isobutoxycarbonylaminoéthyle, sec-butoxycarbonylaminoéthyle, tert-butoxycarbonylaminoéthyle, éthoxycarbonylaminopropyle, n-butoxycarbonylaminopropyle, éthoxycarbonylaminobutyle, n-butoxycarbonylaminobutyle étant préférés, ainsi que par exemple n-propoxycarbonylaminopropyle, n-propoxycarbonylaminobutyle, isopropoxycarbonylaminobutyle, cycloalcoxycarbonylaminoalkyle contenant de 5 à 12, de préférence 8 à 11 atomes de carbone, cyclopentyloxycarbonylaminoéthyle, cyclopentyloxycarbonylaminopropyle, cyclopentyloxycarbonylaminobutyle, cyclohexyloxycarbonylaminoéthyle, cyclopentyloxycarbonylaminopropyle, cyclohexyloxycarbonylaminobutyle étant préférés, ainsi que par exemple cyclopropyloxycarbonylaminométhyle, cycloheptyloxycarbonylaminoéthyle ; carbamoylalkyle contenant de 2 à 5, de préférence 2 atomes de carbone, de préférence carbamoylméthyle, ainsi que carbamoyléthyle, carbamoylpropyle, carbamoylbutyle ; alkylaminocarbonylalkyle contenant de 3 à 9, de préférence 3 à 6 atomes de carbone, méthylaminocarbonyléthyle, éthylaminocarbonylméthyle, n-propylaminocarbonylméthyle, isopropylaminocarbonylméthyle, n-butylaminocarbonylméthyle, isobutylaminocarbonylméthyle, sec-butylaminocarbonylméthyle, tertbutylaminocarbonylméthyle étant préférés, ainsi que par exemple éthylaminocarbonyléthyle, éthylaminocarbonylpropyle, éthylaminocarbonylbutyle, n-propylaminocarbonylbutyle, n-butylaminocarbonylbutyle ; dialkylaminocarbonylalkyle contenant de 4 à 11, de préférence 4 à 8 atomes de carbone, diméthylaminocarbonylméthyle, diéthylaminocarbonylméthyle, di-n-propylaminocarbonylméthyle ainsi que par exemple diéthylaminocarbonyléthyle, diéthylaminocarbonylpropyle, diéthylaminocarbonylbutyle ; (1-pyrrolidino)carbonylméthyle, (1-pipéridino)carbonylméthyle ; (1-pipéridino)carbonyléthyle ; cycloalkylaminocarbonylalkyle contenant de 5 à 12, de préférence 7 ou 8 atomes de carbone, cyclopentylaminocarbonylméthyle et cyclohexylaminocarbonylméthyle étant préférés, ainsi que par exemple cyclopropylaminocarbonylméthyle, cyclobutylaminocarbonylméthyle, cycloheptylaminocarbonylméthyle, cyclohexylaminocarbonyléthyle, cyclohexylaminocarbonylpropyle, cyclohexylaminocarbonylbutyle ; alkylaminocarbonylalcoxy contenant de 3 à 10, de préférence 3 à 5 atomes de carbone, méthylaminocarbonylméthoxy étant préféré, ainsi que par exemple méthylaminocarbonyléthoxy, méthylaminocarbonylpropoxy ; dialkylaminocarbonylalcoxy contenant de 4 à 10, de préférence 4 à 7 atomes de carbone, tel que diméthylaminocarbonylméthoxy, diéthylaminocarbonyléthoxy; (pipéridinyl-1) carbonylméthoxy ; cycloalkylaminocarbonylalcoxy contenant de 5 à 11, de préférence 7 ou 8 atomes de carbone, tel que cyclopentylaminocarbonylméthoxy, cyclohexylaminocarbonylméthoxy.

Le groupe Z est avantageusement un groupe phényle ; un groupe benzyle ; ou encore un groupe benzoyle ; un groupe phénylthioalkyle dans lequel l'alkyle est en $C_1$-$C_3$; lesdits groupes étant non substitués, mono ou disubstitués par un halogène, le groupe 2,4-dichlorophényle étant particulièrement préféré, ou par un alcoxy en $C_1$-$C_4$.

Le radical Z peut également représenter un groupe aromatique bicyclique choisi parmi le 1- ou 2-naphtyle ; 1-, 2-, 3-, 4-, 5-, 6-, 7-indényle ; dont une ou plusieurs liaisons peuvent être hydrogénées, lesdits groupes pouvant être non substitués ou contenir éventuellement un ou plusieurs substituants choisis parmi : un halogène et plus particulièrement un atome de fluor, le groupe alkyle, phényle, cyano, hydroxyalkyle, hydroxy, oxo, alkylcarbonylamino et alcoxycarbonyle, thioalkyle dans lesquels les alkyles sont en $C_1$-$C_4$.

Le radical Z peut être aussi un groupe pyridyle, thiadiazolyle, indolyle, indazolyle, imidazolyle, benzimidazolyle, benzotriazolyle, benzofuranyle, benzothiényle, benzothiazolyle, benzisothiazolyle, quinolyle, isoquinolyle, benzoxazolyle, benzisoxazolyle, benzoxazinyle, benzodioxinyle, isoxazolyle, benzopyrannyle, thiazolyle, thiényle, furyle, pyrannyle, chroményle, isobenzofuranyle, pyrrolyle, pyrazolyle, pyrazinyle, pyrimidinyle, pyridazinyle, indolizinyle, phtalazinyle, quinazolinyle, acridinyle, isothiazolyle, isochromanyle, chromanyle, dont une ou plusieurs doubles liaisons peuvent être hydrogénées, lesdits groupes pouvant être non substitués ou contenir éventuellement un ou plusieurs substituants choisis parmi : le groupe alkyle, phényle, cyano, hydroxyalkyle, hydroxy, alkylcarbonylamino et alcoxycarbonyle, thioalkyle dans lesquels les alkyles sont en $C_1$-$C_4$.

Le groupe Ar' est avantageusement un phényle et plus particulièrement un phényle substitué une ou plusieurs fois par un halogène, de préférence par un atome de chlore.

Selon un autre de ses aspects, la présente invention concerne un procédé pour la préparation de composés aromatiques aminés différemment substitués de formule (I) et de leurs sels, caractérisé en ce que :

a) on traite une amine libre de formule :

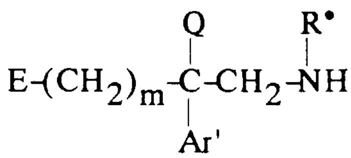

(II)

dans laquelle m, Ar' et Q sont tels que définis précédemment ; R° représente l'hydrogène, un groupe méthyle ou un groupe $(CH_2)_n$-L°, où n, est tel que défini précédemment et L° est l'hydrogène ou un groupe amino protégé par un groupe N-protecteur ; et E représente un groupe hydroxy, un groupe O-protégé tel que tétrahydropyranyl-2 oxy, un groupe mésyloxy ou un groupe :

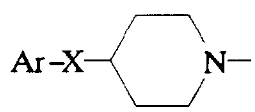

dans lequel Ar et X sont tels que définis précédemment ;

- soit avec un dérivé fonctionnel d'un acide de formule :

HO-CO-Z (III)

dans laquelle Z est tel que défini précédemment, lorsqu'on doit préparer un composé de formule (I) où T est -CO-,

- soit avec un iso(thio)cyanate de formule :

W = C = N-Z (III')

dans laquelle W et Z sont tels que définis précédemment, lorsqu'on doit préparer un composé de formule (I) où T est -C(W)-NH-, pour former le composé de formule:

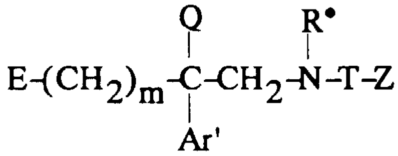

(IV)

b) puis, lorsque E représente le tétrahydropyranyloxy, on élimine le groupe tétrahydropyranyle par hydrolyse acide,

l'hydrolyse pouvant alternativement avoir lieu à l'étape (a) sur l'amine de départ de formule (II),
c) lorsque E représente un groupe hydroxy ou un groupe tétrahydropyranyloxy qui a été hydrolysé, on traite l'alcanolamine N-substituée ainsi obtenue de formule :

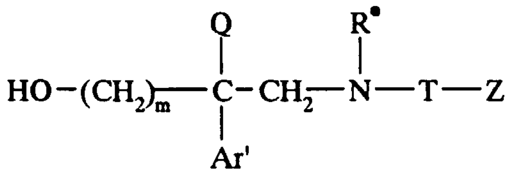

(V)

avec le chlorure de méthanesulfonyle,
d) on fait réagir le mésylate ainsi obtenu à l'étape c) ou directement à l'issue de l'étape a) lorsque E représente un groupe méthanesulfonyloxy de formule :

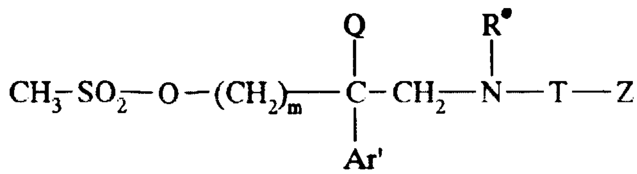

(VI)

avec une amine secondaire de formule :

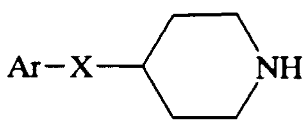

(VII)

dans laquelle Ar et X sont tels que définis précédemment,
e) on élimine les groupes N-protecteurs éventuels et on transforme éventuellement le produit ainsi obtenu à l'étape d) ou directement à l'issue de l'étape a) lorsque E représente un groupe

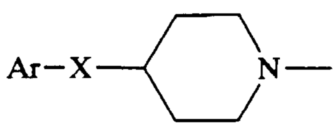

en un de ses sels.

Comme dérivé fonctionnel de l'acide (III), on utilise l'acide lui-même, convenablement activé par exemple par le cyclohexylcarbodiimide ou par l'hexafluorophosphate de benzotriazolyl N-oxytrisdiméthylaminophosphonium (BOP), ou bien un des dérivés fonctionels qui réagissent avec les amines, par exemple un anhydride, un anhydride mixte, le chlorure ou un ester activé. Lorsque Z est un groupe OM, l'acide concerné est l'acide carbonique et, comme dérivé fonctionnel, on utilise le monochlorure, à savoir un chloroformiate Cl-CO-OM.

Les groupes N-protecteurs éventuellement présents dans le groupe $R^{\circ}$ du composé de la formule (II) sont les groupes N-protecteurs classiques bien connus de l'homme de l'art et de préférence ceux qui sont éliminables par hydrolyse acide, tels que le groupe trityle, méthoxytrityle ou BOC.

Lorsque comme produit de départ on utilise un composé de formule (II) où E représente un groupe :

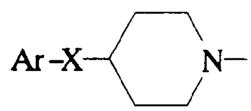

le procédé de la présente invention peut être représenté et illustré en détail par le Schéma 1 ci-après :

SCHEMA 1

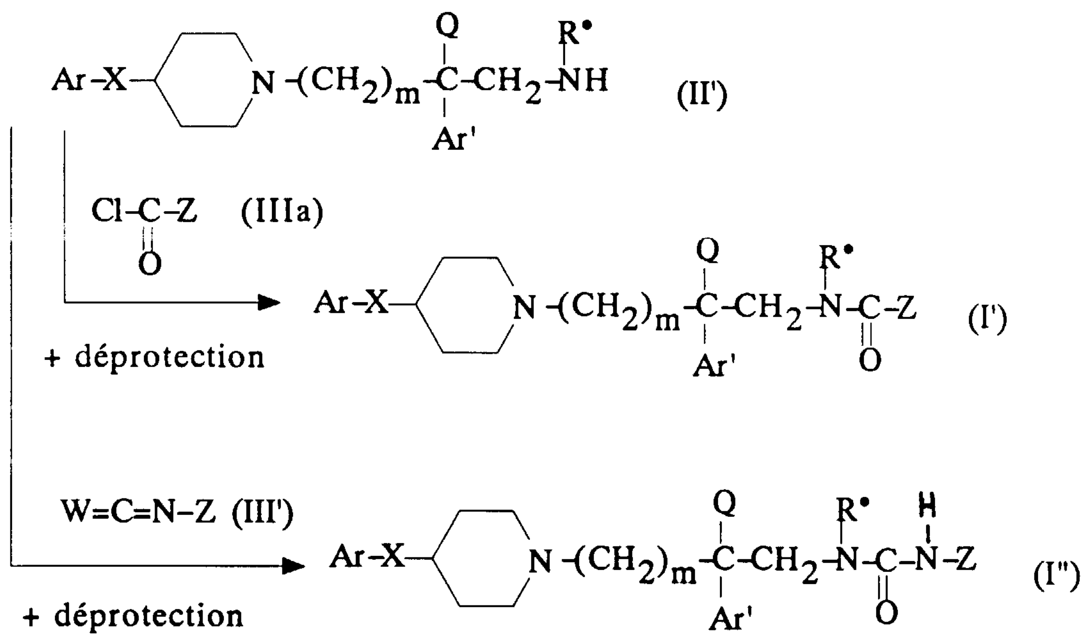


Dans la formule (IIIa) ci-dessus, on considère le chlorure d'acide comme dérivé fonctionnel réactif de l'acide (III). Le chlorure d'acide est utilisé lorsque l'on désire préparer un composé (I') où Z est OM. La réaction avec le chlorure d'acide est effectuée dans un solvant inerte, tel que le dichlorométhane ou le benzène en présence d'une base telle que par exemple la triéthylamine, à température ambiante.

Dans le cas particulier de Z = OM, la réaction du composé (II') avec le chloroformiate de formule :

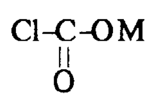

est effecutée selon les méthodes habituelles.

Lorsque Z est autre que OM, on peut utiliser un autre dérivé fonctionnel ou on peut partir de l'acide libre (III) en réalisant un couplage de (II') avec le BOP (hexafluorophosphate de benzotriazolyl N-oxytrisdiméthylaminophosphonium), puis en additionnant l'acide (III) en présence d'une base organique comme par exemple la triéthylamine, dans un solvant comme le dichlorométhane ou le diméthylformamide, à température ambiante, les composés (I') obtenus étant isolés et purifiés selon les méthodes habituelles, comme par exemple la chromatographie ou la recristallisation.

On peut faire aussi réagir (II') avec iso(thio)cyanate W=C=N-Z (III') dans un solvant inerte anhydre tel que par exemple le benzène, pendant une nuit à température ambiante puis traiter le mélange réactionnel selon les méthodes habituelles pour obtenir les composés (I").

Lorsque comme produit de départ on utilise un composé de formule (II) où E représente un groupe tétrahydropyranyloxy, le procédé de la présente invention peut être représenté et illustré à partir du Schéma 2.

Les réactions du composé (II") avec les réactifs (IIIa) et (III') se déroulent comme décrit ci-dessus pour le Schéma 1, le chlorure d'acide (IIIa) pouvant être remplacé par un autre dérivé fonctionnel ou par l'acide libre activé par exemple par le BOP.

L'intermédiaire (IV') ainsi obtenu est déprotégé par hydrolyse acide pour conduire au composé hydroxylé libre (V). La déprotection par hydrolyse du groupe tétrahydropyranyloxy peut être effectuée directement sur le composé (II"). On obtient ainsi le composé hydroxylé (II‴) qui est mis en réaction directement avec les réactifs (IIIa) ou (III') comme décrit dans le Schéma 2 ci-dessous pour fournir le composé (V). On prépare ensuite le mésylate (VI) pour le substituer par une amine secondaire de formule (VII) pour obtenir finalement, après déprotection éventuelle de l'amine L•, les composés (I) selon l'invention.

SCHEMA 2

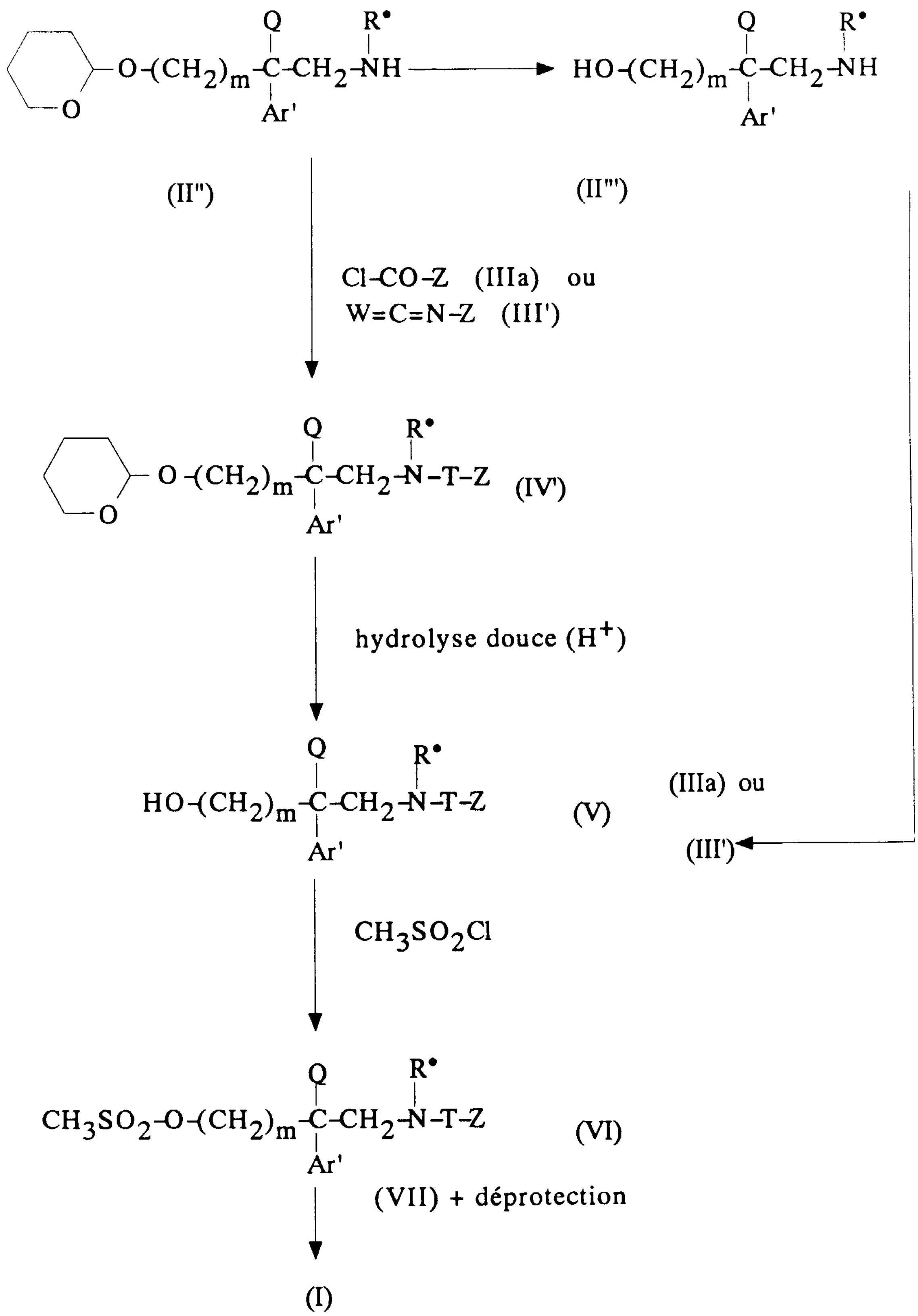


(II") (II''') (IV') (V) (VI) (I)

Lorsque le produit obtenu à la fin de la réaction entre le composé de formule (II) et le composé (III) (comme dérivé

fonctionnel) ou (III'), a la formule IV où E représente un groupe :

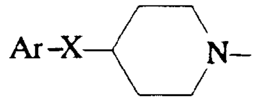

dans lequel Ar et X sont tels que définis ci-dessus, le produit peut soit représenter le produit final soit posséder un groupe amino protégé (L•). Dans ce dernier cas, les groupes N-protecteurs sont hydrolysés selon les méthodes habituelles.

La déprotection est effectuée selon les méthodes connues ; notamment, si l'on utilise, comme groupe O-protecteur, un groupe tétrahydropyranyle, l'hydrolyse peut être effectuée en conditions douces avec de l'acide p-toluènesulfonique dilué. Si la molécule du produit (IV) contient à la fois un groupe tétrahydropyranyloxy et un groupe tritylamino, l'hydrolyse du premier peut être ainsi effectuée en respectant le groupe N-protecteur, alors que l'acide formique libère en même temps les deux groupes protecteurs.

Les produits de formule (I) ainsi obtenus sont isolés, sous forme de base libre ou de sel, selon les techniques classiques.

Lorsque le composé de formule (I) est obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques classiques. Ainsi, on prépare par exemple le chlorhydrate, le bromhydrate, le sulfate, l'hydrogénosulfate, le dihydrogénophosphate, le méthanesulfonate, l'oxalate, le maléate, le fumarate, le naphtalène-2 sulfonate.

A la fin de la réaction, les composés de formule (I) peuvent être isolés sous forme d'un de leurs sels, par exemple le chlorhydrate ou l'oxalate ; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Les sels d'ammonium quaternaires formés avec l'azote de la pipéridine sont préparés par réaction des bases libres des composés (I), pour lesquelles les fonctions aminées autres, éventuellement présentes sont N-protégées par un groupe N-protecteur habituel, avec un excès d'agent alkylant de formule :

A - Q'

dans lequel A représente un groupe partant et est tel que défini précédemment pour (I), de préférence un chlorure ou un iodure et Q' est tel que défini précédemment pour (I) et on chauffe le mélange réactionnel dans un solvant par exemple choisi parmi le dichlorométhane, le chloroforme, l'acétone ou l'acétonitrile à une température comprise entre la température ambiante et le reflux pendant une à plusieurs heures pour obtenir après traitement selon les méthodes habituelles et après déprotection éventuelle, un mélange de conformères axiaux et équatoriaux des sels d'ammonium quaternaires.

De préférence, $A^{\ominus}$ représente un iodure qui peut être échangé par un autre anion ou par un anion pharmacologiquement acceptable, par exemple un chlorure, par élution du composé (I) sur une résine échangeuse d'ion, par exemple l'Amberlite IRA68 ® ou Duolite A375 ®.

Les conformères sont séparés selon les méthodes habituelles, par exemple par chromatographie ou par recristallisation.

Chacun des conformères axiaux ou équatoriaux des composés (I) sous forme de racémiques ou sous forme d'énantiomères R ou S optiquement purs font partie de l'invention.

La résolution des mélanges racémiques (I) permet d'isoler les énantiomères qui font partie de l'invention.

On peut aussi effectuer le dédoublement de mélanges racémiques des produits de formule (II), notamment des produits de formule (II') et (II''') ou de leurs précurseurs, afin de préparer les énantiomères des produits de formule (I).

Le dédoublement des produits de formule (II) est effectué selon EP-A-428434.

Les composés de départ de formule (II) sont préparés à partir de nitriles de formule :

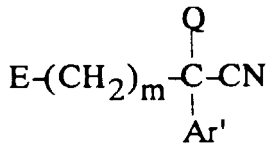

(VIII)

dans laquelle m, E, Q et Ar' sont tels que définis ci-dessus, par réduction et alkylation éventuelle de l'amine obtenue.

Pour la préparation des composés de formule (II) où R• est l'hydrogène, les nitriles de départ de formule (VIII) sont soumis à une hydrogénation dans un alcanol tel que l'éthanol, en présence d'un catalyseur tel que par exemple de nickel de Raney et l'amine libre primaire peut être isolée selon les méthodes classiques.

Lorsqu'on souhaite préparer les composés de formule (II) où R• est méthyle, on traite l'amine libre, obtenue par hydrogénation du nitrile (VIII) comme décrit ci-dessus, avec un chloroformiate, par exemple avec le chloroformiate de formule Cl-CO-OAlk, où Alk est un alkyle en $C_1$-$C_3$, de préférence éthyle, pour obtenir les carbamates de formule :

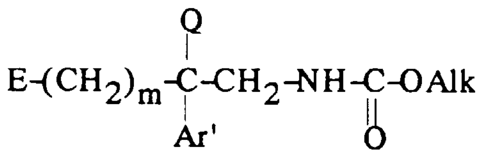

qui sont ensuite réduits par des moyens connus, tels que l'action d'un agent réducteur comme par exemple un hydrure métallique, tel que l'hydrure de sodium et d'aluminium, l'hydrure de lithium et d'aluminium ou par un hydrure de bore, tel que le diméthylsulfure de borane. La réduction est réalisée dans un solvant, tel que l'éther ou le toluène à une température comprise entre la température ambiante et 60°C. La méthylamine ainsi obtenue de formule :

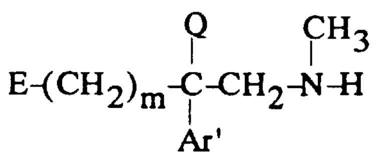

(II, R• = $CH_3$)

est isolée selon les méthodes habituelles.

Pour préparer les composés de formule (II) où R• est un groupe $-(CH_2)_n-L^•$ où n et L• sont tels que définis ci-dessus, on traite l'amine libre, obtenue par hydrogénation du nitrile (VIII) comme décrit ci-dessus, avec un dérivé fonctionnel réactif de l'acide de formule :

$$L^•-(CH_2)_{n-1}-COOH \qquad (IX)$$

pour obtenir un amide de formule :

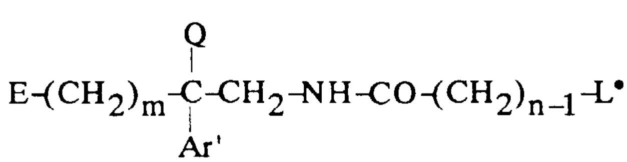

(X)

dans laquelle m, n, E, Ar', Q et L• sont tels que définis ci-dessus.

L'amide (X), par réduction dans les mêmes conditions que celles décrites ci-dessus pour le nitrile (VIII), donne le composé désiré de formule :

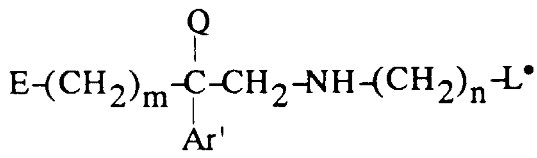

(II, R• = $(CH_2)_n$-L•)

Les nitriles de formule (VIII), sont préparés à partir de nitriles commerciaux ou connus et préparés selon des méthodes connues, de formule :

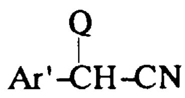

(XI)

qui par alkylation avec un composé de formule :

$$E\text{-}(CH_2)_m\text{-}J \qquad (XII)$$

dans laquelle m et E sont tels que définis ci-dessus et J est un atome d'halogène, par exemple de brome, donnent les composés (VIII) désirés.

De préférence, la synthèse des nitriles de formule (VIII) où E est un groupe tétrahydropyranyloxy est réalisée à partir d'un dérivé tétrahydropyranyloxy (THP-O-) obtenu par réaction entre un alcanol de formule Br-$(CH_2)_m$-OH avec m tel que défini précédemment et le dihydropyrane pour conduire au composé :

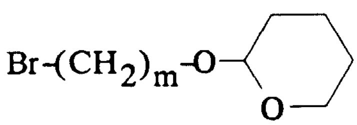

(XII, E = THP-O-, J = Br)

qui est ensuite mis en réaction, en présence d'hydrure alcalin avec le dérivé acétonitrile (XI) pour préparer l'intermédiaire,

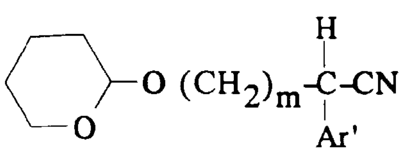

(VIII, E = THP-O-, Q = H)

correspondant aux composés de formule (VIII) qui sont des précurseurs intermédiaires des composés (II') du schéma 1 ci-dessus, où Q est l'hydrogène, lequel peut ensuite être alkylé.

La synthèse des nitriles de formule (VIII) où E représente un groupe :

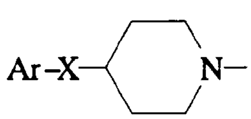

dans lequel Ar et X sont tels que définis précédemment, est effectuée selon des méthodes connues en mettant en réaction sur des dérivés chlorés de formule :

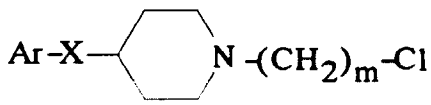 (XIII)

un dérivé nitrile de formule :

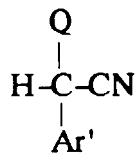 (XIV)

en présence d'amidure de sodium dans un solvant tel que le toluène à des températures comprises entre 30 et 80°C.

Le dérivé chloré (XIII) est préparé par action d'un réactif chlorant tel que par exemple le chlorure de thionyle sur le dérivé hydroxyl de formule :

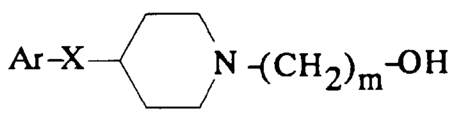 (XV)

lui même préparé à partir de l'amine de formule :

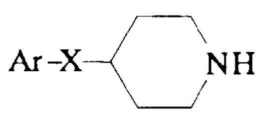 (VII')

sur laquelle on fait réagir, si m = 2, l'oxyde d'éthylène et si m = 3, un halogéno-3 propanol.

Les amines de départ de formule (II) dans lesquelles le groupe E est un groupe de formule :

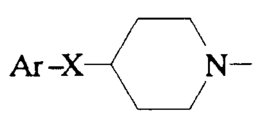

sont des composés nouveaux qui font partie également de l'invention.

Comme indiqué ci-dessus, les intermédiaires qui sont susceptibles de donner des sels avec des acides optiquement actifs peuvent être résolus afin de permettre la préparation des énantiomères des composés de formule (I).

On peut également prévoir la synthèse stéréospécifique d'intermédiaires qui ne donnent pas de sel permettant la séparation.

Un intermédiaire particulièrement adapté pour une telle synthèse stéréospécifique est l'alcool de formule (V) ci-dessus.

Ainsi, selon un autre de ses aspects, la présente invention concerne les énantiomères et un procédé pour la préparation des énantiomères des composés de formule (I) et de leurs sels ; lesdits énantiomères répondent à la formule (I*) ci-après :

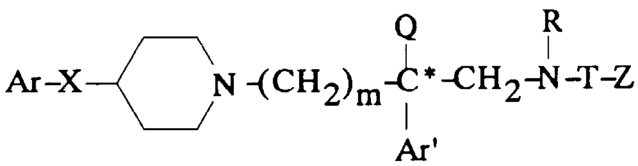 (I*)

dans laquelle :

Ar, Ar', Z, X, Q, R, T et m sont tels que définis précédemment et "*" signifie que l'atome de carbone ainsi marqué a une configuration absolue (+) ou (-) déterminée.

Ce procédé est caractérisé en ce que on traite un composé de formule :

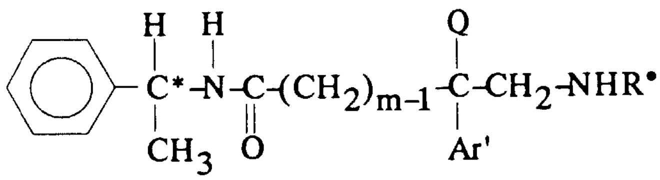 (XVII*)

dans un solvant tel que par exemple le dioxane, en milieu acide, par exemple en présence d'acide chlorhydrique pour fournir l'aminoacide de formule :

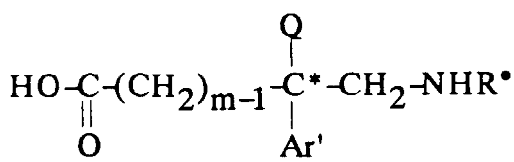 (XVIII*)

qui est estérifié dans un alcanol AlkOH, où Alk est un alkyle de 1 à 4 atomes de carbone, en milieu acide, puis on traite l'ester correspondant de formule :

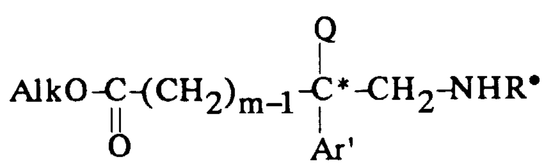 (XIX*)

dans lequel Alk, Q, Ar', R• et m sont tels que définis ci-dessus,

- soit avec un dérivé fonctionnel d'un acide de formule :

$$HO\text{-}CO\text{-}Z \qquad (III)$$

- soit avec un iso(thio)cyanate de formule :

$$W{=}C{=}N\text{-}Z \qquad (III')$$

Z et W étant tels que définis ci-dessus,

selon les conditions opératoires identiques à celles utilisées pour la préparation des dérivés (IV) ci-dessus, pour obtenir l'ester de formule :

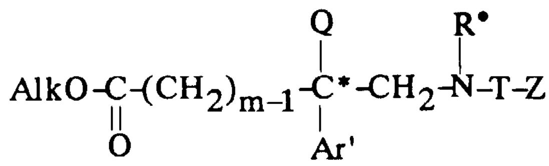 (XX*)

qui est alors réduit en alcool correspondant de formule :

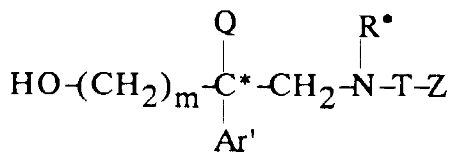 (V*)

L'alcool (V*) est transformé en dérivé méthanesulfonate de formule :

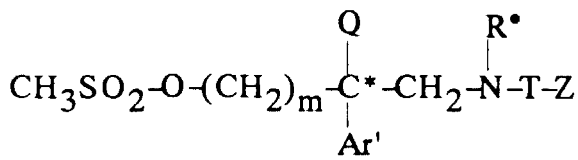 (VI*)

selon les conditions opératoires identiques à celles utilisées pour la préparation des dérivés (VI) ci-dessus.

La substitution du mésylate (VI*) par un groupe de formule :

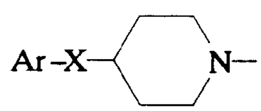 (VII)

selon les conditions décrites pour l'obtention de (I) ci-dessus permet la préparation des dérivés (I*), après déprotection éventuelle, lesquels sont ensuite éventuellement transformés en l'un de leurs sels selon les méthodes classiques de salification ou en un de leurs sels d'ammonium quaternaire.

Les composés de formule (XVII*) sont connus ou peuvent être aisément préparés selon la méthode décrite par G. Helmchen et al., Angew. Chem. Int. Ed. Engl., 1979, 1, 18, 65 ; selon le schéma suivant :

## SCHEMA 3

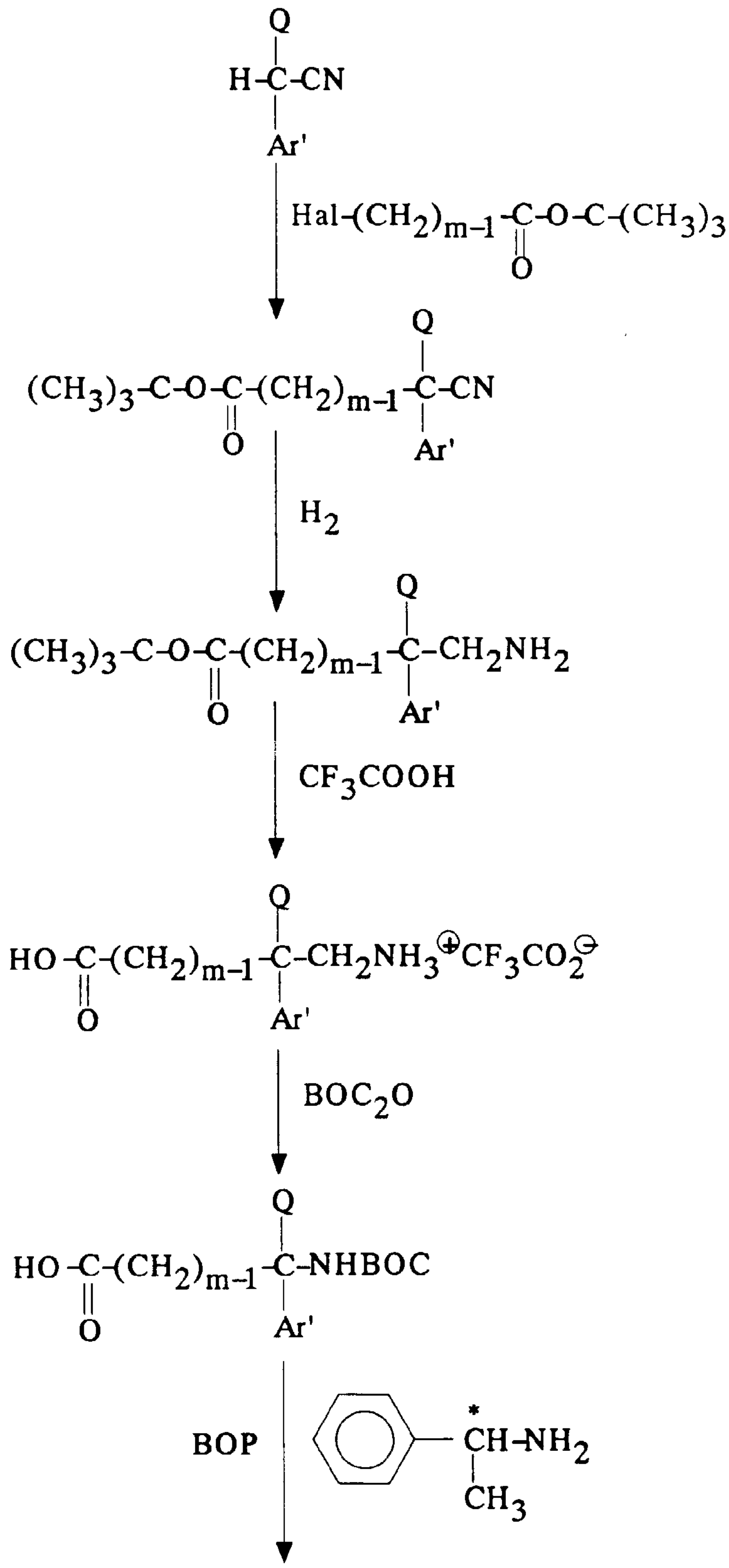


(XVII*, R• = BOC)

Les produits de formule (I*) ainsi obtenus sont isolés, sous forme de base libre ou de sel, selon les techniques conventionnelles.

Lorsque le composé de formule (I*) est obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques conventionnelles. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le 2-naphtalènesulfonate.

A la fin de la réaction, les composés de formule (I*) peuvent être isolés sous forme d'un de leurs sels, par exemple le chlorhydrate ou l'oxalate ou sous forme d'un de leur sel d'ammonium quaternaire ; dans ce cas, s'il est nécessaire la base libre peut être préparée par neutralisation dudit sel avec une base minérale ou organique.

Les composés selon l'invention ont fait l'objet d'essais biochimiques.

Les composés (I) et (I*) et leurs sels ont montré des propriétés antagonistes de la liaison de la substance P dans des essais réalisés sur des membranes de cortex de rat et de cellules lymphoblastiques IM9, selon M.A. Cascieri et al., J. Biol. Chem., 1983, 258, 5158-5164 et D.D. Paya et al., J. Immunol., 1984, 133, 3260-3265.

Les mêmes composés et leurs sels ont montré des propriétés antagonistes de la liaison de la NKA dans des essais réalisés sur des membranes de duodénum de rat, selon L. Bergstom et al., Mol. Pharmacol., 1987, 32 764-771.

Les mêmes composés et leurs sels ont montré des propriétés antagonistes de la liaison de l'élédoïsine dans des essais réalisés sur des membranes de rat selon A.C. Foster et al., Br. J. Pharmacol., 1988, 94, 602-608.

L'élédoïsine est un peptide d'origine batracienne qui est équivalent à la neurokinine B.

Les composés selon l'invention sont des antagonistes de la substance P, de la neurokinine A ou la neurokinine B.

Ainsi, le composé 4 antagonise la liaison de la substance P avec un Ki de 41 nanomolaire, le composé 8 antagonise la liaison de la neurokinine A avec un Ki de 5,5 nanomolaire et le composé 9 antagonise la liaison de l'élédoïsine avec un Ki de 400 nanomolaire.

Les composés de la présente invention sont généralement administrés en unité de dosage. Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ou (I*) ou un de leurs sels pharmaceutiquement acceptables.

Les composés de formule (I) ou (I*) ci-dessus et leurs sels pharmaceutiquement acceptables peuvent être utilisés à des doses journalières de 0,01 à 100 mg par kilo de poids corporel du mammifére à traiter, de préférence à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Dans les compositions pharmaceutiques de la présente invention pour l'administration orale, sublinguale, sous-cutanée, intramusculaire, intraveineuse, transdermique topique ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange le principe actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylènglycol ou le butylèneglycol.

Pour une administration par inhalation on utilise un aérosol contenant par exemple du trioléate de sorbitane ou de l'acide oléique ainsi que du trichlorométhane, du dichlorofluorométhane, du dichlorotétrafluoroéthane ou tout autre gaz propulseur biologiquement compatible.

Le principe actif peut être formulé également sous forme de microcapsules, éventuellement avec un ou plusieurs supports ou additifs.

Les compositions susdites peuvent également renfermer d'autres produits actifs tels que, par exemple, des bronchidilatateurs, des antitussifs ou des antihistaminiques.

Les exemples suivants illustrent l'invention sans toutefois la limiter.

Les points de fusion ou de décomposition des produits, F, ont été mesurés au banc chauffant Koffler. Les spectres de résonnance magnétique nucléaire de $^{13}C$ ont été effectués à 50 MHz dans le diméthylsulfoxyde.

EXEMPLE 1

Chlorhydrate de N[4-(4-phénoxy-1-pipéridinyl)-2-(3,4-dichlorophényl)butyl]-2,4-dichlorobenzamide.

(I) : Ar -X- = 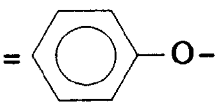 ; m = 2 ; Q = H ;

Ar' = 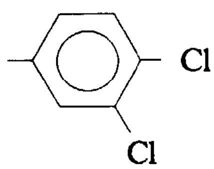 ; R = H ; -T-Z = 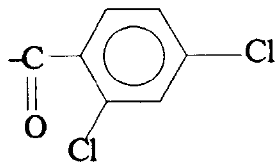

A) Préparation de l'amine :

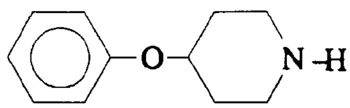

60,6 g de 4-hydroxypipéridine sont dissous dans un mélange de 320 ml de dioxane et 80 ml d'eau. On ajoute alors rapidement 144 g de $BOC_2O$ et le mélange réactionnel est chauffé à 80°C pendant une heure et demie après l'addition. On concentre sous vide, reprend le résidu dans l'éther, lave trois fois à l'eau, décante la phase éthérée, sèche sur $Na_2SO_4$, filtre et concentre sous vide pour obtenir 116 g d'une huile jaunâtre qui est dissoute dans 300 ml d'hexane puis cristallise pour fournir 100 g de cristaux.

F = 68-70°C.

100 g des cristaux préparés précédemment et 55,5 g de triéthylamine sont dissous dans 500 ml de dichlorométhane. On ajoute alors goutte à goutte 60,1 g de chlorure de mésyle en refroidissant dans la glace. A la fin de l'addition, on laisse revenir à température ambiante et abandonne le mélange réactionnel pendant une nuit. Le dichlorométhane est concentré sous vide et le résidu est repris dans l'eau et extrait à l'acétate d'éthyle. Les phases organiques sont lavées à l'eau, puis avec une solution de $NaHCO_3$ à 5 %, puis avec une solution saturée de NaCl et ensuite concentrées sous vide pour fournir des cristaux qui sont recristallisés dans 250 ml d'acétate d'éthyle additionnés de 500 ml d'hexane.

m = 135,2 g

F = 99°C.

0,83 g d'hydrure de sodium à 55 % dans l'huile sont mis en suspension dans 150 ml de diméthylformamide puis on ajoute rapidement 3,67 g de phénol en solution dans 10 ml de diméthylformamide. Le mélange est agité à température ambiante pendant 30 minutes puis on ajoute 8,37 g du produit obtenu précédemment et chauffe le mélange réactionnel à 80°C pendant 4 heures. Le solvant est concentré sous vide, le résidu est repris dans une solution d'hydroxyde de sodium à 10 % et extrait à l'éther. La phase éthérée est lavée successivement avec une solution d'hydroxyde de sodium à 5 % puis avec une solution saturée de NaCl, séchée sur $Na_2SO_4$ et concentrée sous vide. Le résidu obtenu est traité par une solution chauffée à 50-60°C de 30 ml de méthanol, 10 ml d'acide chlorhydrique concentré et

10 ml d'eau pendant une heure puis le mélange est concentré sous vide et recristallisé dans 100 ml d'acétate d'éthyle.
m = 3,44 g.

B) Préparation du 1-(2,4-dichlorobenzoylamino)-2-(3,4-dichlorophényl)-4-mésyloxybutane.

a) 3-(3,4-Dichlorophényl)-1-(2-tétrahydropyranyloxy)-3-cyanopropane.

20 g d'hydrure de sodium à 55-60 % dans l'huile sont mis en suspension dans 200 ml de tétrahydrofurane sec. On ajoute goutte à goutte à 20°C, en 30 minutes, une solution de 85 g de 3,4-dichlorophénylacétonitrile dans 500 ml de tétrahydrofurane puis on agite le mélange réactionnel à température ambiante pendant 2 heures. Le mélange est refroidi à -20°C et on ajoute une solution de 98 g de 1-bromo-2-tétrahydropyranyloxy éthane dans 100 ml de tétrahydrofurane, on laisse revenir le mélange à température ambiante et après 2 heures on ajoute une solution de 50 g de chlorure d'ammonium dans 3 litres d'eau. On extrait avec 1,5 litres d'éther, lave avec une solution saturée de chlorure de sodium, décante, sèche sur $MgSO_4$ et concentre sous vide.

Le résidu est chromatographié sur gel de silice, éluant : dichlorométhane. Les fractions de produit pur sont concentrées sous vide pour fournir 83,6 g d'une huile.

b) 1-Amino-2-(3,4-dichlorophényl)-4-(2-tétrahydropyranyloxy)butane.

83,6 g du nitrile obtenu précédemment sont mis en solution dans 100 ml d'éthanol absolu. On ajoute 350 mg d'ammoniaque concentré puis, sous balayage d'azote on ajoute du nickel de Raney (10 % de la quantité d'amine de départ). On hydrogène ensuite sous atmosphère d'hydrogène à température ambiante et pression ordinaire.

11,9 litres d'hydrogène sont absorbés en 3 heures. Le catalyseur est séparé par filtration sur célite, le filtrat est concentré sous vide, le résidu est repris dans une solution saturée de chlorure de sodium. Après extraction à l'éther et séchage sur $MgSO_4$ on obtient 82,5 g d'une huile.

c) 1-(2,4-Dichlorobenzoylamino)-2-(3,4-dichlorophényl)-4-(2-tétrahydropyranyloxy)-butane.

80 g de l'amine obtenue précédemment sont mis en solution dans 800 ml de dichlorométhane. La solution est refroidie à 0°C, on ajoute 38,4 ml de triéthylamine puis 55 g de chlorure de l'acide 2,4-dichlorobenzoïque. Le mélange réactionnel est alors agité à température ambiante pendant une heure puis lavé à l'eau. La phase organique est décantée, séchée sur $MgSO_4$ et concentrée sous vide pour fournir 120 g d'une huile.

d) 1-(2,4-Dichlorobenzoylamino)-2-(3,4-dichlorophényl)-4-butanol.

120 g du produit obtenu précédemment sont mis en solution dans 1 litre de méthanol en présence de 12 g d'acide paratoluènesulfonique. Le mélange réactionnel est agité pendant 18 heures à température ambiante puis concentré sous vide. Le résidu est repris dans le dichlorométhane et lavé avec une solution à 10 % de carbonate de sodium. La phase organique est décantée et séchée sur $MgSO_4$ pour fournir 106 g d'une huile.

e) 1-(2,4-Dichlorobenzoylamino)-2-(3,4-dichlorophényl)-4-mésyloxybutane.

106 g de l'alcool obtenu précédemment sont mis en solution dans 2 l de dichlorométhane puis on ajoute à la solution refroidie à 0°C, 44 ml de triéthylamine et 24,2 ml de chlorure de mésyle. Le mélange réactionnel est agité à 0°C pendant 45 minutes, lavé trois fois à l'eau glacée, décanté, séché sur $MgSO_4$ et concentré sous vide.

Le résidu est recristallisé de l'éther isopropylique.

m = 95 g.

C) Composé 1

On prépare une solution de 3,6 ml de triéthylamine dans 2 ml de diméthylformamide puis on ajoute lentement 2,1 g de 4-phénoxypipéridine préparé comme précédemment selon A et libéré avec NaOH. On ajoute ensuite à cette solution 2,2 g du méthanesulfonate préparé selon B, étape e, chauffe le mélange réactionnel à 60°C pendant une heure, ajoute 0,1 g de 4-phénoxypipéridine et chauffe encore à 60°C pendant 30 minutes. Le mélange réactionnel est versé dans l'eau, extrait plusieurs fois à l'éther, les phases éthérées sont décantées, séchées sur $Na_2SO_4$, filtrées et concentrées sous vide. Le résidu est chromatographié sur gel de silice éluant : dichlorométhane/méthanol 93/3 (v/v) puis 95/5 (v/v). La concentration des fractions pures fournit 1,9 g du produit attendu sous forme de base ; on prépare alors le chlorhydrate dans l'acétate d'éthyle.

m = 1,5 g
F = 210°C.

EXEMPLE 2

Chlorhydrate de N[4-(4-phénylthio-1-pipéridinyl)-2-(3,4-dichlorophényl)-butyl]-4-fluoro-1-naphtalènecarboxamide.

(I) : Ar -X- = 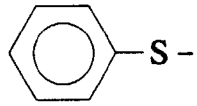 ; m = 2 ; Q = H ;

Ar' = 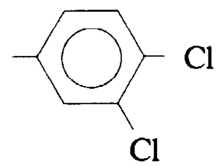 ; R = H ; -T-Z = 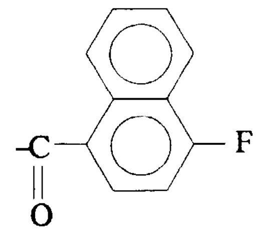

A) Préparation de l'amine :

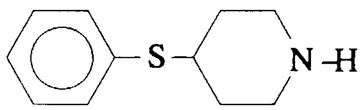

20,2 g de 4-hydroxypipéridine sont mis en solution dans 80 ml de dioxane et 20 ml d'eau. On ajoute rapidement 48 g de $BOC_2O$ et chauffe le mélange réactionnel à reflux pendant une nuit. Les solvants sont concentrés sous vide et le résidu est recristallisé dans l'hexane.

On obtient 30 g de cristaux.

9,0 g du produit préparé précédemment et 5 g de triéthylamine sont dissous dans 60 ml de dichlorométhane puis on ajoute goutte à goutte une solution de 5,4 g de chlorure de mésyle dans 20 ml de dichlorométhane. Le mélange réactionnel est agité pendant deux heures à température ambiante et les solvants sont concentrés sous vide. Le résidu est repris dans l'eau et extrait à l'acétate d'éthyle. Les phases organiques sont séparées et lavées successivement avec une solution de $NaHCO_3$ à 5 % puis avec une solution saturée de NaCl, séchées sur $Na_2SO_4$ et concentrées sous vide. Le résidu est recristallisé dans un mélange acétate d'éthyle/hexane.

On obtient 10,4 g de cristaux.

1,5 g d'hydrure de sodium à 55 % dans l'huile sont mis en suspension dans 150 ml de diméthylformamide puis on ajoute à température ambiante, 4,29 g de thiophénol. Après 30 minutes d'agitation, on ajoute 8,37 g du produit préparé précédemment et laisse le mélange réactionnel pendant une nuit à température ambiante. On concentre le solvant sous vide, reprend le résidu dans une solution d'hydroxyde de sodium et extrait à l'éther. La phase organique est séparée et lavée successivement avec une solution d'hydroxyde de sodium à 5 %, une fois à l'eau puis avec une solution saturée de NaCl, séchée sur $Na_2SO_4$ et concentrée sous vide.

On obtient 8,56 g d'un résidu huileux.

8,5 g du produit obtenu ci-dessus sont chauffés à 40-50°C dans un mélange de 50 ml de méthanol, 20 ml d'acide chlorhydrique concentré et 10 ml d'eau, pendant une heure et demie. Les solvants sont concentrés sous vide et le résidu recristallisé dans l'acétate d'éthyle.

m = 5,42 g
F = 159-161°C.

B) Préparation du 1-(4-fluoro-1-naphtoylamino)-2-(3,4-dichlorophényl)-4-mésyloxy butane.

a) 3-(3,4-Dichlorophényl)-1-(2-tétrahydropyranyloxy)-3-cyanopropane.

20 g d'hydrure de sodium à 55-60 % dans l'huile sont mis en suspension dans 200 ml de tétrahydrofurane sec. On ajoute goutte à goutte à 20°C, en 30 minutes, une solution de 85 g de 3,4-dichlorophénylacétonitrile dans 500 ml de tétrahydrofurane puis on agite le mélange réactionnel à température ambiante pendant 2 heures. Le mélange est refroidi à -20°C et on ajoute une solution de 98 g de 1-bromo-2-tétrahydropyranyloxyéthane dans 100 ml de tétrahydrofurane, on laisse revenir le mélange à température ambiante et après 2 heures on ajoute une solution de 50 g de chlorure d'ammonium dans 3 litres d'eau. On extrait avec 1,5 litres d'éther, lave avec une solution saturée de chlorure de sodium, décante, sèche sur $MgSO_4$ et concentre sous vide.

Le résidu est chromatographié sur gel de silice, éluant : dichlorométhane. Les fractions de produit pur sont concentrés sous vide pour fournir 83,6 g d'une huile.

b) 1-Amino-2-(3,4-dichlorophényl)-4-(2-tétrahydropyranyloxy)butane.

83,6 g du nitrile obtenu précédemment sont mis en solution dans 100 ml d'éthanol absolu. On ajoute 350 ml d'ammoniaque concentré puis, sous balayage d'azote on ajoute du nickel de Raney (10 % de la quantité d'amine de départ). On hydrogène ensuite sous atmosphère d'hydrogène à température ambiante et pression ordinaire.

11,9 litres d'hydrogène sont absorbés en 3 heures. Le catalyseur est séparé par filtration sur célite, le filtrat est concentré sous vide, le résidu est repris dans une solution saturée de chlorure de sodium. Après extraction à l'éther et séchage sur $MgSO_4$ on obtient 82,5 g d'une huile.

c) 2-(3,4-Dichlorophényl)-1-(4-fluoro-1-naphtoylamino)-4-(2-tétrahydropyranyloxy)-butane.

4,8 g d'amine préparée précédemment et 3 ml de triéthylamine sont dissous dans 50 ml de chlorure de méthylène. On ajoute alors goutte à goutte une solution de 5 g de chlorure de l'acide 4-fluoronaphtoïque dans 10 ml de dichlorométhane. Après l'addition, le mélange réactionnel est chauffé à reflux pendant 15 minutes et concentré sous vide. Le résidu est repris dans l'eau et extrait à l'éther. La phase éthérée est séparée et lavée successivement avec une solution de $NaHCO_3$ à 5 % et de NaCl saturée. Après séchage sur $Na_2SO_4$ et évaporation sous vide des solvants, on obtient 7,35 g d'un produit huileux.

d) 2-(3,4-Dichlorophényl)-1-(4-fluoro-1-naphtoylamino)-4-butanol.

A une solution de 13 g du composé obtenu selon l'étape précédente c), dans 80 ml de méthanol, on ajoute 4 ml de résine Amberlyst A® acide, agite le mélange pendant une heure à température ambiante et chauffe à reflux pendant 30 minutes. On sépare la résine par filtration sur célite et concentre le filtrat sous vide.

m = 10,7 g.

e) 2-(3,4-Dichlorophényl)-1-(4-fluoro-1-naphtoylamino)-4-mésyloxybutane.

A une solution de 10,5 g de l'alcool obtenu précédemment dans 100 ml de dichlorométhane on ajoute 4,3 g de triéthylamine puis 3,5 g de chlorure de mésyle. A la fin de l'addition, on lave successivement à l'eau puis avec une solution saturée de NaCl. La phase organique est séparée par décantation, séchée sur $Na_2SO_4$ et concentrée sous vide. L'huile obtenue cristallise dans l'éther.

m = 10,15 g.

C) Composé 2.

2,3 g de l'amine 4-phénylthiopipéridine préparée précédemment (selon A) et libéré avec NaOH et 1,4 ml de triéthylamine sont mis en solution dans 10 ml de diméthylformamide puis on ajoute 2,8 g du mésylate préparé précédemment et chauffe le mélange à 80°C pendant 45 minutes. Le mélange est versé dans l'eau et extrait à l'acétate d'éthyle. La phase organique est séchée sur $Na_2SO_4$ et concentrée sous vide. Le résidu est chromatographié sur gel de silice, éluant : dichlorométhane/méthanol 93/7 (v/v) puis 92/8 (v/v). La concentration des fractions pures fournit une huile qui est dissoute dans l'acétate d'éthyle. L'addition d'éther saturé d'acide chlorhydrique permet la préparation du chlorhydrate qui cristallise.

m = 1 g

F = 211°C.

En procédant selon les exemples 1 ou 2 ci-dessus on prépare les composés décrits dans le tableau 1 ci-dessous.

## TABLEAU 1

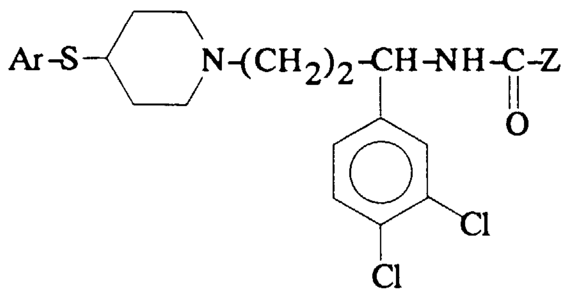

| n° exemple | Ar | Z | F ; °C | Sel |
|---|---|---|---|---|
| 3 | 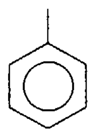 | 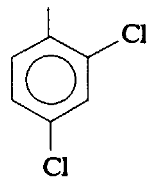 | 112 | HCl |
| 4 | 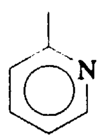 | 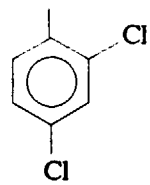 | 178 | 2HCl |
| 5 | 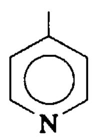 | 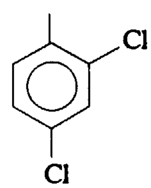 | 220 | 2HCl |
| 6 | 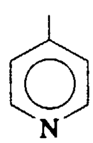 | 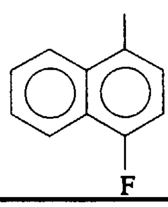 | 198 | 2HCl |

EXEMPLE 7

Dichlorhydrate de N[4-(4-anilino-1-pipéridinyl)-2-(3,4-dichlorophényl)-butyl]-2,4-dichlorobenzamide.

(I) : Ar -X- = 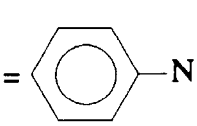 NH-; m = 2 ; Q = H ;

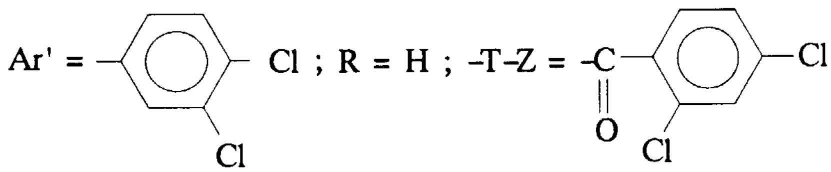

A - L'amine

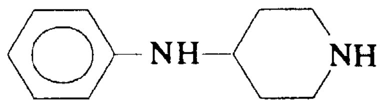

est commerciale

B - Composé 7

de 1-(2,4-dichlorobenzoylamino)-2-(3,4-dichlorophényl)-4-mésyloxybutane 1 g préparé comme précédemment selon l'exemple 1B et 0,8 g de 4-anilinopipéridine(commerciale) sont mis en solution dans 1 ml de diméthylformamide et le mélange réactionnel est chauffé à 60°C pendant une heure. On verse ensuite la solution dans l'eau, extrait à l'acétate d'éthyle, sépare la phase organique qui est lavée à l'eau, séchée sur $NaSO_4$ et concentrée sous vide. Le résidu est purifié par chromatographie sur gel de silice, éluant : dichlorométhane/méthanol 97/3 (v/v).

La concentration des fractions pures fournit un résidu qui est transformé en chlorhydrate et recristallisé dans l'éthanol.

m = 0,25 g

F = 214°C.

EXEMPLE 8

Chlorhydrate de N[4-(N'-4-acétylanilino-1-pipéridinyl)-2-(3,4-dichlorophényl)butyl]-N-méthylbenzamide.

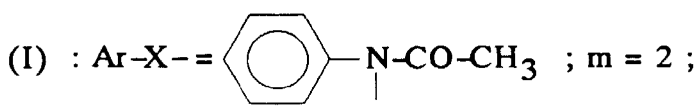

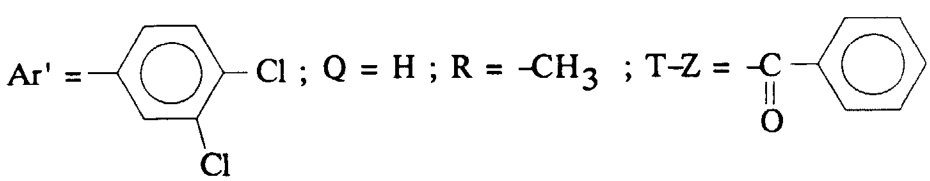

A) 3-(3,4-Dichlorophényl)-1-(2-tétrahydropyranyloxy)-3-cyanopropane.

20 g d'hydrure de sodium à 55-60 % dans l'huile sont mis en suspension dans 200 ml de tétrahydrofurane sec. On ajoute goutte à goutte à 20°C, en 30 minutes, une solution de 85 g de 3,4-dichlorophénylacétonitrile dans 500 ml de tétrahydrofurane puis on agite le mélange réactionnel à température ambiante pendant 2 heures. Le mélange est refroidi à -20°C et on ajoute une solution de 98 g de 1-bromo-2-tétrahydropyranyloxyéthane dans 100 ml de tétrahydrofurane, on laisse revenir le mélange à température ambiante et après 2 heures on ajoute une solution de 50 g de chlorure d'ammonium dans 3 litres d'eau. On extrait avec 1,5 litres d'éther, lave avec une solution saturée de chlorure de sodium, décante, sèche sur $MgSO_4$ et concentre sous vide.

Le résidu est chromatographié sur gel de silice, éluant : dichlorométhane. Les fractions de produit pur sont concentrées sous vide pour fournir 83,6 g d'une huile.

B) 1-Amino-2-(3,4-dichlorophényl)-4-(2-tétrahydropyranyloxy)butane.

83,6 g du nitrile obtenu précédemment sont mis en solution dans 100 ml d'éthanol absolu. On ajoute 350 ml d'ammoniaque concentré puis sous balayage d'azote on ajoute du nickel de Raney (10 % de la quantité d'amine de départ). On hydrogène ensuite sous atmosphère d'hydrogène à température ambiante et pression ordinaire.

11,9 litres d'hydrogène sont absorbés en 3 heures. Le catalyseur est séparé par filtration sur célite, le filtrat est concentré sous vide, le résidu est repris dans une solution saturée de chlorure de sodium Après extraction à l'éther et séchage sur $MgSO_4$ on obtient 82,5 g d'une huile.

C) 1-Ethoxycarboxamido-2-(3,4-dichlorophényl)-4-(2-tétrahydropyranyloxy)butane.

A 31,8 g de produit précédemment obtenu en solution dans 150 ml de dichlorométhane on ajoute 10,1 g de triéthylamine, puis 10,8 g de chloroformiate d'éthyle. On agite une demi-heure à température ambiante, lave à l'eau, sèche sur sulfate de sodium et évapore à sec.

D) 1-Méthylamino-2-(3,4-dichlorophényl)-4-(2-tétrahydropyranyloxy)butane.

L'huile précédemment obtenue en solution dans 150 ml de tétrahydrofuranne est ajoutée à une suspension de 7,6 g d'hydrure de lithium aluminium dans 100 ml de tétrahydrofuranne à reflux. Après deux heures de reflux on refroidit, ajoute 30 ml de NaOH 5N, filtre le précipité et évapore la solution.

E) N-1-méthylbenzoylamino-2-(3,4-dichlorophényl)-4-(2-tétrahydropyranyloxy)butane.

14,05 g de chlorure de benzoyle en solution dans 50 ml de dichlorométhane sont ajoutés goutte à goutte à une solution du produit précédemment obtenu et de 10,1 g de triéthylamine dans 150 ml de dichlorométhane. On agite une demi heure à température ambiante, évapore à sec, reprend le résidu à l'éther, lave à l'eau, sèche sur sulfate de sodium et évapore à sec. On purifie par chromatographie sur gel de silice, éluant : dichlorométhane/acétate d'éthyle 9/1 (v/v).

On obtient 28,5 g d'une huile incolore.

F) N-1-méthylbenzoylamino-2-(3,4-dichlorophényl)-4-hydroxybutane.

A une solution de 21,7 g du produit précédemment obtenu dans 150 ml de méthanol on ajoute 15 ml d'une solution d'éther saturé en acide chlorhydrique, on agite une demi-heure à température ambiante, évapore à sec et cristallise le produit dans l'éther.

On obtient ainsi 16,9 g.

F = 137-139°C.

G) N-1-méthylbenzoylamino-2-(3,4-dichlorophényl)-4-mésyloxybutane.

4,6 g de chlorure de mésyle en solution dans 25 ml de dichlorométhane sont ajoutés goutte à goutte à 14 g du produit précédemment obtenu et 4 g de triéthylamine en solution dans 100 ml de dichlorométhane. On agite une heure à température ambiante, évapore à sec, reprend à l'acétate d'éthyle, lave à l'éther.

On obtient ainsi 15,4 g.

F = 100-102°C.

H) Composé 8.

A 2 g de N-4-acétylanilinopipéridine on ajoute 1 g du produit obtenu précédemment, puis on dissout le mélange dans 5 ml de diméthylformamide. Le mélange réactionnel est chauffé à 80°C pendant 2 heures puis on ajoute de la glace, extrait à l'éther, lave la phase éthérée à l'eau, sèche sur $MgSO_4$ et concentre sous vide. Le résidu est chromatographié sur gel de silice, éluant : dichlorométhane/méthanol 92/8 (v/v).

La concentration des fractions pures fournit un résidu qui est repris dans le dichlorométhane puis on additionne de l'éther saturé d'acide chlorhydrique et sépare le chlorhydrate par filtration.

m = 0,92 g

F = 108°C.

En procédant selon les exemples 7 et 8 ci-dessus on prépare les composés 9, 10 et 10 décrits dans le tableau 2 ci-dessous.

## TABLEAU 2

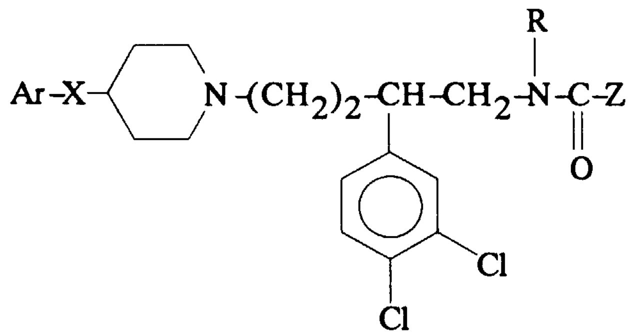

| n° exemple | Z | Ar–X– | R | F ; °C | Sel |
|---|---|---|---|---|---|
| 9 | 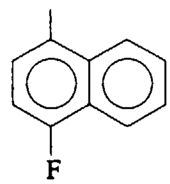 | 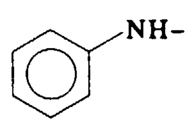 | H | 175 | 2HCl |
| 10 | 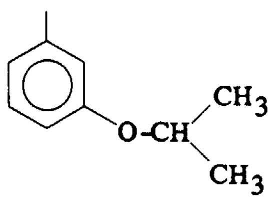 | 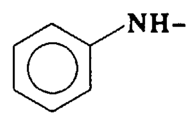 | $-CH_3$ | 205–207 | 2HCl |
| 11 | 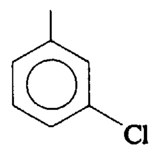 | 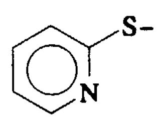 | $-CH_3$ | 105 | 2HCl |

EXEMPLE 12

Dichlorhydrate de N-[4-(4-(1-méthyl-2-imidazolylthio)-1-pipéridinyl)-2-naphtylbutyl]-2,4-diméthoxybenzamide.

(I) : Ar -X- = 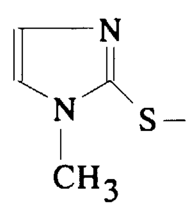 ; m = 2 ; Q = H ;

Ar' = 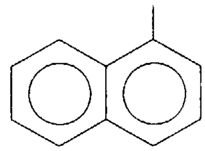 ; R = H ; -T-Z = 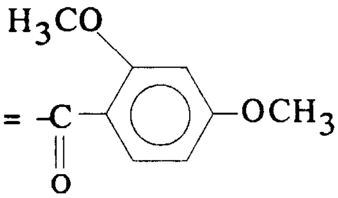

A - Préparation de l'amine

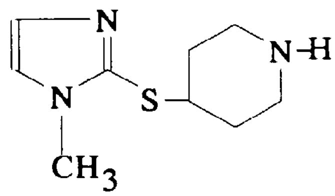

En procédant selon l'exemple 1A et en remplaçant le phénol par le 1-méthyl-2-mercaptoimidazole, on prépare l'amine ci-dessus.

F = 209°C (chlorhydrate).

B - Composé 12.

2,8 g de 1-(2,4-diméthoxybenzoylamino)-2-(1-naphtyl)-4-mésyloxybutane préparé en procédant selon l'exemple 1 sont chauffés à 80°C pendant une heure et demie en présence de 1,35 g de l'amine préparée ci-dessus et de 2,02 g de triéthylamine dans 8 ml de diméthylformamide. On laisse ensuite refroidir le mélange réactionnel puis ajoute de l'eau glacée. Le précipité obtenu est séparé par filtration, et dissout dans le dichlorométhane. On lave à l'eau puis la phase organique est séchée sur $MgSO_4$ et concentrée sous vide. Le résidu est chromatographié sur gel de silice, éluant : dichlorométhane/méthanol 100/2 (v/v).

La concentration des produits purs fournit un résidu qui est repris dans le dichlorométhane puis on additionne de l'éther chlorhydrique. Le chlorhydrate est séparé par filtration.

m = 0,25 g

F = 146-150°C.

En procédant selon l'exemple 12 ci-dessus, on prépare les composés décrit dans le tableau 3 ci-dessous.

## TABLEAU 3

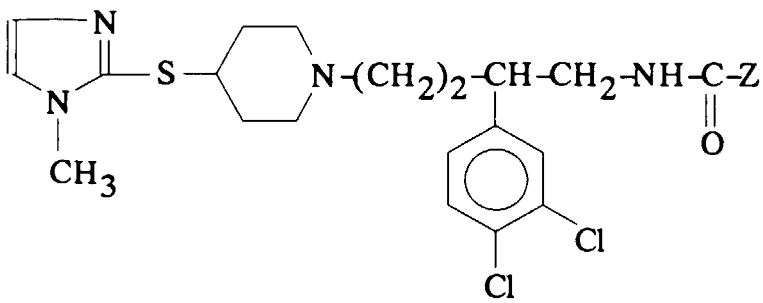

| n° exemple | Z | F ; °C | Sel |
|---|---|---|---|
| 13 | 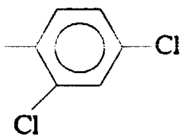 | 215 | 2HCl |
| 14 | 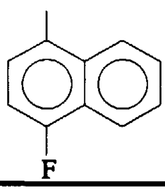 | 186 | 2HCl |

EXEMPLE 15 Dichlorhydrate de N-méthyl[4-(4-anilino-1-pipéridinyl)-2-(3,4-dichloro-phényl)butyl]-4-fluoro-1-naphtalènecarboxamide (-).

(I) : Ar -X- = 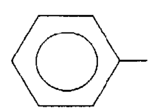 NH- ; m = 2 ; Q = H ;

Ar' = 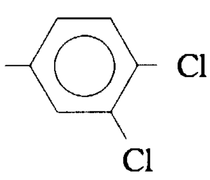 ; R = $CH_3$ ; -T-Z = 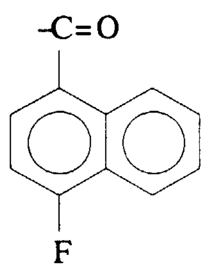

L'énantiomère (-) du composé ci-dessus est préparé à partir de l'aminoalcool racémique duquel on sépare les énantiomères selon la méthode décrite dans la demande de brevet EP-A-428434, comme indiqué ci-dessous.

- Enantiomère 1-amino-2-(3,4-dichlorophényl)-4-butanol (+) .

A 59,65 g d'acide D (-) tartrique en solution dans 2 litres de méthanol chauffés au reflux, on ajoute 93 g de l'aminoalcool racémique, en solution dans 300 ml de méthanol. On laisse revenir à température ambiante, filtre les cristaux, lave au méthanol et sèche sous vide à 50°C sur $P_2O_5$.

m = 64,8 g

$[\alpha]_D^{20}$ = -5,2°(c = 1 dans l'eau).

On recristallise ensuite dans 2,96 l de méthanol, filtre les cristaux, les lave au méthanol et sèche sous vide à 50°C sur $P_2O_5$.

m = 45,3 g

$[\alpha]_D^{20}$ = -4,5°(c = 1 dans l'eau).

F = 201°C.

Le D (-) tartrate est repris dans 250 ml d'eau, on alcalinise avec une solution concentrée d'hydroxyde de sodium et extrait avec 3 fois 200 ml de dichlorométhane, lave avec une solution saturée de chlorure de sodium, décante, sèche sur $MgSO_4$, filtre et concentre sous vide. Le résidu est repris dans l'éther isopropylique, le mélange est agité pendant une heure à température ambiante, les cristaux sont filtrés et lavés à l'éther isopropylique.

m = 24,7 g

$[\alpha]_D^{20}$ = +9,0° (c = 1 dans le méthanol).

F = 79-80°C.

- Enantiomère 1-amino-2-(3,4-dichlorophényl)-4-butanol (-).

En procédant comme précédemment et en utilisant l'acide L (+) tartrique on obtient l'énantiomère (-).

$[\alpha]_D^{20}$ = -9,2· (c = 1 dans le méthanol).

F = 79-80·C.

A) 1-Amino-2-(3,4-dichlorophényl)-4-(2-tétrahydropyranyloxy)butane.

12,50 g de 1-amino-2-(3,4-dichlorophényl)-4-butanol (+) sont mis en solution dans un mélange de 150 ml de dichlorométhane et 50 ml de DMF. On ajoute de l'éther saturé d'acide chlorhydrique jusqu'à pH = 12 puis 5,39 g de tétrahydropyrane et on chauffe le mélange réactionnel à reflux pendant une heure. On concentre les solvants sous vide, reprend le résidu dans l'éther et sépare le précipité par filtration. m = 15,5 g.

B) 2-(3,4-Dichlorophényl)-4-(2-tétrahydropyranyloxy)-4-fluoro-1-naphtalènecarboxamide.

15,5 g du produit préparé précédemment sont mis en solution dans 100 ml de dichlorométhane en présence de 13,3 g de triéthylamine. On ajoute 10,6 g du chlorure de l'acide 4-fluoronaphtoïque en solution dans 10 ml de dichlorométhane puis on laisse le mélange réactionnel pendant 3 heures à température ambiante sous agitation. On concentre les solvants sous vide, reprend le résidu dans l'eau, extrait à l'acétate d'éthyle et lave successivement avec une solution NaOH 10 %, à l'eau puis avec une solution saturée en NaCl. Les phases organiques sont séchées sur $MgSO_4$ et concentrées sous vide.

m = 21 g.

C) N-méthyl[2-(3,4-dichlorophényl)-4-(2-tétrahydropyranyloxy)butane]-4-fluoro-1-naphtalènecarboxamide.

1,56 g de NaH à 55 % sont mis en suspension dans 60 ml de DMF puis on ajoute lentement 21,3 g du produit préparé précédemment en solution dans 120 ml de DMF. Le mélange est agité pendant 15 minutes puis on ajoute, goutte à goutte 12,3 g d'iodure de méthyle en solution dans 20 ml de DMF. Le mélange réactionnel est agité à température ambiante pendant une demi-heure puis concentré sous vide. Le résidu est repris dans l'eau, extrait à l'éther, puis lavé à l'eau puis avec une solution saturée de NaCl. La phase éthérée est séchée sur $MgSO_4$ et concentrée sous vide.

m = 18,6.

D) N-méthyl[2-(3,4-dichlorophényl)-4-butanol]-4-fluoro-1-naphtalènecarboxamide.

18,57 g du produit préparé précédemment sont mis en solution dans 300 ml de méthanol puis on ajoute 2 ml d'éther saturé d'acide chlorhydrique et chauffe le mélange réactionnel à reflux pendant 5 heures. On concentre les solvants sous vide, reprend le résidu dans une solution HCl 2N, extrait à l'éther et lave successivement avec de l'eau, une solution de $NaHCO_3$ 5 %, à l'eau puis avec une solution saturée de NaCl. La phase éthérée est concentrée sous

vide et le résidu est chromatographié sur gel de silice, éluant : $CH_2Cl_2/CH_3OH$ 97/3 (v/v). Les fractions de produit pur sont concentrées sous vide.

m = 9,31 g

$[\alpha]_D^{20}$ = -31,8° (c = 1 dans le méthanol).

F = 125-127°C.

EXEMPLE 16

Iodure de 4-(α-méthylanilino)-N(a)-méthyl[2-(3,4-dichlorophényl)-4-(4-fluoro-1-naphtalènecarboxamino)N'-méthylbutyl]pipéridinium (-).

(I) : Ar-X = 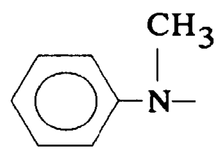 ; Q' = $CH_3$ ; $A^{\ominus} = I^{\ominus}$ ; Q = H ;

Ar' = 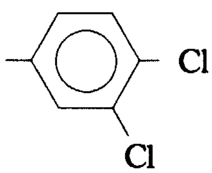 ; R = $CH_3$ ; -T-Z = 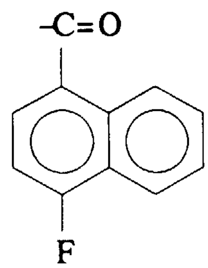

2,41 g du composé préparé précédemment selon l'exemple 15, sous forme de base libre, 24 ml d'iodure de méthyle et 1 ml d'acétone sont laissés sous agitation à température ambiante pendant 24 heures.

Le mélange réactionnel est concentré sous vide et le résidu est chromatographié sur gel de silice, éluant : $CH_2Cl_2/CH_3OH$ 97/3 (v/v).

Le produit élué en premier correspond à celui dont le méthyle sur l'azote de la pipéridine est en position axiale. La concentration de la fraction correspondante fourni un résidu qui précipite dans l'éther.

m = 1,11 g

F = 152-154°C

$[\alpha]_D^{20}$ = -28,3° (c = 1 dans le méthanol).

EXEMPLE 17

Iodure de 4-(α-méthylanilino)-N(a)-méthyl[2-(3,4-dichlorophényl)-4-(3-isopropoxybenzamido)-N'-méthylbutyl]pipéridinium.

(I) : Ar-X = 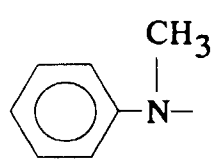 ; Q' = $CH_3$ ; $A^{\ominus} = I^{\ominus}$ ; Q = H ;

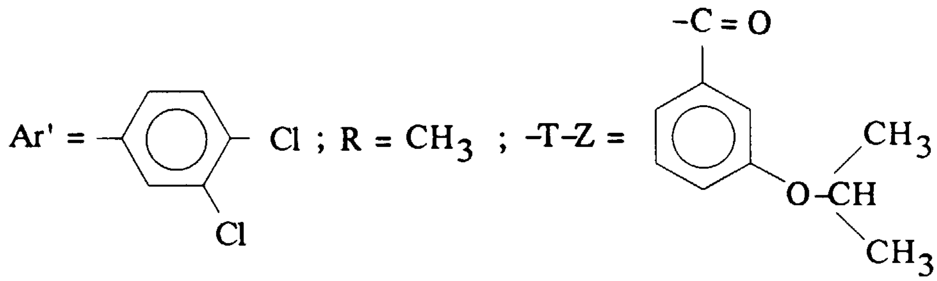

En procédant selon l'exemple 16, à partir du composé décrit à l'exemple 10, sous forme de base libre, on prépare le composé ci-dessus.

F = 131-133°C.

Spectre de RMN $^{13}C$ :

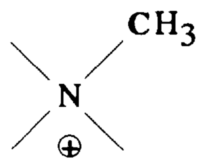

axial : $\delta$ = 68 ppm

**Revendications**

**Revendications pour les Etats contractants suivants : AT, BE, CH, LI, DE, DK, FR, GB, IT, LU, NL, PT, SE**

**1.** Composés de formule :

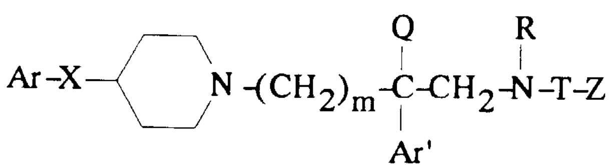

(I)

dans laquelle :

- m est égal à 2 ou 3 ;
- Ar représente un phényle non substitué ou substitué une ou plusieurs fois par un atome d'halogène, par un alkyle en $C_1$-$C_3$, par un trifluorométhyle, par un alcoxy dans lequel l'alkyle est en $C_1$-$C_3$, par un hydroxyle ou par un méthylènedioxy ; un groupe thiényle, pyridyle, imidazolyle substitué ou non par un alkyle en $C_1$-$C_3$ ;
- Ar' représente un groupe phényle non substitué, mono ou di-substitué par un atome d'halogène, par un alkyle en $C_1$-$C_3$, par un trifluorométhyle, par un alcoxy dans lequel l'alkyle est en $C_1$-$C_3$, par hydroxyle ou par un méthylènedioxy ; un groupe thiényle ; un groupe imidazolyle ou un groupe benzothiényle non substitués ou substitués par un halogène ; un groupe naphtyle non substitué ou substitué par un halogène ; un groupe biphényle ; un indolyle non substitué ou substitué sur l'azote par un groupe benzyle ;
- X représente un atome d'oxygène, un atome de soufre, un groupe -NH-, un groupe

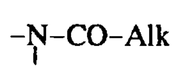

ou un groupe

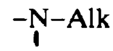

dans lesquels Alk est un groupe alkyle en $C_1$-$C_3$ ;

- Q représente l'hydrogène, un groupe alkyle en $C_1$-$C_4$ ou un groupe aminoalkyle de formule $-(CH_2)_q$-Am', où q est 2 ou 3 et Am' est un groupe pipéridino, benzyl-4 pipéridino ou dialkylamino, chaque alkyle pouvant contenir de 1 à 4 atomes de carbone ;
- R représente l'hydrogène, un groupe méthyle ou un groupe $(CH_2)_n$-L, où n est un nombre entier de 2 à 6 et L est l'hydrogène ou un groupe amino ;
- T représente un groupe choisi parmi :

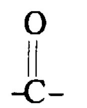 et 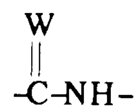

W étant un atome d'oxygène ou de soufre, et

- Z représente soit M ou OM lorsque T représente :

  le groupe

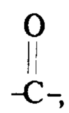

  soit M lorsque T représente le groupe

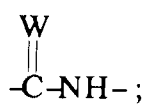

  M représente l'hydrogène ou un alkyle droit ou ramifié en $C_1$-$C_6$ ; un α-hydroxybenzyle, un α-alkylbenzyle ou un phénylalkyle dans lesquels le groupe alkyle comprend de 1 à 3 atomes de carbone, non substitué, mono ou polysubstitué sur le cycle aromatique par un halogène, un hydroxy, un alcoxy de 1 à 4 atomes de carbone, un alkyle de 1 à 4 atomes de carbone ; un pyridylalkyle dans lequel le groupe alkyle comprend de 1 à 3 atomes de carbone ; un naphtylalkyle dans lequel le groupe alkyle comprend de 1 à 3 atomes de carbone ; un pyridylthioalkyle dans lequel le groupe alkyle comprend de 1 à 3 atomes de carbone ; un styryle ; un (méthyl-1) imidazolyl-2 thioalkyle dans lequel le groupe alkyle comprend de 1 à 3 atomes de carbone ; un oxo-1 phénylindan-3 yle-2 ;

  un groupe phényle non substitué ou substitué par un ou plusieurs substituants choisis parmi halogène, CN, OH, $NH_2$, NH-CO-$NH_2$, $NO_2$, $CONH_2$, $CF_3$, alkyle en $C_1$-$C_{10}$, alcényle contenant 2 à 10 atomes de carbone , alcynyle contenant 2 à 10 atomes de carbone, cycloalkyle contenant 3 à 8 atomes de carbone, bicycloalkyle contenant 4 à 11 atomes de carbone, hydroxyalkyle contenant 1 à 5 atomes de carbone, alcoxy contenant 1 à 10 atomes de carbone, alcoxyalkyle contenant 2 à 10 atomes de carbone, alcoxyalcoxyalkyle contenant 3 à 10 atomes de carbone, alcoxyalcoxy contenant 2 à 10 atomes de carbone, alcényloxy contenant 2 à 10 atomes de carbone, alcényloxyalkyle contenant 3 à 10 atomes de carbone, alcynyloxy contenant 2 à 10 atomes de carbone, alcynyloxyalkyle contenant 3 à 10 atomes de carbone, cycloalcoxy contenant 3 à 8 atomes de carbone, alkylthio contenant 1 à 10 atomes de carbone, alkylthioalkyle contenant 2 à 10 atomes de carbone, acylamino contenant 1 à 7 atomes de carbone, acylaminoalkyle contenant 2 à 8 atomes de carbone, acyloxy contenant 1 à 6 atomes de carbone, alcoxycarbonyle contenant 2 à 5 atomes de carbon, cycloalcoxycarbonyle contenant 4 à 8 atomes de carbone, alkylaminocarbonylamino contenant 2 à 4 atomes de carbone, dialkylaminocarbonylamino contenant 3 à 7 atomes de carbone, cycloalkylaminocarbonylamino contenant 4 à 8 atomes de carbone, alkylaminocarbonylaminoalkyle contenant 3 à 9 atomes de carbone, dialkylaminocarbonylaminoalkyle contenant 4 à 11 atomes de carbone, cycloalkylaminocarbonylaminoalkyl contenant de 5 à12 atomes de carbone, alcoxycarbonylaminoalkyle contenant 3 à 12 atomes de carbone, cycloalcoxycarbonylaminoalkyle contenant 5 à 12 atomes de carbone, carbamoylalkyle contenant 2 à 5 atomes de carbone, alkylaminocarbonylalkyle contenant 3 à 9 atomes de carbone, dialkylaminocarbonylalkyle contenant 4 à 11 atomes de carbone, (1-pyrrolidino)carbonylméthyle, (1-pipéridino)carbonyl-méthyle, (1-pipéridino)carbonyléthyle, cycloalkylaminocarbonylalkyle contenant de 5 à 12 atomes de carbone, alkylaminocarbonylalcoxy contenant 3 à 10 atomes de carbone, dialkylaminocarbonylalcoxy contenant 4 à 10 atomes de carbone, (pipéridinyl-1)carbonylméthoxy, cycloalkylaminocarbonylalcoxy contenant 5 à 11 atomes de carbone, (1-pyrrolidino)-carbonylamino, (1-pipéridino)-carbonylamino, (1-pyrrolidino)carbonylaminoéthyle, (1-pipéridino)-carbonylaminoéthyle;

un groupe benzoyle ou un groupe phénylthioalkyle dans lequel l'alkyle est en $C_1$-$C_3$, lesdits groupes pouvant être non substitués ou mono ou disubstitués par un halogène ou par un alcoxy en $C_1$-$C_4$; un groupe naphtyle ou indényle, dont une ou plusieurs liaisons peuvent être hydrogénées, lesdits groupes pouvant être non substitués ou substitués une ou plusieurs fois par un halogène, un groupe alkyle, phényle, cyano, hydroxyalkyle, hydroxy, oxo, alkylcarbonylamino, alcoxycarbonyle, thioalkyle dans lesquels les alkyles sont en $C_1$-$C_4$ ;

un groupe pyridyle, thiadiazolyle, indolyle, indazolyle, imidazolyle, benzimidazolyle, quinolyle, benzotriazolyle, benzofurannyle, benzothiényle, benzothiazolyle, benzisothiazolyle, isoquinolyle, benzoxazolyle, benzisoxazolyle, benzoxazinyle, benzodioxinyle, isoxazolyle, benzopyranyle, thiazolyle, thiényle, furyle, pyrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle, dont une ou plusieurs doubles liaisons peuvent être hydrogénées, lesdits groupes pouvant être non substitués ou substitués une ou plusieurs fois par un groupe alkyle, phényle, cyano, hydroxyalkyle, hydroxy, alkylcarbonylamino, alcoxycarbonyle, thioalkyle dans lesquels les alkyles sont en $C_1$-$C_4$,

ou un de leurs sels avec des acides minéraux ou organiques ou un de leurs sels d'ammonium quaternaire formés avec l'azote de la pipéridine.

**2.** Composés selon la revendication 1, sous forme optiquement pure, de formule :

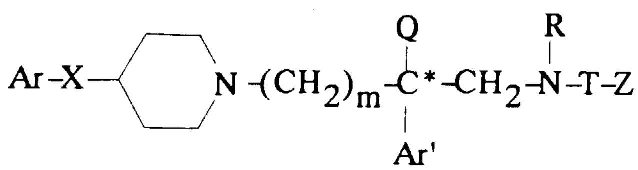

(I*)

dans laquelle :

- "*" signifie que l'atome de carbone ainsi marqué a une configuration absolue (+) ou (-) déterminée ;
- m, Ar et Ar', X, Q, R, T et Z sont tels que définis dans la revendication 1.

ou un de leurs sels avec des acides minéraux ou organiques ou un de leurs sels d'ammonium formés avec l'azote de la pipéridine.

**3.** Composés selon l'une des revendications 1 ou 2, caractérisés en ce qu'ils sont sous la forme d'un sel d'ammonium quaternaire, le groupe de formule

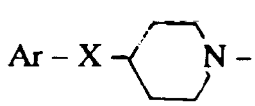

étant alors représenté par le groupe :

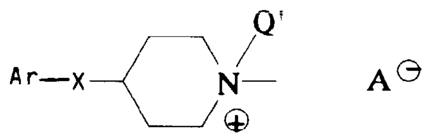

dans laquelle

- . Q' représente un groupe alkyle en $C_1$-$C_6$ ou un groupe benzyle et
- . $A^{\ominus}$ représente un anion choisi parmi chlorure, bromure, iodure, acétate, méthanesulfonate ou paratoluènesulfonate.

**4.** Composé selon la revendication 1, qui est le N-[2-(3,4-Dichlorophényl)4-(4-(2-pyridylthio)-1-pipéridinyl)butyl]-2,4-dichlorobenzamide ou un de ses sels pharmaceutiquement acceptables.

**5.** Composé selon la revendication 1, qui est le N-[4-(N'-4-acétylanilino-1-pipéridinyl)-2-(3,4-dichlorophényl)-butyl]-N-méthylbenzamide ou un de ses sels pharmaceutiquement acceptables.

**6.** Composé selon la revendication 1, qui est le N-[4-(4-(1-méthyl-2-imidazolylthio)-1-pipéridinyl)-2-naphtyl-butyl]-2,4-diméthoxybenzamide ou un de ses sels pharmaceutiquement acceptables.

**7.** Procédé pour la préparation de composés de formule (I) selon la revendication 1, caractérisé en ce que

a) on traite une amine libre de formule :

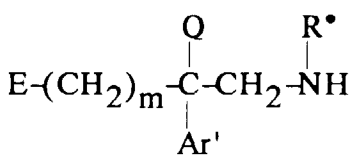

(II)

dans laquelle m, Ar' et Q sont tels que définis précédemment dans la revendication 1 ; R° représente d'hydrogène, un groupe méthyle ou un groupe $(CH_2)_n$-L°, où n est tel que défini précédemment dans la revendication 1 et L° est l'hydrogène ou un groupe amino protégé par un groupe N-protecteur hydrolysable en milieu acide ; et E représente un groupe hydroxy, méthanesulfonyloxy, tétrahydropyranyl-2 oxy, ou un groupe :

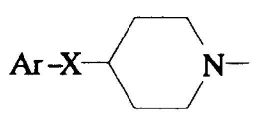

où Ar et X sont tels que définis dans la revendication 1;

- soit avec un dérivé fonctionnel d'un acide de formule :

HO-CO-Z (III)

dans laquelle Z est tel que défini dans la revendication 1, lorsqu'on doit préparer un composé de formule (I) où T est -CO-,

- soit avec un iso(thio)cyanate de formule :

W = C = N-Z (III')

dans laquelle W et Z sont tels que définis dans la revendication 1, lorsqu'on doit préparer un composé

de formule (I) où T est -C(W)-NH-, pour former le composé de formule :

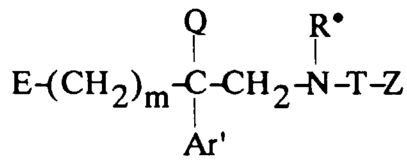

(IV)

b) puis, lorsque E représente le tétrahydropyranyloxy, on élimine le groupe tétrahydropyranyle par hydrolyse acide, l'hydrolyse pouvant alternativement avoir lieu à l'étape (a) sur l'amine de départ de formule (II),
c) lorsque E représente un groupe hydroxy ou un groupe tétrahydropyranyloxy qui a été hydrolysé, on traite l'alcanolarnine N-substituée ainsi obtenue de formule :

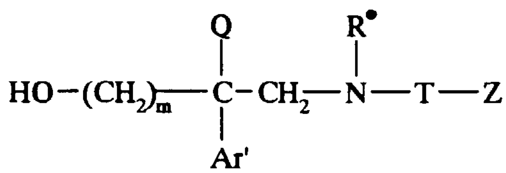

(V)

avec le chlorure de méthanesulfonyle,
d) on fait réagir le mésylate ainsi obtenu à l'étape c) ou directement à l'issue de l'étape a) lorsque E représente un groupe méthanesulfonyloxy de formule :

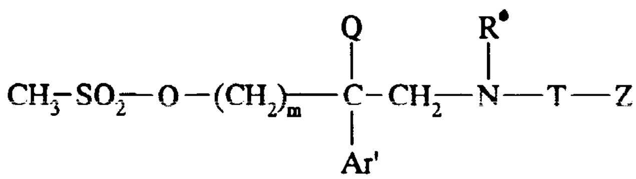

(VI)

avec une amine secondaire de formule :

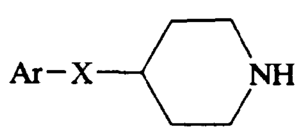

(VII)

dans laquelle Ar et X sont tels que définis dans la revendication 1,
e) on élimine les groupes N-protecteurs éventuels et on transforme éventuellement le produit ainsi obtenu à l'étape d) ou directement à l'issue de l'étape a) lorsque E représente un groupe

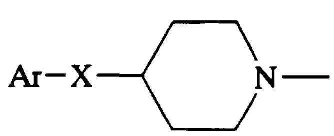

en un de ses sels.

**8.** Procédé stéréosélectif pour la préparation de composés optiquement purs de formule (I*) selon la revendication 2, caractérisé en ce que on traite un composé de formule :

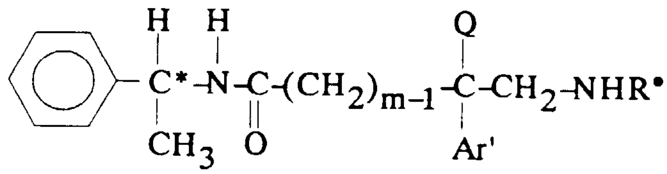 (XVII*)

dans un solvant, en milieu acide, pour fournir l'aminoacide de formule :

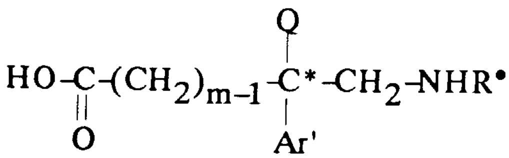 (XVIII*)

qui est estérifié dans un alcanol AlkOH, dans lequel Alk est un alkyle de 1 à 4 atomes de carbone, en milieu acide, puis on traite l'ester correspondant de formule :

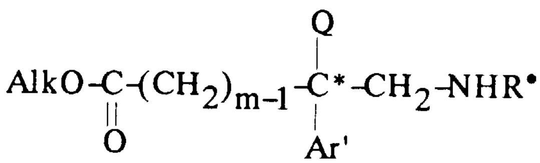 (XIX*)

dans lequel Alk est tel que défini ci-dessus ; Q, Ar', et m sont tels que définis dans la revendication 1 et R• est tel que défini dans la revendication 7,

- soit avec un dérivé fonctionnel d'un acide de formule :

HO-CO-Z (III)

- soit avec un iso(thio)cyanate de formule :

W=C=N-Z (III')

Z et W étant tels que définis dans la revendication 1, on soumet l'ester ainsi obtenu de formule :

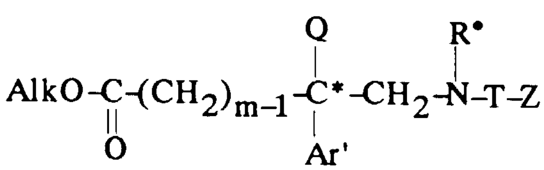 (XX*)

à l'action d'un agent réducteur et on transforme l'alcool correspondant de formule :

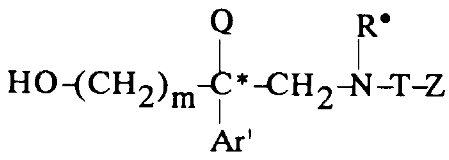 (V*)

en son ester méthanesulfonate de formule :

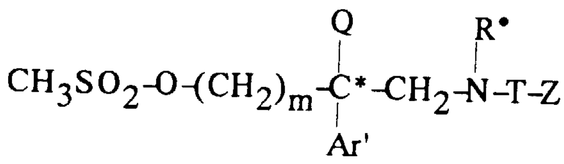 (VI*)

qui, par traitement avec une amine de formule :

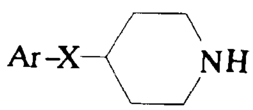 (VII)

dans laquelle Ar et X sont tels que définis dans la revendication 1, donne le composé de formule (I*).

**9.** Composé de formule :

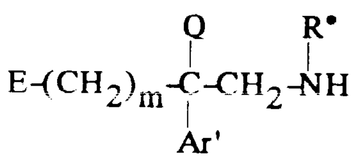 (II)

dans laquelle E représente le groupe de formule :

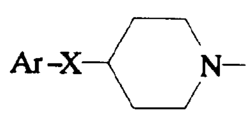

m, Q, Ar', Ar et X sont tels que définis dans la revendication 1 et R• représente l'hydrogène, un groupe méthyle ou un groupe $(CH_2)_nL^\bullet$ où n est tel que défini dans la revendication 1 et L• est l'hydrogène ou un groupe amino protégé.

**10.** Composition pharmaceutique contenant, en tant que principe actif, un composé de formule (I) ou (I*) selon l'une des revendications 1 ou 2.

**11.** Composition pharmaceutique selon la revendication 10, sous forme d'unité de dosage, dans laquelle le principe actif est mélangé à au moins un excipient pharmaceutique.

**12.** Composition selon la revendication 11 contenant de 2,5 à 1000 mg de principe actif.

**13.** Composés de formule (I) selon l'une quelconque des revendications 1 à 3, dans laquelle Z représente un groupe phényle, un groupe benzyle, un groupe benzoyle, un groupe phénylthioalkyle dans lequel l'alkyle est en $C_1$-$C_3$,

lesdits groupes étant non substitués, mono- ou di-substitués par un halogène ou par un alcoxy en $C_1$-$C_4$.

**14.** Composés de formule (I) selon l'une quelconque des revendications 1 à 3, dans laquelle :

- Ar représente un phényle substitué une ou plusieurs fois par un atome de chlore ou de fluor, et/ou
- Ar' représente un groupe phényle, mono ou di-substitué par un atome de chlore ou de fluor ; un groupe imidazolyle ou un groupe benzothiényle substitué par un atome de chlore ou de fluor ; un groupe naphtyle substitué par un atome de fluor.

**Revendications pour l'Etat contractant suivant : ES**

**1.** Procédé pour la préparation de composés de formule :

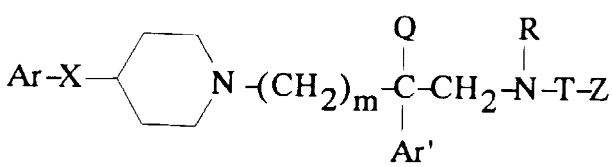

(I)

dans laquelle :

- m est égal à 2 ou 3 ;
- Ar représente un phényle non substitué ou substitué une ou plusieurs fois par un atome d'halogène, par un alkyle en $C_1$-$C_3$, par un trifluorométhyle, par un alcoxy dans lequel l'alkyle est en $C_1$-$C_3$, par un hydroxyle ou par un méthylènedioxy ; un groupe thiényle, pyridyle, imidazolyle substitué ou non par un alkyle en $C_1$-$C_3$ ;
- Ar' représente un groupe phényle non substitué, mono ou di-substitué par un atome d'halogène, par un alkyle en $C_1$-$C_3$, par un trifluorométhyle, par un alcoxy dans lequel l'alkyle est en $C_1$-$C_3$, par hydroxyle ou par un méthylènedioxy ; un groupe thiényle ; un groupe imidazolyle ou un groupe benzothiényle non substitués ou substitués par un halogène ; un groupe naphtyle non substitué ou substitué par un halogène ; un groupe biphényle ; un indolyle non substitué ou substitué sur l'azote par un groupe benzyle ;
- X représente un atome d'oxygène, un atome de soufre, un groupe -NH-, un groupe

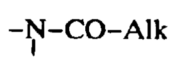

ou un groupe

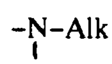

dans lesquels Alk est un groupe alkyle en $C_1$-$C_3$ ;
- Q représente l'hydrogène, un groupe alkyle en $C_1$-$C_4$ ou un groupe aminoalkyle de formule $-(CH_2)_q$-Am', où q est 2 ou 3 et Am' est un groupe pipéridino, benzyl-4 pipéridino ou dialkylamino, chaque alkyle pouvant contenir de 1 à 4 atomes de carbone ;
- R représente l'hydrogène, un groupe méthyle ou un groupe $(CH_2)_n$-L, où n est un nombre entier de 2 à 6 et L est l'hydrogène ou un groupe amino ;
- T représente un groupe choisi parmi :

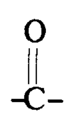

et

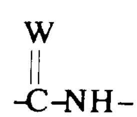

W étant un atome d'oxygène ou de soufre, et

- Z représente soit M ou OM lorsque T représente :

le groupe

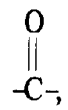

soit M lorsque T représente le groupe

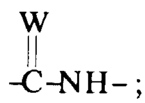

M représente l'hydrogène ou un alkyle droit ou ramifié en $C_1$-$C_6$ ; un α-hydroxybenzyle, un α-alkylbenzyle ou un phénylalkyle dans lesquels le groupe alkyle comprend de 1 à 3 atomes de carbone, non substitué, mono ou polysubstitué sur le cycle aromatique par un halogène, un hydroxy, un alcoxy de 1 à 4 atomes de carbone, un alkyle de 1 à 4 atomes de carbone ; un pyridylalkyle dans lequel le groupe alkyle comprend de 1 à 3 atomes de carbone ; un naphtylalkyle dans lequel le groupe alkyle comprend de 1 à 3 atomes de carbone ; un pyridylthioalkyle dans lequel le groupe alkyle comprend de 1 à 3 atomes de carbone ; un styryle ; un (méthyl-1) imidazolyl-2 thioalkyle dans lequel le groupe alkyle comprend de 1 à 3 atomes de carbone ; un oxo-1 phénylindan-3 yle-2 ;
un groupe phényle non substitué ou substitué par un ou plusieurs substituants choisis parmi halogène, CN, OH, $NH_2$, NH-CO-$NH_2$, $NO_2$, $CONH_2$, $CF_3$, alkyle en $C_1$-$C_{10}$, alcényle contenant 2 à 10 atomes de carbone , alcynyle contenant 2 à 10 atomes de carbone, cycloalkyle contenant 3 à 8 atomes de carbone, bicycloalkyle contenant 4 à 11 atomes de carbone, hydroxyalkyle contenant 1 à 5 atomes de carbone, alcoxy contenant 1 à 10 atomes de carbone, alcoxyalkyle contenant 2 à 10 atomes de carbone, alcoxyalcoxyalkyle contenant 3 à 10 atomes de carbone, alcoxyalcoxy contenant 2 à 10 atomes de carbone, alcényloxy contenant 2 à 10 atomes de carbone, alcényloxyalkyle contenant 3 à 10 atomes de carbone, alcynyloxy contenant 2 à 10 atomes de carbone, alcynyloxyalkyle contenant 3 à 10 atomes de carbone, cycloalcoxy contenant 3 à 8 atomes de carbone, alkylthio contenant 1 à 10 atomes de carbone, alkylthioalkyle contenant 2 à 10 atomes de carbone, acylamino contenant 1 à 7 atomes de carbone, acylaminoalkyle contenant 2 à 8 atomes de carbone, acyloxy contenant 1 à 6 atomes de carbone, alcoxycarbonyle contenant 2 à 5 atomes de carbon, cycloalcoxycarbonyle contenant 4 à 8 atomes de carbone, alkylaminocarbonylamino contenant 2 à 4 atomes de carbone, dialkylaminocarbonylamino contenant 3 à 7 atomes de carbone, cycloalkylaminocarbonylamino contenant 4 à 8 atomes de carbone, alkylaminocarbonylaminoalkyle contenant 3 à 9 atomes de carbone, dialkylaminocarbonylaminoalkyle contenant 4 à 11 atomes de carbone, cycloalkylaminocarbonylaminoalkyl contenant de 5 à12 atomes de carbone, alcoxycarbonylaminoalkyle contenant 3 à 12 atomes de carbone, cycloalcoxycarbonylaminoalkyle contenant 5 à 12 atomes de carbone, carbamoylalkyle contenant 2 à 5 atomes de carbone, alkylaminocarbonylalkyle contenant 3 à 9 atomes de carbone, dialkylaminocarbonylalkyle contenant 4 à 11 atomes de carbone, (1-pyrrolidino)carbonylméthyle, (1-pipéridino)carbonyl-méthyle, (1-pipéridino)carbonyléthyle, cycloalkylaminocarbonylalkyle contenant de 5 à 12 atomes de carbone, alkylaminocarbonylalcoxy contenant 3 à 10 atomes de carbone, dialkylaminocarbonylalcoxy contenant 4 à 10 atomes de carbone, (pipéridinyl-1)carbonylméthoxy, cycloalkylaminocarbonylalcoxy contenant 5 à 11 atomes de carbone, (1-pyrrolidino)-carbonylamino, (1-pipéridino)-carbonylamino, (1-pyrrolidino)carbonylaminoéthyle, (1-pipéridino)-carbonylaminoéthyle;
un groupe benzoyle ou un groupe phénylthioalkyle dans lequel l'alkyle est en $C_1$-$C_3$, lesdits groupes pouvant être non substitués ou mono ou disubstitués par un halogène ou par un alcoxy en $C_1$-$C_4$; un groupe naphtyle ou indényle, dont une ou plusieurs liaisons peuvent être hydrogénées, lesdits groupes pouvant être non substitués ou substitués une ou plusieurs fois par un halogène, un groupe alkyle, phényle, cyano, hydroxyalkyle, hydroxy, oxo, alkylcarbonylamino, alcoxycarbonyle, thioalkyle dans lesquels les alkyles sont en $C_1$-$C_4$ ;

un groupe pyridyle, thiadiazolyle, indolyle, indazolyle, imidazolyle, benzimidazolyle, quinolyle, benzotriazolyle, benzofurannyle, benzothiényle, benzothiazolyle, benzisothiazolyle, isoquinolyle, benzoxazolyle, benzisoxazolyle, benzoxazinyle, benzodioxinyle, isoxazolyle, benzopyranyle, thiazolyle, thiényle, furyle, pyrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle, dont une ou plusieurs doubles liaisons peuvent être hydrogénées, lesdits groupes pouvant être non substitués ou substitués une ou plusieurs fois par un groupe alkyle, phényle, cyano, hydroxyalkyle, hydroxy, alkylcarbonylamino, alcoxycarbonyle, thioalkyle dans lesquels les alkyles sont en $C_1$-$C_4$,

ou un de leurs sels avec des acides minéraux ou organiques ou un de leurs sels d'ammonium quaternaire formés avec l'azote de la pipéridine, caractérisé en ce que

a) on traite une amine libre de formule :

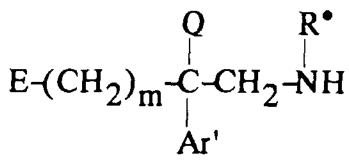

(II)

dans laquelle m, Ar' et Q sont tels que définis précédemment ; R° représente l'hydrogène, un groupe méthyle ou un groupe $(CH_2)_n$-L°, où n est tel que défini précédemment dans la revendication 1 et L° est l'hydrogène ou un groupe amino protégé par un groupe N-protecteur hydrolysable en milieu acide ; et E représente un groupe hydroxy, méthanesulfonyloxy, tétrahydropyranyl-2 oxy, ou un groupe :

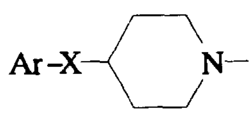

où Ar et X sont tels que définis précédemment ;
- soit avec un dérivé fonctionnel d'un acide de formule :

HO-CO-Z (III)

dans laquelle Z est tel que défini précédemment, lorsqu'on doit préparer un composé de formule (I) où T est -CO-,
- soit avec un iso(thio)cyanate de formule :

W = C = N-Z (III')

dans laquelle W et Z sont tels que définis précédemment, lorsqu'on doit préparer un composé de formule (I) où T est -C(W)-NH-, pour former le composé de formule :

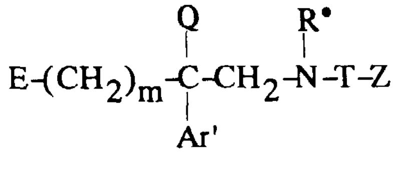

(IV)

b) puis, lorsque E représente le tétrahydropyranyloxy, on élimine le groupe tétrahydropyranyle par hydrolyse

acide, l'hydrolyse pouvant alternativement avoir lieu à l'étape (a) sur l'amine de départ de formule (II),

c) lorsque E représente un groupe hydroxy ou un groupe tétrahydropyranyloxy qui a été hydrolysé, on traite l'alcanolamine N-substituée ainsi obtenue de formule :

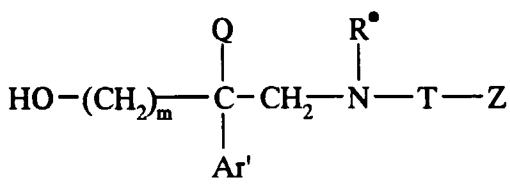

(V)

avec le chlorure de méthanesulfonyle,

d) on fait réagir le mésylate ainsi obtenu à l'étape c) ou directement à l'issue de l'étape a) lorsque E représente un groupe méthanesulfonyloxy de formule :

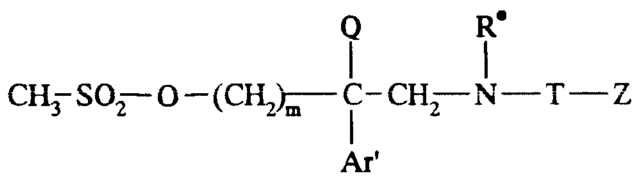

(VI)

avec une amine secondaire de formule :

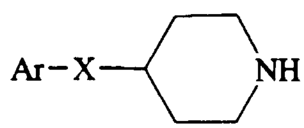

(VII)

dans laquelle Ar et X sont tels que définis dans la revendication 1,

e) on élimine les groupes N-protecteurs éventuels et on transforme éventuellement le produit ainsi obtenu à l'étape d) ou directement à l'issue de l'étape a) lorsque E représente un groupe

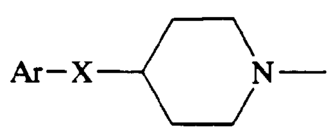

en un de ses sels.

**2.** Procédé stéréosélectif pour la préparation de composés optiquement pur de formule (I*)

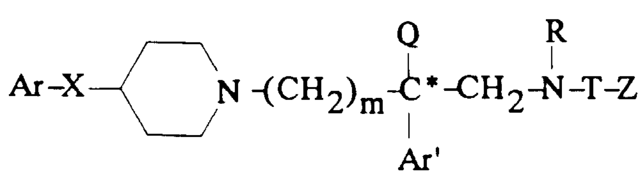

(I*)

dans laquelle :

- "*" signifie que l'atome de carbone ainsi marqué a une configuration absolue (+) ou (-) déterminée ;
- m, Ar et Ar', X, Q, R, T et Z sont tels que définis dans la revendication 1.

ou un de leurs sels avec des acides minéraux ou organiques ou un de leurs sels d'ammonium formés avec l'azote de la pipéridine,

caractérisé en ce que l'on traite un composé de formule :

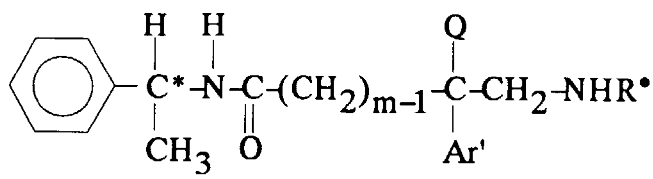 (XVII*)

dans laquelle Ar' et R• et m sont tels que définis dans la revendication 1 ; dans un solvant, en milieu acide, pour fournir l'aminoacide de formule :

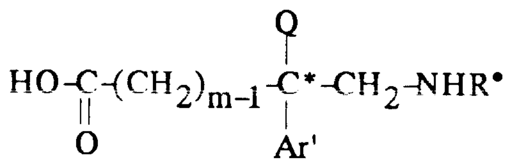 (XVIII*)

qui est estérifié dans un alcanol AlkOH, dans lequel Alk est un alkyle de 1 à 4 atomes de carbone, en milieu acide, puis on traite l'ester correspondant de formule :

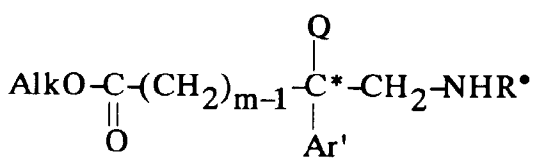 (XIX*)

dans lequel Alk est tel que défini ci-dessus ; Q, Ar', R• et m sont tels que définis dans la revendication 1,

- soit avec un dérivé fonctionnel d'un acide de formule :

HO-CO-Z (III)

- soit avec un iso(thio)cyanate de formule :

W=C=N-Z (III')

Z et W étant tels que définis dans la revendication 1, on soumet l'ester ainsi obtenu de formule :

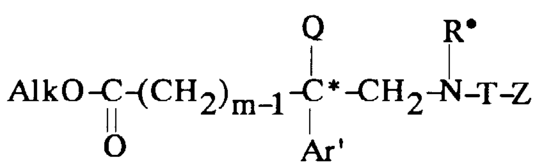 (XX*)

à l'action d'un agent réducteur et on transforme l'alcool correspondant de formule :

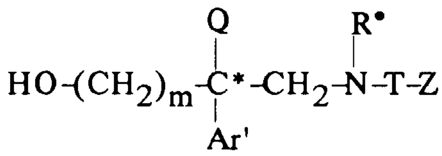 (V*)

en son ester méthanesulfonate de formule :

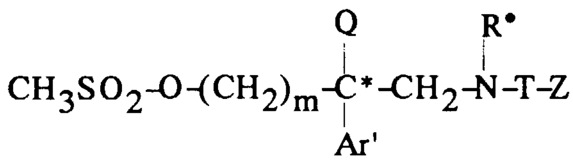 (VI*)

qui, par traitement avec une amine de formule :

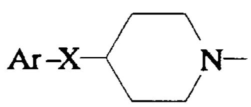 (VII)

dans laquelle Ar et X sont tels que définis dans la revendication 1, donne le composé de formule (I*) ; et on transforme éventuellement le composé de formule (I*) ainsi obtenu en l'un de ses sels.

**3.** Procédé selon l'une des revendications 1 ou 2, caractérisé en ce que l'étape de transformation des composés de formule (I) ou de formule (I*) en leurs sels d'ammonium quaternaire consiste à faire réagir les bases libres des composés (I) ou (I*), pour lesquelles les fonctions aminés autres, éventuellement présentes sont N-protégées par un groupe N-protecteur habituel, avec un excès d'agent alkylant de formule :

A - Q'

Dans lequel A représente un groupe partant et est tel que défini ci-après et on chauffe le mélange réactionnel dans un solvant par exemple choisi parmi le dichlorométhane, le chloroforme, l'acétone ou l'acétonitrile à une température comprise entre la température ambiante et le reflux pendant une à plusieurs heures pour obtenir après traitement selon les méthodes habituelles et après déprotection éventuelle, un mélange de conformères axiaux et équatoriaux des sels d'ammonium quaternaires des composés de formule (I) ou de formule (I*) dans lesquelles le groupe

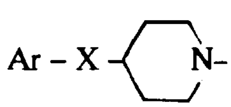

est représenté par le groupe :

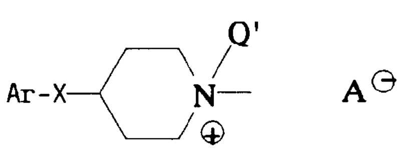

dans laquelle

- Q' représente un groupe alkyle en $C_1$-$C_6$ ou un groupe benzyle et
- $A^{\ominus}$ représente un anion choisi parmi chlorure, bromure, iodure, acétate, méthanesulfonate ou paratoluènesulfonate.

**4.** Procédé selon la revendication 1, caractérisé en ce que l'on fait réagir un composé de formule (II) dans laquelle :

E = THP-O ; m = 2 ;
Ar' = 3,4-dichloro-phényle
$R^\bullet$ = $CH_3$ et Q = H ;
avec un composé de formule (III) dans laquelle Z = phényle et en ce qu'à l'étape d) on utilise un amine de formule (VII) dans laquelle Ar-X est le groupe acétylanilino pour former le composé :
N-[4-(N'-4-acétylanilino-1-pipéridinyl)-2-(3,4-dichlorophényl)-butyl]-N-méthylbenzamide ou un de ses sels pharmaceutiquement acceptables.

**5.** Procédé selon la revendication 1, caractérisé en ce que l'on fait réagir un composé de formule (II) dans laquelle :

E = THP-O ; m = 2 ;
Ar' = naphtyle ;
$R^\bullet$ = H et Q = H ;
avec un composé de formule (III) dans laquelle Z = 2,4-diméthoxyphényle et en ce qu'à l'étape d) on utilise une amine de formule (VII) dans laquelle Ar-X est le groupe 1-méthyl-2-thioimidazolyle pour former le composé :
N-[4-(4-(1-méthyl-2-imidazolylthio)-1-pipéridinyl)-2-naphtylbutyl]-2,4-diméthoxybenzamide ou un de ses sels pharmaceutiquement acceptables.

**6.** Procédé selon la revendication 1, caractérisé en ce que l'on fait réagir un composé de formule (II) dans laquelle :

E = THP-O ; m =2
Ar' = 3,4-dichlorophényle;
$R^\bullet$ = H et Q = H
avec un composé de formule (III) dans laquelle Z est 2,4-dichlorophényle et en ce qu'à l'étape d) on utilise une amine de formule (VII) dans laquelle Ar-X est le groupe 2-thiopyridyle pour former le composé :
N-[2-(3,4-dichlorophényl)-4-(4-(2-pyridylthio)-1-pipéridinyl)butyl]-2,4-dichlorobenzamide ou un de ses sels pharmaceutiquement acceptables.

**7.** Procédé pour l'obtention des composés de formule :

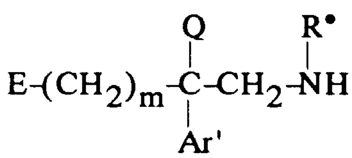

(II)

dans laquelle E représente le groupe de formule :

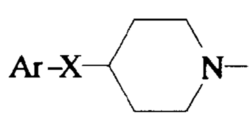

dans laquelle m, Q, Ar', Ar et X sont tels que définis dans la revendication 1 et $R^\bullet$ représente l'hydrogène, un groupe méthyle ou un groupe $(CH_2)_nL^\bullet$, où n est tel que défini précédemment dans la revendication 1 et $L^\bullet$ est l'hydrogène ou un groupe amino protégé ; caractérisé en ce qu'il consiste à réduire le nitrile de formule :

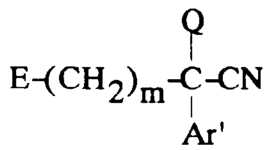

(VIII)

dans laquelle m, E, Q et Ar' sont tels que définis précédemment et à soumettre éventuellement l'amine ainsi obtenue à une alkylation.

**8.** Procédé selon la revendication 1 ou 3, caractérisé en ce que dans l'étape a) on fait réagir un composé de formule (II) dans laquelle E, m, Q, Ar' et $R^{\circ}$ sont tels que définis dans la revendication 1 avec un composé de formule (III) ou (III') dans laquelle Z représente un groupe phényle, un groupe benzyle, un groupe benzoyle, un groupe phénylthioalkyle dans lequel l'alkyle est en $C_1$-$C_3$, lesdits groupes étant non substitués, mono- ou di-substitués par un halogène ou par un alcoxy en $C_1$-$C_4$, pour obtenir un composé de formule (I) dans laquelle Z est tel que défini ci-dessus.

**9.** Procédé stéréosélectif selon la revendication 2 ou 3, caractérisé en ce que dans l'étape a) on utilise un composé de formule (III) ou (III') dans laquelle Z représente un groupe phényle, un groupe benzyle, un groupe benzoyle, un groupe phénylthioalkyle dans lequel l'alkyle est en $C_1$-$C_3$, lesdits groupes étant non substitués, mono- ou di-substitués par un halogène ou par un alcoxy en $C_1$-$C_4$ pour obtenir un composé de formule (I*) dans laquelle Z est tel que défini ci-dessus.

**10.** Procédé selon la revendication 1 ou 3, caractérisé en ce que le groupe Ar des composés de formule (II) ou (VII) représente un phényle substitué une ou plusieurs fois par un atome de chlore ou de fluor, et/ou le groupe Ar' du composé de formule (II) représente un groupe phényle, mono ou di-substitué par un atome de chlore ou de fluor, un groupe imidazolyle ou un groupe benzothiényle substitués par un atome de chlore ou de fluor ; un groupe naphtyle substitué par un atome de fluor.

**11.** Procédé stéréosélectif selon la revendication 2 ou 3, caractérisé en ce que le groupe Ar' du composé (XVII*) représente un groupe phényle, mono ou di-substitué par un atome de chlore ou de fluor, un groupe imidazolyle ou un groupe benzothiényle substitués par un atome de chlore ou de fluor ; un groupe naphtyle substitué par un atome de fluor et/ou le groupe Ar du composé (VII) un phényle substitué une ou plusieurs fois par un atome de chlore ou de fluor.

**12.** Procédé pour la préparation d'une composition pharmaceutique, caractérisé en ce que l'on mélange, en tant que principe actif, un composé de formule (I) ou (I*) préparé par le procédé selon l'une quelconque des revendications 1 à 6 avec un véhicule pharmaceutiquement acceptable.

**Revendications pour l'Etat contractant suivant : GR**

**1.** Composés de formule :

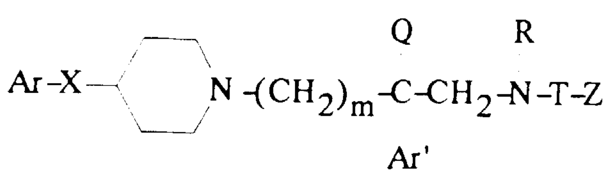

(I)

dans laquelle :

- m est égal à 2 ou 3 ;
- Ar représente un phényle non substitué ou substitué une ou plusieurs fois par un atome d'halogène, par un alkyle en $C_1$-$C_3$, par un trifluorométhyle, par un alcoxy dans lequel l'alkyle est en $C_1$-$C_3$, par un hydroxyle ou

par un méthylènedioxy ; un groupe thiényle, pyridyle, imidazolyle substitué ou non par un alkyle en $C_1$-$C_3$ ;

- Ar' représente un groupe phényle non substitué, mono ou di-substitué par un atome d'halogène, par un alkyle en $C_1$-$C_3$, par un trifluorométhyle, par un alcoxy dans lequel l'alkyle est en $C_1$-$C_3$, par hydroxyle ou par un méthylènedioxy ; un groupe thiényle ; un groupe imidazolyle ou un groupe benzothiényle non substitués ou substitués par un halogène ; un groupe naphtyle non substitué ou substitué par un halogène ; un groupe biphényle ; un indolyle non substitué ou substitué sur l'azote par un groupe benzyle ;
- X représente un atome d'oxygène, un atome de soufre, un groupe -NH-, un groupe

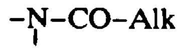

ou un groupe

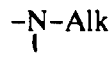

dans lesquels Alk est un groupe alkyle en $C_1$-$C_3$ ;

- Q représente l'hydrogène, un groupe alkyle en $C_1$-$C_4$ ou un groupe aminoalkyle de formule $-(CH_2)_q$-Am', où q est 2 ou 3 et Am' est un groupe pipéridino, benzyl-4 pipéridino ou dialkylamino, chaque alkyle pouvant contenir de 1 à 4 atomes de carbone ;
- R représente l'hydrogène, un groupe méthyle ou un groupe $(CH_2)_n$-L, où n est un nombre entier de 2 à 6 et L est l'hydrogène ou un groupe amino ;
- T représente un groupe choisi parmi :

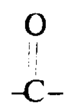 et 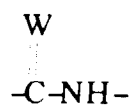

W étant un atome d'oxygène ou de soufre, et

- Z représente soit M ou OM lorsque T représente :

le groupe

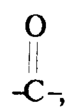

soit M lorsque T représente le groupe

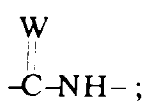

M représente l'hydrogène ou un alkyle droit ou ramifié en $C_1$-$C_6$ ; un $\alpha$-hydroxybenzyle, un $\alpha$-alkylbenzyle ou un phénylalkyle dans lesquels le groupe alkyle comprend de 1 à 3 atomes de carbone, non substitué, mono ou polysubstitué sur le cycle aromatique par un halogène, un hydroxy, un alcoxy de 1 à 4 atomes de carbone, un alkyle de 1 à 4 atomes de carbone ; un pyridylalkyle dans lequel le groupe alkyle comprend de 1 à 3 atomes de carbone ; un naphtylalkyle dans lequel le groupe alkyle comprend de 1 à 3 atomes de carbone ; un pyridylthioalkyle dans lequel le groupe alkyle comprend de 1 à 3 atomes de carbone ; un styryle ; un (méthyl-1) imidazolyl-2 thioalkyle dans lequel le groupe alkyle comprend de 1 à 3 atomes de carbone ; un oxo-1 phénylindan-3 yle-2 ;

un groupe phényle non substitué ou substitué par un ou plusieurs substituants choisis parmi halogène, CN, OH, $NH_2$, NH-CO-$NH_2$, $NO_2$, $CONH_2$, $CF_3$, alkyle en $C_1$-$C_{10}$, alcényle contenant 2 à 10 atomes de

carbone , alcynyle contenant 2 à 10 atomes de carbone, cycloalkyle contenant 3 à 8 atomes de carbone, bicycloalkyle contenant 4 à 11 atomes de carbone, hydroxyalkyle contenant 1 à 5 atomes de carbone, alcoxy contenant 1 à 10 atomes de carbone, alcoxyalkyle contenant 2 à 10 atomes de carbone, alcoxyalcoxyalkyle contenant 3 à 10 atomes de carbone, alcoxyalcoxy contenant 2 à 10 atomes de carbone, alcényloxy contenant 2 à 10 atomes de carbone, alcényloxyalkyle contenant 3 à 10 atomes de carbone, alcynyloxy contenant 2 à 10 atomes de carbone, alcynyloxyalkyle contenant 3 à 10 atomes de carbone, cycloalcoxy contenant 3 à 8 atomes de carbone, alkylthio contenant 1 à 10 atomes de carbone, alkylthioalkyle contenant 2 à 10 atomes de carbone, acylamino contenant 1 à 7 atomes de carbone, acylaminoalkyle contenant 2 à 8 atomes de carbone, acyloxy contenant 1 à 6 atomes de carbone, alcoxycarbonyle contenant 2 à 5 atomes de carbon, cycloalcoxycarbonyle contenant 4 à 8 atomes de carbone, alkylaminocarbonylamino contenant 2 à 4 atomes de carbone, dialkylaminocarbonylamino contenant 3 à 7 atomes de carbone, cycloalkylaminocarbonylamino contenant 4 à 8 atomes de carbone, alkylaminocarbonylaminoalkyle contenant 3 à 9 atomes de carbone, dialkylaminocarbonylaminoalkyle contenant 4 à 11 atomes de carbone, cycloalkylaminocarbonylaminoalkyl contenant de 5 à12 atomes de carbone, alcoxycarbonylaminoalkyle contenant 3 à 12 atomes de carbone, cycloalcoxycarbonylaminoalkyle contenant 5 à 12 atomes de carbone, carbamoylalkyle contenant 2 à 5 atomes de carbone, alkylaminocarbonylalkyle contenant 3 à 9 atomes de carbone, dialkylaminocarbonylalkyle contenant 4 à 11 atomes de carbone, (1-pyrrolidino)carbonylméthyle, (1-pipéridino)carbonyl-méthyle, (1-pipéridino)carbonyléthyle, cycloalkylaminocarbonylalkyle contenant de 5 à 12 atomes de carbone, alkylaminocarbonylalcoxy contenant 3 à 10 atomes de carbone, dialkylaminocarbonylalcoxy contenant 4 à 10 atomes de carbone, (pipéridinyl-1)carbonylméthoxy, cycloalkylaminocarbonylalcoxy contenant 5 à 11 atomes de carbone, (1-pyrrolidino)-carbonylamino, (1-pipéridino)-carbonylamino, (1-pyrrolidino)carbonylaminoéthyle, (1-pipéridino)-carbonylaminoéthyle;

un groupe benzoyle ou un groupe phénylthioalkyle dans lequel l'alkyle est en $C_1$-$C_3$, lesdits groupes pouvant être non substitués ou mono ou disubsstitués par un halogène ou par un alcoxy en $C_1$-$C_4$; un groupe naphtyle ou indényle, dont une ou plusieurs liaisons peuvent être hydrogénées, lesdits groupes pouvant être non substitués ou substitués une ou plusieurs fois par un halogène, un groupe alkyle, phényle, cyano, hydroxyalkyle, hydroxy, oxo, alkylcarbonylamino, alcoxycarbonyle, thioalkyle dans lesquels les alkyles sont en $C_1$-$C_4$ ;

un groupe pyridyle, thiadiazolyle, indolyle, indazolyle, imidazolyle, benzimidazolyle, quinolyle, benzotriazolyle, benzofurannyle, benzothiényle, benzothiazolyle, benzisothiazolyle, isoquinolyle, benzoxazolyle, benzisoxazolyle, benzoxazinyle, benzodioxinyle, isoxazolyle, benzopyranyle, thiazolyle, thiényle, furyle, pyrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle, dont une ou plusieurs doubles liaisons peuvent être hydrogénées, lesdits groupes pouvant être non substitués ou substitués une ou plusieurs fois par un groupe alkyle, phényle, cyano, hydroxyalkyle, hydroxy, alkylcarbonylamino, alcoxycarbonyle, thioalkyle dans lesquels les alkyles sont en $C_1$-$C_4$,

ou un de leurs sels avec des acides minéraux ou organiques ou un de leurs sels d'ammonium quaternaire formés avec l'azote de la pipéridine.

**2.** Composés selon la revendication 1, sous forme optiquement pure, de formule :

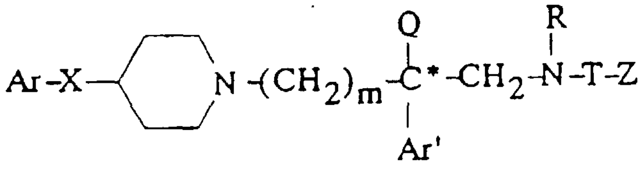

(I*)

dans laquelle :

- "*" signifie que l'atome de carbone ainsi marqué a une configuration absolue (+) ou (-) déterminée ;
- m, Ar et Ar', X, Q, R, T et Z sont tels que définis dans la revendication 1.

ou un de leurs sels avec des acides minéraux ou organiques ou un de leurs sels d'ammonium formés avec l'azote de la pipéridine.

**3.** Composés selon l'une des revendications 1 ou 2, caractérisés en ce qu'ils sont sous la forme d'un sel d'ammonium quaternaire, le groupe de formule

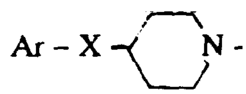

étant alors représenté par le groupe :

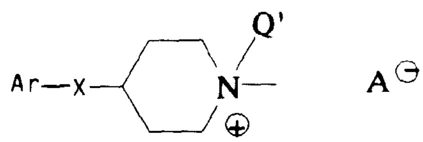

dans laquelle

. Q' représente un groupe alkyle en $C_1$-$C_6$ ou un groupe benzyle et

. $A^{\ominus}$ représente un anion choisi parmi chlorure, bromure, iodure, acétate, méthanesulfonate ou paratoluènesulfonate.

**4.** Composé selon la revendication 1, qui est le N-[2-(3,4-Dichlorophényl)4-(4-(2-pyridylthio)-1-pipéridinyl)butyl]-2,4-dichlorobenzamide ou un de ses sels pharmaceutiquement acceptables.

**5.** Composé selon la revendication 1, qui est le N-[4-(N'-4-acétylanilino-1-pipéridinyl)-2-(3,4-dichlorophényl)-butyl]-N-méthylbenzamide ou un de ses sels pharmaceutiquement acceptables.

**6.** Composé selon la revendication 1, qui est le N-[4-(4-(1-méthyl-2-imidazolylthio)-1-pipéridinyl)-2-naphtyl-butyl]-2,4-diméthoxybenzamide ou un de ses sels pharmaceutiquement acceptables.

**7.** Procédé pour la préparation de composés de formule (I) selon la revendication 1, caractérisé en ce que

a) on traite une amine libre de formule :

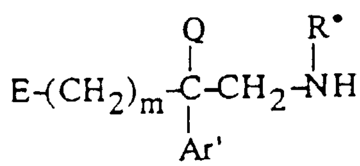

(II)

dans laquelle m, Ar' et Q sont tels que définis précédemment dans la revendication 1 ; $R^{\bullet}$ représente d'hydrogène, un groupe méthyle ou un groupe $(CH_2)_n$-$L^{\bullet}$, où n est tel que défini précédemment dans la revendication 1 et $L^{\bullet}$ est l'hydrogène ou un groupe amino protégé par un groupe N-protecteur hydrolysable en milieu acide ; et E représente un groupe hydroxy, méthanesulfonyloxy, tétrahydropyranyl-2 oxy, ou un groupe :

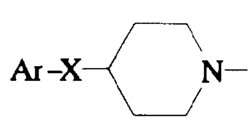

où Ar et X sont tels que définis dans la revendication 1;

- soit avec un dérivé fonctionnel d'un acide de formule :

HO-CO-Z (III)

dans laquelle Z est tel que défini dans la revendication 1, lorsqu'on doit préparer un composé de formule (I) où T est -CO-,

- soit avec un iso(thio)cyanate de formule :

W = C = N-Z (III')

dans laquelle W et Z sont tels que définis dans la revendication 1, lorsqu'on doit préparer un composé de formule (I) où T est -C(W)-NH-, pour former le composé de formule :

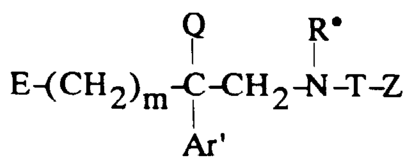

(IV)

b) puis, lorsque E représente le tétrahydropyranyloxy, on élimine le groupe tétrahydropyranyle par hydrolyse acide, l'hydrolyse pouvant alternativement avoir lieu à l'étape (a) sur l'amine de départ de formule (II),
c) lorsque E représente un groupe hydroxy ou un groupe tétrahydropyranyloxy qui a été hydrolysé, on traite l'alcanolamine N-substituée ainsi obtenue de formule :

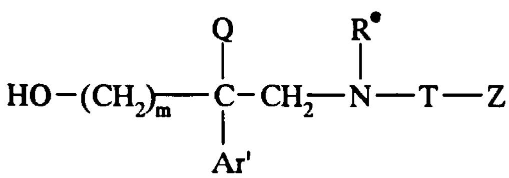

(V)

avec le chlorure de méthanesulfonyle,
d) on fait réagir le mésylate ainsi obtenu à l'étape c) ou directement à l'issue de l'étape a) lorsque E représente un groupe méthanesulfonyloxy de formule :

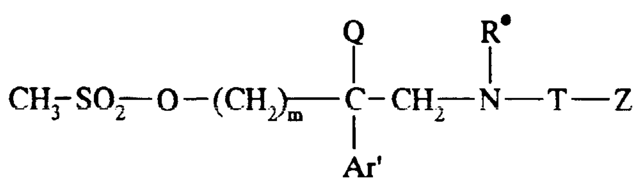

(VI)

avec une amine secondaire de formule :

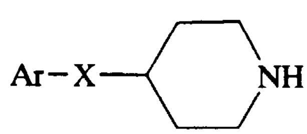

(VII)

dans laquelle Ar et X sont tels que définis dans la revendication 1,
e) on élimine les groupes N-protecteurs éventuels et on transforme éventuellement le produit ainsi obtenu à l'étape d) ou directement à l'issue de l'étape a) lorsque E représente un groupe

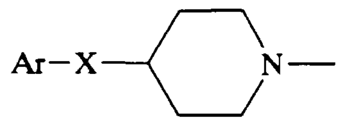

en un de ses sels.

**8.** Procédé stéréosélectif pour la préparation de composés optiquement purs de formule (I*) selon la revendication 2, caractérisé en ce que on traite un composé de formule :

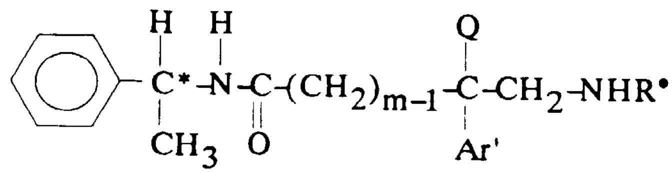 (XVII*)

dans un solvant, en milieu acide, pour fournir l'aminoacide de formule :

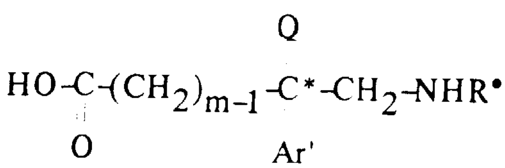 (XVIII*)

qui est estérifié dans un alcanol AlkOH, dans lequel Alk est un alkyle de 1 à 4 atomes de carbone, en milieu acide, puis on traite l'ester correspondant de formule :

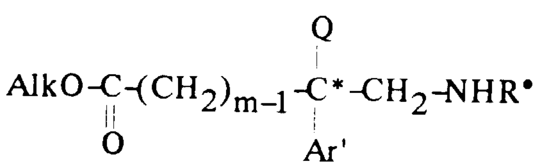 (XIX*)

dans lequel Alk est tel que défini ci-dessus ; Q, Ar', et m sont tels que définis dans la revendication 1 et R• est tel que défini dans la revendication 7,

- soit avec un dérivé fonctionnel d'un acide de formule :

$$HO\text{-}CO\text{-}Z \quad (III)$$

- soit avec un iso(thio)cyanate de formule :

$$W{=}C{=}N\text{-}Z \quad (III')$$

Z et W étant tels que définis dans la revendication 1, on soumet l'ester ainsi obtenu de formule :

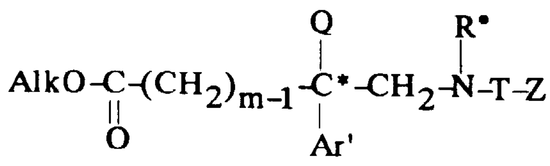

(XX*)

à l'action d'un agent réducteur et on transforme l'alcool correspondant de formule :

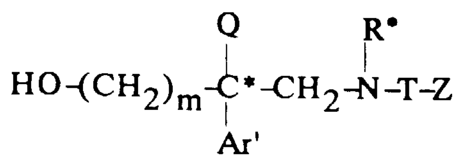

(V*)

en son ester méthanesulfonate de formule :

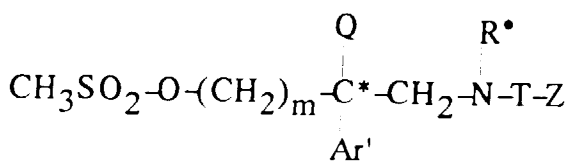

(VI*)

qui, par traitement avec une amine de formule :

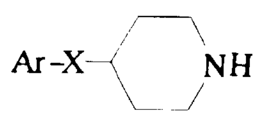

(VII)

dans laquelle Ar et X sont tels que définis dans la revendication 1, donne le composé de formule (I*).

**9.** Composé de formule :

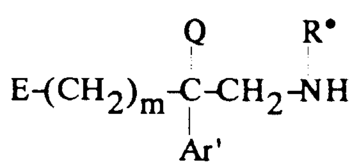

(II)

dans laquelle E représente le groupe de formule :

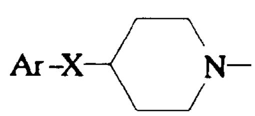

m, Q, Ar', Ar et X sont tels que définis dans la revendication 1 et R• représente l'hydrogène, un groupe méthyle ou un groupe $(CH_2)_nL^{\bullet}$ où n est tel que défini dans la revendication 1 et $L^{\bullet}$ est l'hydrogène ou un groupe amino protégé.

**10.** Composés de formule (I) selon l'une quelconque des revendications 1 à 3, dans laquelle Z représente un groupe phényle, un groupe benzyle, un groupe benzoyle, un groupe phénylthioalkyle dans lequel l'alkyle est en $C_1$-$C_3$, lesdits groupes étant non substitués, mono- ou di-substitués par un halogène ou par un alcoxy en $C_1$-$C_4$.

**11.** Composés de formule (I) selon l'une quelconque des revendications 1 à 3, dans laquelle :

- Ar représente un phényle substitué une ou plusieurs fois par un atome de chlore ou de fluor, et/ou
- Ar' représente un groupe phényle, mono ou di-substitué par un atome de chlore ou de fluor; un groupe imidazolyle ou un groupe benzothiényle substitué par un atome de chlore ou de fluor; un groupe naphtyle substitué par un atome de fluor.

**12.** Procédé pour la préparation d'une composition pharmaceutique, caractérisé en ce que l'on mélange, en tant que principe actif un composé de formule (I) ou (I*) selon l'une quelconque des revendication 1 à 6 avec un véhicule pharmaceutiquement acceptable.

## Patentansprüche

### Patentansprüche für folgende Vertragsstaaten : AT, BE, CH, LI, DE, DK, FR, GB, IT, LU, NL, PT, SE

**1.** Verbindungen der Formel

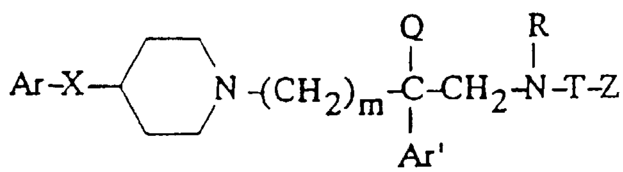

(I)

in der:

- m den Wert 2 oder 3 hat;
- Ar unsubstitiertes oder ein- oder mehrfach durch ein Halogenatom, durch eine $C_1$-$C_3$-Alkylgruppe, durch eine Trifluormethylgruppe, durch eine Alkoxygruppe mit $C_1$-$C_3$-Alkyl, durch eine Hydroxylgruppe oder durch eine Methylendioxygruppe substituiertes Phenyl; eine Thienylgruppe, eine Pyridylgruppe oder eine unsubstituierte oder durch $C_1$-$C_3$-Alkyl substituierte Imidazolylgruppe bedeutet;
- Ar' eine unsubstituierte oder durch ein Halogenatom, durch eine $C_1$-$C_3$-Alkylgruppe, durch eine Trifluormethylgruppe, durch eine Alkoxygruppe mit $C_1$-$C_3$-Alkyl, durch eine Hydroxylgruppe oder durch eine Methylendioxygruppe mono- oder disubstituierte Phenylgruppe; eine Thienylgruppe; eine Imidazolylgruppe oder eine Benzothienylgruppe, die unsubstituiert sind oder durch ein Halogen substituiert sind; eine unsubstituierte oder durch ein Halogen substituierte Naphthylgruppe; eine Biphenylgruppe; eine unsubstituierte oder am Stickstoff durch eine Benzylgruppe substituierte Indolylgruppe bedeutet;
- X ein Sauerstoffatom, ein Schwefelatom, eine Gruppe -NH-, eine Gruppe

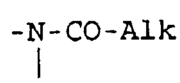

oder eine Gruppe

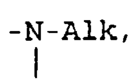

worin Alk eine $C_1$-$C_3$-Alkylgruppe darstellt, bedeutet;
- Q Wasserstoff, eine $C_1$-$C_4$-Alkylgruppe oder eine Aminoalkylgruppe der Formel $-(CH_2)_q$-Am', worin q den Wert

2 oder 3 hat und Am' eine Piperidinogruppe, eine 4-Benzyl-piperidinogruppe oder Dialkylaminogruppe darstellt, worin jeder Alkylrest 1 bis 4 Kohlenstoffatome aufweisen kann, bedeutet;

- R Wasserstoff, eine Methylgruppe oder eine $(CH_2)_n$-L-Gruppe bedeutet, worin n eine ganze Zahl mit einem Wert von 2 bis 6 ist und L Wasserstoff oder eine Aminogruppe darstellt;
- T eine Gruppe bedeutet, die ausgewählt ist unter

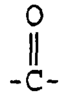 und 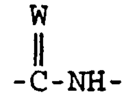

wobei W ein Sauerstoff- oder Schwefelatom bedeutet, und

- Z entweder die Bedeutung M oder OM hat, wenn T die Gruppe

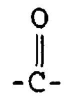

darstellt, oder Z die Bedeutung M hat, wenn T die Gruppe

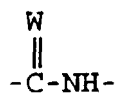

bedeutet;

- M eine der folgenden Bedeutungen hat: Wasserstoff oder eine geradkettige oder verzweigte $C_1$-$C_6$-Alkylgruppe, eine α-Hydroxybenzylgruppe, eine α-Alkylbenzyl- oder Phenylalkylgruppe, in der die Alkylgruppe 1 bis 3 Kohlenstoffatome aufweist, die am aromatischen Ring unsubstituiert oder durch ein Halogenatom, eine Hydroxygruppe, eine Alkoxygruppe mit 1 bis 4 Kohlenstoffatomen oder eine Alkylgruppe mit 1 bis 4 Kohlenstoffatomen mono- oder polysubstituiert ist; eine Pyridylalkylgruppe, in der die Alkylgruppe 1 bis 3 Kohlenstoffatome umfaßt; eine Naphthylalkylgruppe, in der die Alkylgruppe 1 bis 3 Kohlenstoffatome umfaßt; eine Pyridylthioalkylgruppe, in der die Alkylgruppe 1 bis 3 Kohlenstoffatome umfaßt; eine Styrylgruppe; eine (1-Methyl)-imidazolyl-2-thioalkylgruppe, in der die Alkylgruppe 1 bis 3 Kohlenstoffatome umfaßt; eine 1-Oxo-3-phenylindan-2-ylgruppe;

eine Phenylgruppe, die unsubstituiert ist oder durch einen oder mehrere der aus folgender Gruppe ausgewählten Susbtituenten substituiert ist: Halogen, CN, OH, $NH_2$, NH-CO-$NH_2$, $NO_2$, $CONH_2$, $CF_3$, $C_1$-$C_{10}$-Alkyl, Alkenyl mit 2 bis 10 Kohlenstoffatomen, Alkinyl mit 2 bis 10 Kohlenstoffatomen, Cycloalkyl mit 3 bis 8 Kohlenstoffatomen, Bicycloalkyl mit 4 bis 11 Kohlenstoffatomen, Hydroxyalkyl mit 1 bis 5 Kohlenstoffatomen, Alkoxy mit 1 bis 10 Kohlenstoffatomen, Alkoxyalkyl mit 2 bis 10 Kohlenstoffatomen, Alkoxyalkoxyalkyl mit 3 bis 10 Kohlenstoffatomen, Alkoxyalkoxy mit 2 bis 10 Kohlenstoffatomen, Alkenyloxy mit 2 bis 10 Kohlenstoffatomen, Alkenyloxyalkyl mit 3 bis 10 Kohlenstoffatomen, Alkinyloxy mit 2 bis 10 Kohlenstoffatomen, Alkinyloxyalkyl mit 3 bis 10 Kohlenstoffatomen, Cycloalkoxy mit 3 bis 8 Kohlenstoffatomen, Alkylthio mit 1 bis 10 Kohlenstoffatomen, Alkylthioalkyl mit 2 bis 10 Kohlenstoffatomen, Acylamino mit 1 bis 7 Kohlenstoffatomen, Acylaminoalkyl mit 2 bis 8 Kohlenstoffatomen, Acyloxy mit 1 bis 6 Kohlenstoffatomen, Alkoxycarbonyl mit 2 bis 5 Kohlenstoffatomen, Cycloalkoxycarbonyl mit 4 bis 8 Kohlenstoffatomen, Alkylaminocarbonylamino mit 2 bis 4 Kohlenstoffatomen, Dialkylaminocarbonylamino mit 3 bis 7 Kohlenstoffatomen, Cycloalkylaminocarbonylamino mit 4 bis 8 Kohlenstoffatomen, Alkylaminocarbonylaminoalkyl mit 3 bis 9 Kohlenstoffatomen, Dialkylaminocarbonylaminoalkyl mit 4 bis 11 Kohlenstoffatomen, Cycloalkylaminocarbonylaminoalkyl mit 5 bis 12 Kohlenstoffatomen, Alkoxycarbonylaminoalkyl mit 3 bis 12 Kohlenstoffatomen, Cycloalkoxycarbonylaminoalkyl mit 5 bis 12 Kohlenstoffatomen, Carbamoylalkyl mit 2 bis 5 Kohlenstoffatomen, Alkylaminocarbonylalkyl mit 3 bis 9 Kohlenstoffatomen, Dialkylaminocarbonylalkyl mit 4 bis 11 Kohlenstoffatomen, (1-Pyrrolidino)-carbonylmethyl, (1-Piperidino)-carbonylmethyl, (1-Piperidino)-carbonylethyl, Cycloalkylaminocarbonylalkyl mit 5 bis 12 Kohlenstoffatomen, Alkylaminocarbonylalkoxy mit 3 bis 10 Kohlenstoffatomen, Dialkylaminocarbonylalkoxy mit 4 bis 10 Kohlenstoffatomen, (1-Piperidinyl)-carbonylmethoxy, Cycloalkylaminocarbonylalkoxy mit 5 bis 11 Kohlenstoffatomen, (1-Pyrrolidino)-carbonylamino, (1-Piperdino)-carbonylamino, (1-Pyrrolidino)-carbonylaminoethyl und (1-Piperidino)-carbonylaminoethyl;
eine Benzoylgruppe oder eine Phenylthioalkylgruppe mit einer $C_1$-$C_3$-Alkylgruppe; wobei diese Gruppen unsubstituiert oder durch ein Halogen oder durch eine $C_1$-$C_4$-Alkoxygruppe mono- oder disubstituiert sein können; eine Naphthyl- oder Indenylgruppe, bei denen eine oder mehrere Bindungen hydriert sein können, wobei diese Gruppen unsubstituiert oder ein- oder mehrfach durch ein Halogen, eine Alkyl-, Phenyl-, Cyano-, Hydroxyalkyl-, Hydroxy-, Oxo-, Alkylcarbonylamino-, Alkoxycarbonyl- oder Thioalkylgruppe, worin die Alkylgruppen 1 bis 4 Kohlenstoffatome aufweisen, substituiert sind;
eine Pyridyl-, Thiadiazolyl-, Indolyl-, Indazolyl-, Imidazolyl-, Benzimidazolyl-, Chinolyl-, Benzotriazolyl-, Benzofuranyl-, Benzothienyl-, Benzothiazolyl-, Benzisothiazolyl-, Isochinolyl-, Benzoxazolyl-, Benzisoxazolyl-, Benzoxazinyl-, Benzodioxinyl-, Isoxazolyl-, Benzopyranyl-, Thiazolyl-, Thienyl-, Furyl-, Pyranyl-, Chromenyl-, Isobenzofuranyl-, Pyrrolyl-, Pyrazolyl-, Pyrazinyl-, Pyrimidinyl-, Pyridazinyl-, Indolizinyl-, Phthalazinyl-, Chinazolinyl-, Acridinyl-, Isothiazolyl-, Isochromanyl- und Chromanylgruppe, bei denen eine oder mehrere Doppelbindungen hydriert sein können, wobei diese Gruppen unsubstituiert oder ein- oder mehrfach durch eine Alkyl-, Phenyl-, Cyano-, Hydroxyalkyl-, Hydroxy-, Alkylcarbonylamino-, Alkoxycarbonyl- oder Thioalkylgruppe mit $C_1$-$C_4$-Alkylgruppen substituiert sein können;
oder ein Salz davon mit anorganischen oder organischen Säuren oder ein mit dem Stickstoff von Piperidin gebildetes quaternäres Ammoniumsalz davon.

**2.** Verbindungen nach Anspruch 1 in optisch reiner Form der Formel

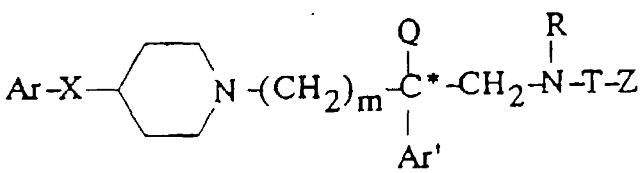

(I*)

in der

- "*" bedeutet, daß das auf diese Weise markierte Kohlenstoffatom eine festgestellte absolute (+)- oder (-)-Konfiguration aufweist;
- m, Ar und Ar', X, Q, R, T und Z die in Anspruch 1 definierten Bedeutungen haben;

oder ein Salz davon mit anorganischen oder organischen Säuren oder ein Ammoniumsalz davon mit dem Stickstoff des Piperidins.

**3.** Verbindungen nach einem der Ansprüche 1 oder 2, dadurch gekennzeichnet, daß sie in Form eines quaternären Ammoniumsalzes vorliegen, deren Gruppe der Formel

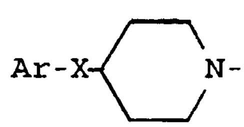

dann durch die folgende Gruppe wiedergeben wird:

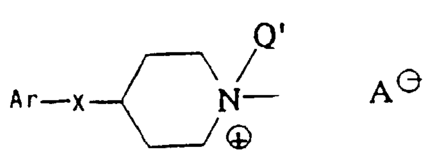

in der

- Q' eine $C_1$-$C_6$-Alkylgruppe oder eine Benzylgruppe bedeutet und

- $A^{\ominus}$ ein unter Chlorid, Bromid, Iodid, Acetat, Methansulfonat oder p-Toluolsulfonat ausgewähltes Anion bedeutet.

**4.** Verbindung nach Anspruch 1, nämlich N-[2-(3,4-Dichlorphenyl)-4-(4-(2-pyridylthio)-1-piperidinyl)-butyl]-2,4-dichlorbenzamid oder ein pharmazeutisch verträgliches Salz davon.

**5.** Verbindung nach Anspruch 1, nämlich N-[4-(N'-4-Acetylanilino-1-piperidinyl)-2-(3,4-dichlorphenyl)-butyl]-N-methylbenzamid oder ein pharmazeutisch verträgliches Salz davon.

**6.** Verbindung nach Anspruch 1, nämlich N-[4-(4-(1-Methyl-2-imidazolylthio)-1-piperidinyl)-2-naphthylbutyl]-2,4-dimethoxybenzamid oder ein pharmazeutisch verträgliches Salz davon.

**7.** Verfahren zur Herstellung der Verbindungen der Formel (I) nach Anspruch 1, dadurch gekennzeichnet, daß man

a) ein freies Amin der Formel

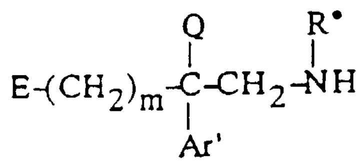

(II)

in der m, Ar' und Q die vorstehend in Anspruch 1 definierten Bedeutungen haben; $R^{\bullet}$ Wasserstoff, eine Methylgruppe oder eine $(CH_2)_n$-$L^{\bullet}$-Gruppe bedeutet, worin n die vorstehend in Anspruch 1 definierte Bedeutung hat und $L^{\bullet}$ Wasserstoff oder eine Aminogruppe, die durch eine in saurem Medium hydrolysierbare N-Schutzgruppe geschützt ist, bedeutet; und E eine Hydroxygruppe, Methansulfonyloxygruppe, Tetrahydropyranyl-2-oxygruppe oder eine Gruppe der Formel

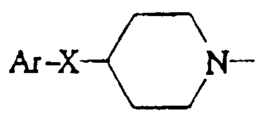

bedeutet oder Ar und X die in Anspruch 1 definierten Bedeutungen haben;

- entweder mit einem funktionellen Derivat einer Säure der Formel

HO-CO-Z (III)

in der Z die in Anspruch 1 definierte Bedeutung hat, falls eine Verbindung der Formel (I), in der T die Bedeutung -CO- hat, herzustellen ist,
- oder mit einem Iso(thio)cyanat der Formel

W=C=N-Z (III')

in der W und Z die in Anspruch 1 definierten Bedeutungen haben, wenn eine Verbindung der Formel (I), worin T die Bedeutung -C(W)-NH- hat, herzustellen ist, behandelt, wodurch eine Verbindung der Formel

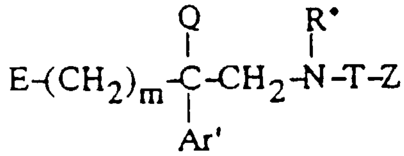

(IV)

entsteht,

b) sodann, wenn E Tetrahydropyranyloxy bedeutet, die Tetrahydropyranylgruppe durch saure Hydrolyse entfernt, wobei die Hydrolyse alternativ in der Stufe (a) am Ausgangsamin der Formel (II) stattfinden kann,

c) Wenn E eine Hydroxylgruppe oder eine Tetrahydropyranyloxygruppe, der hydrolysiert wurde, bedeutet, das auf diese Weise erhaltene N-substituierte Alkanolamin der Formel

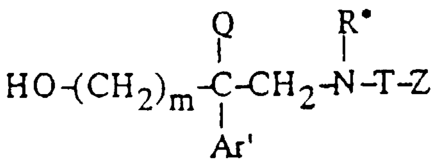

(V)

mit Methansulfonylchlorid behandelt,

d) das in der Stufe c) oder, wenn E eine Methanesulfonyloxygruppe bedeutet, direkt am Ende der Stufe a) erhaltene Mesylat der Formel

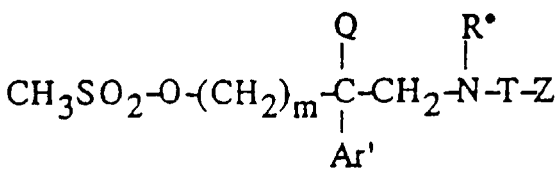

(VI)

mit einem sekundären Amin der Formel

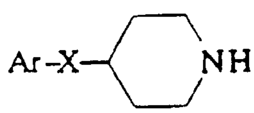

(VII)

in der Ar und X die in Anspruch 1 definierten Bedeutungen haben, umsetzt,

e) die gegebenenfalls vorhandenen N-Schutzgruppen entfernt und gegebenenfalls das in der Stufe d) oder, wenn E eine Gruppe

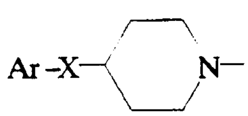

bedeutet, direkt am Ende der Stufe a) erhaltene Produkt in ein Salz überführt.

**8.** Stereoselektives Verfahren zur Herstellung der optisch reinen Verbindungen der Formel (I*) gemäß Anspruch 2, dadurch gekennzeichnet, daß man eine Verbindung der Formel

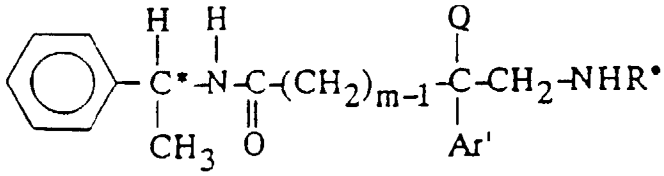

(XVII*)

in einem Lösungsmittel in saurem Medium behandelt, wodurch man eine Aminosäure der Formel

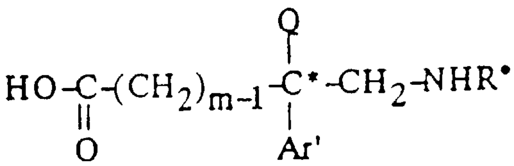

(XVIII*)

erhält, die in einem Alkanol AlkOH, in der Alk eine Alkylgruppe mit 1 bis 4 Kohlenstoffatomen bedeutet, in einem sauren Medium verestert wird, wonach man den entsprechenden Ester der Formel

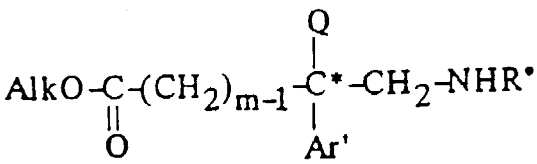

(XIX*)

in der Alk die vorstehend definierte Bedeutung hat; Q, Ar' und m die in Anspruch 1 definierten Bedeutungen haben und R• die in Anspruch 7 definierte Bedeutung hat,

- entweder mit einem funktionellen Derivat einer Säure der Formel

HO-CO-Z (III)

- oder mit einem Iso(thio)cyanat der Formel

W=C=N-Z (III')

worin Z und W die vorstehend in Anspruch 1 definierten Bedeutungen haben, behandelt, den auf diese Weise erhaltenen Ester der Formel

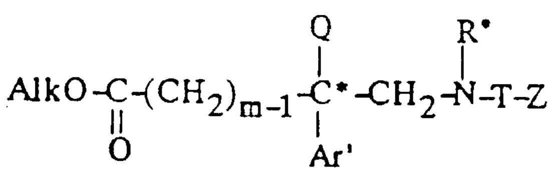

(XX*)

der Einwirkung eines Reduktionsmittels unterwirft und den erhaltenen Alkohol der Formel

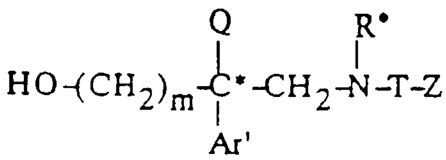 (V*)

in den Methansulfonatester der Formel

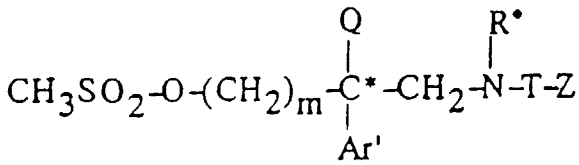 (VI*)

überführt, der dann durch Behandlung mit einem Amin der Formel

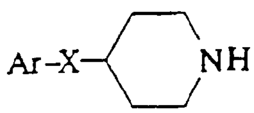 (VII)

in der Ar und X die in Anspruch 1 definierten Bedeutungen haben, die Verbindung der Formel (I*) ergibt.

**9.** Verbindung der Formel

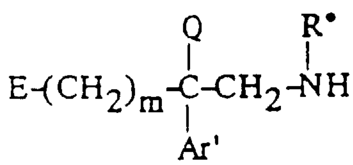 (II)

in der E eine Gruppe der folgenden Formel bedeutet

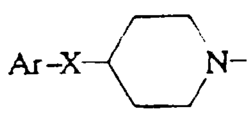

worin m, Q, Ar', Ar und X die in Anspruch 1 definierten Bedeutungen haben und R• Wasserstoff, eine Methylgruppe oder eine $(CH_2)_n$L• -Gruppe bedeutet, worin n die in Anspruch 1 definierte Bedeutung hat und L• Wasserstoff oder eine geschützte Aminogruppe bedeutet.

**10.** Pharmazeutische Zusammensetzung, enthaltend als Wirkstoff eine Verbindung der Formel (I) oder (I*) nach einem der Ansprüche 1 oder 2.

**11.** Pharmazeutische Zusammensetzung nach Anspruch 10 in Form einer Dosiseinheit, in der der Wirkstoff mit mindestens einem pharmazeutischen Trägerstoff vermischt ist.

**12.** Zusammensetzung nach Anspruch 11, enthaltend 2,5 bis 1000 mg Wirkstoff.

13. Verbindungen der Formel (I) nach einem der Ansprüche 1 bis 3, in der Z eine Phenylgruppe, eine Benzylgruppe, eine Benzoylgruppe oder eine Phenylthioalkylgruppe mit einer $C_1$-$C_3$-Alkylgruppe bedeutet, wobei diese Gruppen unsubstituiert oder durch ein Halogen oder eine $C_1$-$C_4$-Alkoxygruppe mono- oder disubstituiert sein können.

14. Verbindungen der Formel (I) nach einem der Ansprüche 1 bis 3, worin:

- Ar eine ein- oder mehrfach durch Chlor- oder Fluoratome substituierte Phenylgruppe bedeutet und/oder
- Ar' eine Phenylgruppe, die durch ein Chlor- oder Fluoratom mono- oder disubstituiert ist; eine Imidazolylgruppe oder eine Benzothienylgruppe, die durch ein Chloroder Fluoratom substituiert sind; oder eine durch ein Fluoratom substituierte Naphthylgruppe bedeutet.

**Patentansprüche für folgenden Vertragsstaat : ES**

1. Verfahren zur Herstellung von Verbindungen der Formel

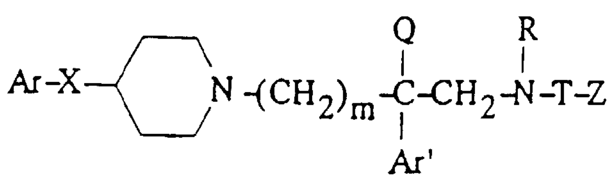

(I)

in der:

- m den Wert 2 oder 3 hat;
- Ar unsubstitiertes oder ein- oder mehrfach durch ein Halogenatom, durch eine $C_1$-$C_3$-Alkylgruppe, durch eine Trifluormethylgruppe, durch eine Alkoxygruppe mit $C_1$-$C_3$-Alkyl, durch eine Hydroxylgruppe oder durch eine Methylendioxygruppe substituiertes Phenyl; eine Thienylgruppe, eine Pyridylgruppe oder eine unsubstituierte oder durch $C_1$-$C_3$-Alkyl substituierte Imidazolylgruppe bedeutet;
- Ar' eine unsubstituierte oder durch ein Halogenatom, durch eine $C_1$-$C_3$-Alkylgruppe, durch eine Trifluormethylgruppe, durch eine Alkoxygruppe mit $C_1$-$C_3$-Alkyl, durch eine Hydroxylgruppe oder durch eine Methylendioxygruppe mono- oder disubstituierte Phenylgruppe; eine Thienylgruppe; eine Imidazolylgruppe oder eine Benzothienylgruppe, die unsubstituiert sind oder durch ein Halogen substituiert sind; eine unsubstituierte oder durch ein Halogen substituierte Naphthylgruppe; eine Biphenylgruppe; eine unsubstituierte oder am Stickstoff durch eine Benzylgruppe substituierte Indolylgruppe bedeutet;
- X ein Sauerstoffatom, ein Schwefelatom, eine Gruppe -NH-, eine Gruppe

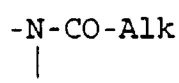

oder eine Gruppe

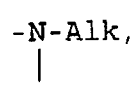

worin Alk eine $C_1$-$C_3$-Alkylgruppe darstellt, bedeutet;
- Q Wasserstoff, eine $C_1$-$C_4$-Alkylgruppe oder eine Aminoalkylgruppe der Formel $-(CH_2)_q$-Am', worin q den Wert 2 oder 3 hat und Am' eine Piperidinogruppe, eine 4-Benzyl-piperidinogruppe oder Dialkylaminogruppe darstellt, worin jeder Alkylrest 1 bis 4 Kohlenstoffatome aufweisen kann, bedeutet;
- R Wasserstoff, eine Methylgruppe oder eine $(CH_2)_n$-L-Gruppe bedeutet, worin n eine ganze Zahl mit einem Wert von 2 bis 6 ist und L Wasserstoff oder eine Aminogruppe darstellt;
- T eine Gruppe bedeutet, die ausgewählt ist unter

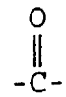 und 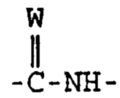

wobei W ein Sauerstoff- oder Schwefelatom bedeutet und

- Z entweder die Bedeutung M oder OM hat, wenn T die Gruppe

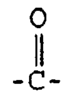

darstellt, oder Z die Bedeutung M hat, wenn T die Gruppe

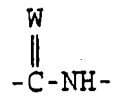

bedeutet;

- M eine der folgenden Bedeutungen hat: Wasserstoff oder eine geradkettige oder verzweigte $C_1$-$C_6$-Alkylgruppe, eine α-Hydroxybenzylgruppe, eine α-Alkylbenzyl- oder Phenylalkylgruppe, in der die Alkylgruppe 1 bis 3 Kohlenstoffatome aufweist, die am aromatischen Ring unsubstituiert oder durch ein Halogenatom, eine Hydroxygruppe, eine Alkoxygruppe mit 1 bis 4 Kohlenstoffatomen oder eine Alkylgruppe mit 1 bis 4 Kohlenstoffatomen mono- oder polysubstituiert ist; eine Pyridylalkylgruppe, in der die Alkylgruppe 1 bis 3 Kohlenstoffatome umfaßt; eine Naphthylalkylgruppe, in der die Alkylgruppe 1 bis 3 Kohlenstoffatome umfaßt; eine Pyridylthioalkylgruppe, in der die Alkylgruppe 1 bis 3 Kohlenstoffatome umfaßt; eine Styrylgruppe; eine (1-Methyl)-imidazolyl-2-thioalkylgruppe, in der die Alkylgruppe 1 bis 3 Kohlenstoffatome umfaßt; eine 1-Oxo-3-phenylindan-2-ylgruppe;

  eine Phenylgruppe, die unsubstituiert ist oder durch einen oder mehrere der aus folgender Gruppe ausgewählten Susbtituenten substituiert ist: Halogen, CN, OH, $NH_2$, NH-CO-$NH_2$, $NO_2$, $CONH_2$, $CF_3$, $C_1$-$C_{10}$-Alkyl, Alkenyl mit 2 bis 10 Kohlenstoffatomen, Alkinyl mit 2 bis 10 Kohlenstoffatomen, Cycloalkyl mit 3 bis 8 Kohlenstoffatomen, Bicycloalkyl mit 4 bis 11 Kohlenstoffatomen, Hydroxyalkyl mit 1 bis 5 Kohlenstoffatomen, Alkoxy mit 1 bis 10 Kohlenstoffatomen, Alkoxyalkyl mit 2 bis 10 Kohlenstoffatomen, Alkoxyalkoxyalkyl mit 3 bis 10 Kohlenstoffatomen, Alkoxyalkoxy mit 2 bis 10 Kohlenstoffatomen, Alkenyloxy mit 2 bis 10 Kohlenstoffatomen, Alkenyloxyalkyl mit 3 bis 10 Kohlenstoffatomen, Alkinyloxy mit 2 bis 10 Kohlenstoffatomen, Alkinyloxyalkyl mit 3 bis 10 Kohlenstoffatomen, Cycloalkoxy mit 3 bis 8 Kohlenstoffatomen, Alkylthio mit 1 bis 10 Kohlenstoffatomen, Alkylthioalkyl mit 2 bis 10 Kohlenstoffatomen, Acylamino mit 1 bis 7 Kohlenstoffatomen, Acylaminoalkyl mit 2 bis 8 Kohlenstoffatomen, Acyloxy mit 1 bis 6 Kohlenstoffatomen, Alkoxycarbonyl mit 2 bis 5 Kohlenstoffatomen, Cycloalkoxycarbonyl mit 4 bis 8 Kohlenstoffatomen, Alkylaminocarbonylamino mit 2 bis 4 Kohlenstoffatomen, Dialkylaminocarbonylamino mit 3 bis 7 Kohlenstoffatomen, Cycloalkylaminocarbonylamino mit 4 bis 8 Kohlenstoffatomen, Alkylaminocarbonylaminoalkyl mit 3 bis 9 Kohlenstoffatomen, Dialkylaminocarbonylaminoalkyl mit 4 bis 11 Kohlenstoffatomen, Cycloalkylaminocarbonylaminoalkyl mit 5 bis 12 Kohlenstoffatomen, Alkoxycarbonylaminoalkyl mit 3 bis 12 Kohlenstoffatomen, Cycloalkoxycarbonylaminoalkyl mit 5 bis 12 Kohlenstoffatomen, Carbamoylalkyl mit 2 bis 5 Kohlenstoffatomen, Alkylaminocarbonylalkyl mit 3 bis 9 Kohlenstoffatomen, Dialkylaminocarbonylalkyl mit 4 bis 11 Kohlenstoffatomen, (1-Pyrrolidino)-carbonylmethyl, (1-Piperidino)-carbonylmethyl, (1-Piperidino)-carbonylethyl, Cycloalkylaminocarbonylalkyl mit 5 bis 12 Kohlenstoffatomen, Alkylaminocarbonylalkoxy mit 3 bis 10 Kohlenstoffatomen, Dialkylaminocarbonylalkoxy mit 4 bis 10 Kohlenstoffatomen, (1-Piperidinyl)-carbonylmethoxy, Cycloalkylaminocarbonylalkoxy mit 5 bis 11 Kohlenstoffatomen, (1-Pyrrolidino)-carbonylamino, (1-Piperdino)-carbonylamino, (1-Pyrrolidino)-carbonylaminoethyl und (1-Piperidino)-carbonylaminoethyl;

  eine Benzoylgruppe oder eine Phenylthioalkylgruppe mit einer $C_1$-$C_3$-Alkylgruppe; wobei diese Gruppen unsubstituiert oder durch ein Halogen oder durch eine $C_1$-$C_4$-Alkoxygruppe mono- oder disubstituiert sein können; eine Naphthyl- oder Indenylgruppe, bei denen eine oder mehrere Bindungen hydriert sein können, wobei diese Gruppen unsubstituiert oder einoder mehrfach durch ein Halogen, eine Alkyl-, Phenyl-, Cyano-, Hydroxyalkyl-, Hydroxy-, Oxo-, Alkylcarbonylamino-, Alkoxycarbonyl- oder Thioalkylgruppe, worin die Alkylgruppen

1 bis 4 Kohlenstoffatome aufweisen, substituiert sind;
eine Pyridyl-, Thiadiazolyl-, Indolyl-, Indazolyl-, Imidazolyl-, Benzimidazolyl-, Chinolyl-, Benzotriazolyl-, Benzofuranyl-, Benzothienyl-, Benzothiazolyl-, Benzisothiazolyl-, Isochinolyl-, Benzoxazolyl-, Benzisoxazolyl-, Benzoxazinyl-, Benzodioxinyl-, Isoxazolyl-, Benzopyranyl-, Thiazolyl-, Thienyl-, Furyl-, Pyranyl-, Chromenyl-, Isobenzofuranyl-, Pyrrolyl-, Pyrazolyl-, Pyrazinyl-, Pyrimidinyl-, Pyridazinyl-, Indolizinyl-, Phthalazinyl-, Chinazolinyl-, Acridinyl-, Isothiazolyl-, Isochromanyl- und Chromanylgruppe, bei denen eine oder mehrere Doppelbindungen hydriert sein können, wobei diese Gruppen unsubstituiert oder ein- oder mehrfach durch eine Alkyl-, Phenyl-, Cyano-, Hydroxyalkyl-, Hydroxy-, Alkylcarbonylamino-, Alkoxycarbonyl- oder Thioalkylgruppe mit $C_1$-$C_4$-Alkylgruppen substituiert sein können;
oder eines Salzes davon mit anorganischen oder organischen Säuren oder eines mit dem Stickstoff von Piperidin gebildeten quaternären Ammoniumsalz davon,

- dadurch gekennzeichnet, daß man

a) ein freies Amin der Formel

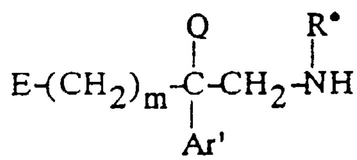

(II)

in der m, Ar' und Q die vorstehend in Anspruch 1 definierten Bedeutungen haben; R Wasserstoff, eine Methylgruppe oder eine $(CH_2)_n$-$L^\bullet$-Gruppe bedeutet, worin n die vorstehend in Anspruch 1 definierte Bedeutung hat und $L^\bullet$ Wasserstoff oder eine Aminogruppe, die durch eine in saurem Medium hydrolysierbare N-Schutzgruppe geschützt ist, bedeutet; und E eine Hydroxygruppe, Methansulfonyloxygruppe, Tetrahydropyranyl-2-oxygruppe oder eine Gruppe der Formel

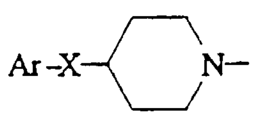

bedeutet oder Ar und X die vorstehend definierten Bedeutungen haben;

- entweder mit einem funktionellen Derivat einer Säure der Formel

HO-CO-Z (III)

in der Z die vorstehend definiert Bedeutung hat, falls eine Verbindung der Formel (I), in der T die Bedeutung -CO-hat, herzustellen ist,
- oder mit einem Iso(thio)cyanat der Formel

W=C=N-Z (III')

in der W und Z die vorstehend definierten Bedeutungen haben, wenn eine Verbindung der Formel (I), worin T die Bedeutung -C(W)-NH- hat, herzustellen ist, behandelt, wodurch eine Verbindung der Formel

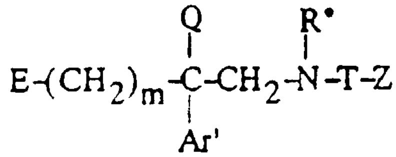 (IV)

entsteht,

b) sodann, wenn E Tetrahydropyranyloxy bedeutet, die Tetrahydropyranylgruppe durch saure Hydrolyse entfernt, wobei die Hydrolyse alternativ in der Stufe (a) am Ausgangsamin der Formel (II) stattfinden kann,

c) Wenn E eine Hydroxylgruppe oder eine Tetrahydropyranyloxygruppe, der hydrolysiert wurde, bedeutet, das auf diese Weise erhaltene N-substituierte Alkanolamin der Formel

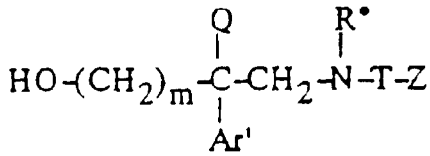 (V)

mit Methansulfonylchlorid behandelt,

d) das in der Stufe c) oder, wenn E eine Methanesulfonyloxygruppe bedeutet, direkt am Ende der Stufe a) erhaltene Mesylat der Formel

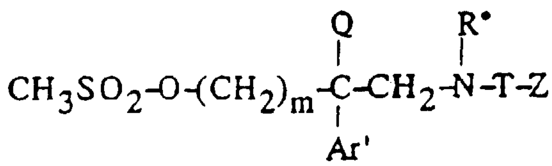 (VI)

mit einem sekundären Amin der Formel

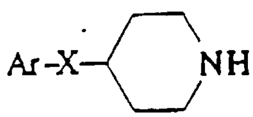 (VII)

in der Ar und X die in Anspruch 1 definierten Bedeutungen haben, umsetzt,

e) die gegebenenfalls vorhandenen N-Schutzgruppen entfernt und gegebenenfalls das in der Stufe d) oder, wenn E eine Gruppe

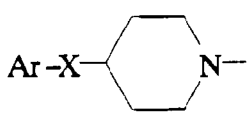

bedeutet, direkt am Ende der Stufe a) erhaltene Produkt in ein Salz überführt.

**2.** Stereoselektives Verfahren zur Herstellung von Verbindungen in optisch reiner Form der Formel

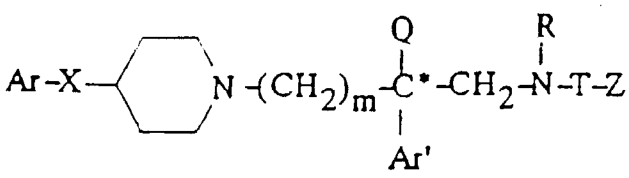

(I*)

in der

- "*" bedeutet, daß das auf diese Weise markierte Kohlenstoffatom eine festgestellte absolute (+)- oder (-)-Konfiguration aufweist;
- m, Ar und Ar', X, Q, R, T und Z die in Anspruch 1 definierten Bedeutungen haben;

oder eines Salzes davon mit anorganischen oder organischen Säuren oder eines Ammoniumsalzes davon mit dem Stickstoff des Piperidins,

dadurch gekennzeichnet, daß man eine Verbindung der Formel

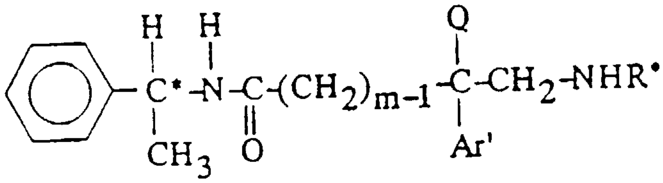

(XVII*)

in der Ar', R• und m die in Anspruch 1 definierten Bedeutungen haben, in einem Lösungsmittel in saurem Medium behandelt, wodurch man eine Aminosäure der Formel

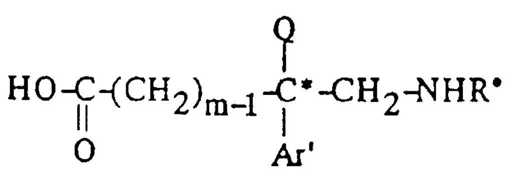

(XVIII*)

erhält, die in einem Alkanol AlkOH, in der Alk eine Alkylgruppe mit 1 bis 4 Kohlenstoffatomen bedeutet, in einem sauren Medium verestert wird, wonach man den entsprechenden Ester der Formel

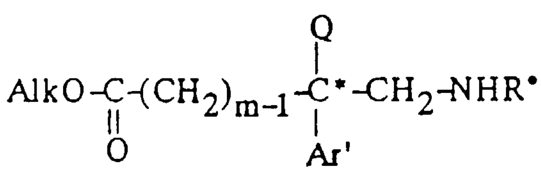

(XIX*)

in der Alk die vorstehend definierte Bedeutung hat; und Q, Ar', R• und m die in Anspruch 1 definierten Bedeutungen haben,

- entweder mit einem funktionellen Derivat einer Säure der Formel

HO-CO-Z (III)

- oder mit einem Iso(thio)cyanat der Formel

W=C=N-Z (III')

worin Z und W die vorstehend in Anspruch 1 definierten Bedeutungen haben, behandelt, den auf diese Weise erhaltenen Ester der Formel

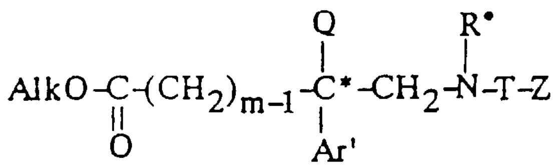 (XX*)

der Einwirkung eines Reduktionsmittels unterwirft und den erhaltenen Alkohol der Formel

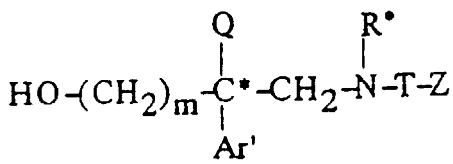 (V*)

in den Methansulfonatester der Formel

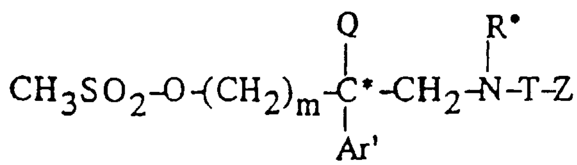 (VI*)

überführt, der dann durch Behandlung mit einem Amin der Formel

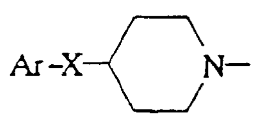 (VII)

in der Ar und X die in Anspruch 1 definierten Bedeutungen haben, die Verbindung der Formel (I*) ergibt; und gegebenenfalls die auf diese Weise erhaltene Verbindung der Formel (I*) in ein Salz überführt.

**3.** Verfahren nach einem der Ansprüche 1 oder 2, dadurch gekennzeichnet, daß die Stufe der Umwandlung der Verbindungen der Formel (I) oder der Formel (I*) in ihre quaternären Ammoniumsalaze darin besteht, daß man die freien Basen der Verbindungen (I) oder (I*), deren gegebenenfalls vorhandene weitere Aminofunktionen mit einer üblichen N-Schutzgruppe N-geschützt sind, mit einem Überschuß eines Alkylyierungsmsittels der Formel

A-Q'

in der A eine austretende Gruppe bedeutet, die der vorstehenden Definition entspricht, umsetzt und das Reaktionsgemisch in einem Lösungsmittel, das z.B. unter Dichlormethan, Chloroform, Aceton oder Acetonitrtil ausgewählt ist, bei einer Temperatur zwischen Umgebungstemperatur und Rückflußtemperatur eine bis mehrere Stunden umsetzt, wodurch man nach Behandlung gemäß üblichen Verfahren und gegebenenfalls nach Schutzgruppenentfernung ein Gemisch von axialen und äquatorialien Konformationsisomeren der quaternären Ammoniumsalze der Verbindungen der Formel (I) oder der Formel (I*) erhält, in der die Gruppe

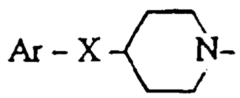

durch folgende Formel wiedergegeben ist,

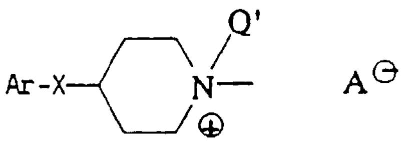

in der

- Q' eine $C_1$-$C_6$-Alkylgruppe oder Benzylgrupppe bedeutet und
- $A^{\ominus}$ ein unter Chlorid, Bromid, Jodid, Acetat, Methansulfonat und p-Toluolsulfonat ausgewähltes Anion bedeutet.

**4.** Verfahren nach Anspruch 1, dadurch gekennzeichnet, daß man eine Verbindung der Formel (II), in der

E = THP-O; m = 2;
Ar' = 3,4-Dichlorphenyl;
$R^{\bullet}$ = $CH_3$; und Q = H;
mit einer Verbindung der Formel (III), in der Z = Phenyl, umsetzt und
daß man in der Stufe d) ein Amin der Formel (VII), in der Ar-X eine Acetylanilinogruppe bedeutet, verwendet, wodurch man die folgende Verbindung erhält:
N-[4-(N'-4-Acetylanilino-1-piperidinyl)-2-(3,4-dichlorphenyl)-butyl]-N-methylbenzamid oder ein pharmazeutisch verträgliches Salz davon.

**5.** Verfahren nach Anspruch 1, dadurch gekennzeichnet, daß man eine Verbindung der Formel (II), in der

E = THP-O; m = 2;
Ar' = Naphthyl;
$R^{\bullet}$ = H; und Q = H;
mit einer Verbindung der Formel (III), in der Z = 2,4-Dimethoxyphenyl, umsetzt und
daß man in der Stufe d) ein Amin der Formel (VII), in der Ar-X eine 1-Methyl-2-thioimidazolylgruppe bedeutet, verwendet, wodurch man die folgende Verbindung erhält:
N-[4-(4-(1-Methyl-2-imidazolylthio)-1-piperidinyl)-2-naphthylbutyl]-2,4-dimethoxybenzamid oder ein pharmazeutisch verträgliches Salz davon.

**6.** Verfahren nach Anspruch 1, dadurch gekennzeichnet, daß man eine Verbindung der Formel (II), in der

E = THP-O; m = 2;
Ar' = 3,4-Dichlorphenyl;
$R^{\bullet}$ = H; und Q = H;
mit einer Verbindung der Formel (III), in der Z = 2,4-Dichlorphenyl, umsetzt und
daß man in der Stufe d) ein Amin der Formel (VII), in der Ar-X eine 2-Thiopyridylgruppe bedeutet, verwendet, wodurch man die folgende Verbindung erhält:
N-[2-(3,4-Dichlorphenyl)-4-(4-(2-pyridylthio)-1-piperidinyl)-butyl]-2,4-dichlorbenzamid oder ein pharmazeutisch verträgliches Salz davon.

**7.** Verfahren zur Herstellung von Verbindungen der Formel

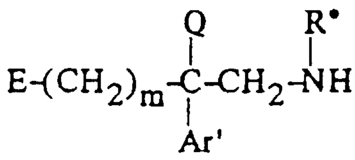

(II)

in der E eine Gruppe der folgenden Formel bedeutet

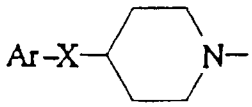

worin m, Q, Ar', Ar und X die in Anspruch 1 definierten Bedeutungen haben und R• Wasserstoff, eine Methylgruppe oder eine $(CH_2)_nL^\bullet$ -Gruppe bedeutet, worin n die in Anspruch 1 definierte Bedeutung hat und L• Wasserstoff oder eine geschützte Aminogruppe bedeutet; dadurch gekennzeichnet, daß man ein Nitril der Formel

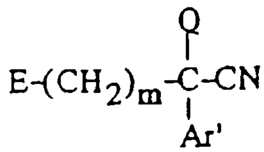

(VIII)

in der m, E, Q und Ar' die vorstehend definierten Bedeutungen haben, reduziert und das auf diese Weise erhaltene Amin gegebenenfalls einer Alkylierung unterwirft.

**8.** Verfahren nach Anspruch 1 oder 3, dadurch gekennzeichnet, daß man in Stufe a) eine Verbindung der Formel (II), in der E, m, Q, Ar' und R• die in Anspruch definierten Bedeutungen haben, mit einer Verbindung der Formel (III) oder (III'), in der Z eine Phenylgruppe, eine Benzylgruppe, eine Benzoylgruppe oder eine Phenylthioalkylgruppe mit $C_1$-$C_3$-Alkyl bedeutet, wobei diese Gruppen unsubstituiert oder durch Halogen oder $C_1$-$C_4$-Alkoxy mono- oder disubstituiert sein können, umsetzt, wodurch man eine Verbindung der Formel (I) erhält, in der Z die vorstehend definierte Bedeutung hat.

**9.** Stereoselektives Verfahren nach Anspruch 2 oder 3, dadurch gekennzeichnet, daß man in Stufe a) eine Verbindung der Formel (III) oder (III'), in der Z eine Phenylgruppe, eine Benzylgruppe, eine Benzoylgruppe oder eine Phenylthioalkylgruppe mit $C_1$-$C_3$-Alkyl bedeutet, wobei diese Gruppen unsubstituiert oder durch Halogen oder $C_1$-$C_4$-Alkoxy mono- oder disubstituiert sein können, verwendet, wodurch man eine Verbindung der Formel (I*) erhält, in der Z die vorstehend definierte Bedeutung hat.

**10.** Verfahren nach Anspruch 1 oder 3, dadurch gekennzeichnet, daß die Gruppe Ar der Verbindungen der Formeln (II) oder (VII) eine ein- oder mehrfach durch ein Chlor- oder Fluoratom substituierte Phenylgruppe bedeutet und/oder Ar' eine durch ein Chlor- oder Fluoratom mono- oder disubstiuierte Phenylgruppe, eine Imidazolylgruppe oder eine Benzothienylgruppe, die durch ein Chlor- oder Fluoratom substituiert sein können, oder eine durch ein Fluoratom substiutierte Naphthylgruppe bedeutet.

**11.** Stereoselektives Verfahren nach Anspruch 2 oder 3, dadurch gekennzeichnet, daß die Gruppe Ar' der Verbindung (XVII*) eine durch ein Chlor- oder Fluoratom mono- oder disubstituierte Phenylgruppe, eine Imidazolylgruppe oder eine Benzothienylgruppe, die durch ein Chlor- oder Fluoratom substituiert sein können; oder eine durch ein Fluoratom substiutierte Naphthylgruppe bedeutet und/oder die Gruppe Ar der Verbindung (VII) eine ein- oder mehrfach durch ein Chloroder Fluoratom substituierte Phenylgruppe bedeutet.

**12.** Verfahren zur Herstellung einer pharmazeutischen Zusammensetzung, dadurch gekennzeichnet, daß man als Wirkstoff eine Verbindung der Formel (I) oder (I*), die durch das Verfahren nach einem der Ansprüche 1 bis 6 hergestellt worden ist, mit einem pharmazeutisch verträglichen Träger vermischt.

**Patentansprüche für folgenden Vertragsstaat : GR**

1. Verbindungen der Formel

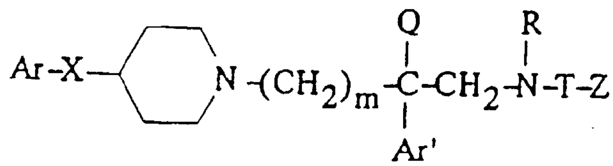 (I)

in der:

- m den Wert 2 oder 3 hat;
- Ar unsubstitiertes oder ein- oder mehrfach durch ein Halogenatom, durch eine $C_1$-$C_3$-Alkylgruppe, durch eine Trifluormethylgruppe, durch eine Alkoxygruppe mit $C_1$-$C_3$-Alkyl, durch eine Hydroxylgruppe oder durch eine Methylendioxygruppe substituiertes Phenyl; eine Thienyigruppe, eine Pyridylgruppe oder eine unsubstituierte oder durch $C_1$-$C_3$-Alkyl substituierte Imidazolylgruppe bedeutet;
- Ar' eine unsubstituierte oder durch ein Halogenatom, durch eine $C_1$-$C_3$-Alkylgruppe, durch eine Trifluormethylgruppe, durch eine Alkoxygruppe mit $C_1$-$C_3$-Alkyl, durch eine Hydroxylgruppe oder durch eine Methylendioxygruppe mono- oder disubstituierte Phenylgruppe; eine Thienylgruppe; eine Imidazolylgruppe oder eine Benzothienylgruppe, die unsubstituiert sind oder durch ein Halogen substituiert sind; eine unsubstituierte oder durch ein Halogen substituierte Naphthylgruppe; eine Biphenylgruppe; eine unsubstituierte oder am Stickstoff durch eine Benzylgruppe substituierte Indolylgruppe bedeutet;
- X ein Sauerstoffatom, ein Schwefelatom, eine Gruppe -NH-, eine Gruppe

  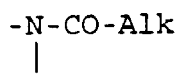

  oder eine Gruppe

  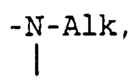

  worin Alk eine $C_1$-$C_3$-Alkylgruppe darstellt, bedeutet;
- Q Wasserstoff, eine $C_1$-$C_4$-Alkylgruppe oder eine Aminoalkylgruppe der Formel $-(CH_2)_q$-Am', worin q den Wert 2 oder 3 hat und Am' eine Piperidinogruppe, eine 4-Benzyl-piperidinogruppe oder Dialkylaminogruppe darstellt, worin jeder Alkylrest 1 bis 4 Kohlenstoffatome aufweisen kann, bedeutet;
- R Wasserstoff, eine Methylgruppe oder eine $(CH_2)_n$-L-Gruppe bedeutet, worin n eine ganze Zahl mit einem Wert von 2 bis 6 ist und L Wasserstoff oder eine Aminogruppe darstellt;
- T eine Gruppe bedeutet, die ausgewählt ist unter

  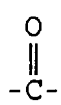 und 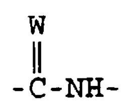

  wobei W ein Sauerstoff- oder Schwefelatom bedeutet und
- Z entweder die Bedeutung M oder OM hat, wenn T die Gruppe

  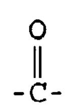

darstellt, oder Z die Bedeutung M hat, wenn T die Gruppe

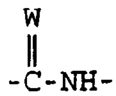

bedeutet;

- M eine der folgenden Bedeutungen hat: Wasserstoff oder eine geradkettige oder verzweigte $C_1$-$C_6$-Alkylgruppe, eine α-Hydroxybenzylgruppe, eine α-Alkylbenzyl- oder Phenylalkylgruppe, in der die Alkylgruppe 1 bis 3 Kohlenstoffatome aufweist, die am aromatischen Ring unsubstituiert oder durch ein Halogenatom, eine Hydroxygruppe, eine Alkoxygruppe mit 1 bis 4 Kohlenstoffatomen oder eine Alkylgruppe mit 1 bis 4 Kohlenstoffatomen mono- oder polysubstituiert ist; eine Pyridylalkylgruppe, in der die Alkylgruppe 1 bis 3 Kohlenstoffatome umfaßt; eine Naphthylalkylgruppe, in der die Alkylgruppe 1 bis 3 Kohlenstoffatome umfaßt; eine Pyridylthioalkylgruppe, in der die Alkylgruppe 1 bis 3 Kohlenstoffatome umfaßt; eine Styrylgruppe; eine (1-Methyl)-imidazolyl-2-thioalkylgruppe, in der die Alkylgruppe 1 bis 3 Kohlenstoffatome umfaßt; eine 1-Oxo-3-phenylindan-2-ylgruppe;

  eine Phenylgruppe, die unsubstituiert ist oder durch einen oder mehrere der aus folgender Gruppe ausgewählten Susbtituenten substituiert ist: Halogen, CN, OH, $NH_2$, NH-CO-$NH_2$, $NO_2$, $CONH_2$, $CF_3$, $C_1$-$C_{10}$-Alkyl, Alkenyl mit 2 bis 10 Kohlenstoffatomen, Alkinyl mit 2 bis 10 Kohlenstoffatomen, Cycloalkyl mit 3 bis 8 Kohlenstoffatomen, Bicycloalkyl mit 4 bis 11 Kohlenstoffatomen, Hydroxyalkyl mit 1 bis 5 Kohlenstoffatomen, Alkoxy mit 1 bis 10 Kohlenstoffatomen, Alkoxyalkyl mit 2 bis 10 Kohlenstoffatomen, Alkoxyalkoxyalkyl mit 3 bis 10 Kohlenstoffatomen, Alkoxyalkoxy mit 2 bis 10 Kohlenstoffatomen, Alkenyloxy mit 2 bis 10 Kohlenstoffatomen, Alkenyloxyalkyl mit 3 bis 10 Kohlenstoffatomen, Alkinyloxy mit 2 bis 10 Kohlenstoffatomen, Alkinyloxyalkyl mit 3 bis 10 Kohlenstoffatomen, Cycloalkoxy mit 3 bis 8 Kohlenstoffatomen, Alkylthio mit 1 bis 10 Kohlenstoffatomen, Alkylthioalkyl mit 2 bis 10 Kohlenstoffatomen, Acylamino mit 1 bis 7 Kohlenstoffatomen, Acylaminoalkyl mit 2 bis 8 Kohlenstoffatomen, Acyloxy mit 1 bis 6 Kohlenstoffatomen, Alkoxycarbonyl mit 2 bis 5 Kohlenstoffatomen, Cycloalkoxycarbonyl mit 4 bis 8 Kohlenstoffatomen, Alkylaminocarbonylamino mit 2 bis 4 Kohlenstoffatomen, Dialkylaminocarbonylamino mit 3 bis 7 Kohlenstoffatomen, Cycloalkylaminocarbonylamino mit 4 bis 8 Kohlenstoffatomen, Alkylaminocarbonylaminoalkyl mit 3 bis 9 Kohlenstoffatomen, Dialkylaminocarbonylaminoalkyl mit 4 bis 11 Kohlenstoffatomen, Cycloalkylaminocarbonylaminoalkyl mit 5 bis 12 Kohlenstoffatomen, Alkoxycarbonylaminoalkyl mit 3 bis 12 Kohlenstoffatomen, Cycloalkoxycarbonylaminoalkyl mit 5 bis 12 Kohlenstoffatomen, Carbamoylalkyl mit 2 bis 5 Kohlenstoffatomen, Alkylaminocarbonylalkyl mit 3 bis 9 Kohlenstoffatomen, Dialkylaminocarbonylalkyl mit 4 bis 11 Kohlenstoffatomen, (1-Pyrrolidino)-carbonylmethyl, (1-Piperidino)-carbonylmethyl, (1-Piperidino)-carbonylethyl, Cycloalkylaminocarbonylalkyl mit 5 bis 12 Kohlenstoffatomen, Alkylaminocarbonylalkoxy mit 3 bis 10 Kohlenstoffatomen, Dialkylaminocarbonylalkoxy mit 4 bis 10 Kohlenstoffatomen, (1-Piperidinyl)-carbonylmethoxy, Cycloalkylaminocarbonylalkoxy mit 5 bis 11 Kohlenstoffatomen, (1-Pyrrolidino)-carbonylamino, (1-Piperdino)-carbonylamino, (l-Pyrrolidino)-carbonylaminoethyl und (1-Piperidino)-carbonylaminoethyl;

  eine Benzoylgruppe oder eine Phenylthioalkylgruppe mit einer $C_1$-$C_3$-Alkylgruppe; wobei diese Gruppen unsubstituiert oder durch ein Halogen oder durch eine $C_1$-$C_4$-Alkoxygruppe mono- oder disubstituiert sein können; eine Naphthyl- oder Indenylgruppe, bei denen eine oder mehrere Bindungen hydriert sein können, wobei diese Gruppen unsubstituiert oder einoder mehrfach durch ein Halogen, eine Alkyl-, Phenyl-, Cyano-, Hydroxyalkyl-, Hydroxy-, Oxo-, Alkylcarbonylamino-, Alkoxycarbonyl- oder Thioalkylgruppe, worin die Alkylgruppen 1 bis 4 Kohlenstoffatome aufweisen, substituiert sind;

  eine Pyridyl-, Thiadiazolyl-, Indolyl-, Indazolyl-, Imidazolyl-, Benzimidazolyl-, Chinolyl-, Benzotriazolyl-, Benzofuranyl-, Benzothienyl-, Benzothiazolyl-, Benzisothiazolyl-, Isochinolyl-, Benzoxazolyl-, Benzisoxazolyl-, Benzoxazinyl-, Benzodioxinyl-, Isoxazolyl-, Benzopyranyl-, Thiazolyl-, Thienyl-, Furyl-, Pyranyl-, Chromenyl-, Isobenzofuranyl-, Pyrrolyl-, Pyrazolyl-, Pyrazinyl-, Pyrimidinyl-, Pyridazinyl-, Indolizinyl-, Phthalazinyl-, Chinazolinyl-, Acridinyl-, Isothiazolyl-, Isochromanyl- und Chromanylgruppe, bei denen eine oder mehrere Doppelbindungen hydriert sein können, wobei diese Gruppen unsubstituiert oder ein- oder mehrfach durch eine Alkyl-, Phenyl-, Cyano-, Hydroxyalkyl-, Hydroxy-, Alkylcarbonylamino-, Alkoxycarbonyl- oder Thioalkylgruppe mit $C_1$-$C_4$-Alkylgruppen substituiert sein können;

  oder ein Salz davon mit anorganischen oder organischen Säuren oder ein mit dem Stickstoff von Piperidin gebildetes quaternäres Ammoniumsalz davon.

**2.** Verbindungen nach Anspruch 1 in optisch reiner Form der Formel

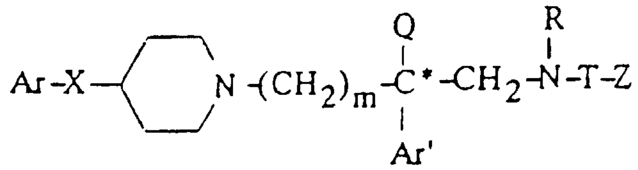

(I*)

in der

- "*" bedeutet, daß das auf diese Weise markierte Kohlenstoffatom eine festgestellte absolute (+)- oder (-)-Konfiguration aufweist;
- m, Ar und Ar', X, Q, R, T und Z die in Anspruch 1 definierten Bedeutungen haben;

oder ein Salz davon mit anorganischen oder organischen Säuren oder ein Ammoniumsalz davon mit dem Stickstoff des Piperidins.

3. Verbindungen nach einem der Ansprüche 1 oder 2, dadurch gekennzeichnet, daß sie in Form eines quaternären Ammoniumsalzes vorliegen, deren Gruppe der Formel

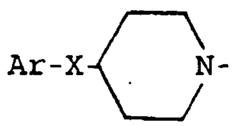

dann durch die folgende Gruppe wiedergeben wird:

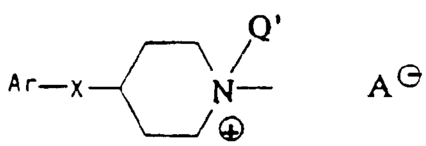

in der

- Q' eine $C_1$-$C_6$-Alkylgruppe oder eine Benzylgruppe bedeutet und
- $A^{\ominus}$ ein unter Chlorid, Bromid, Iodid, Acetat, Methansulfonat oder p-Toluolsulfonat ausgewähltes Anion bedeutet.

4. Verbindung nach Anspruch 1, nämlich N-[2-(3,4-Dichlorphenyl)-4-(4-(2-pyridylthio)-1-piperidinyl)-butyl]-2,4dichlorbenzamid oder ein pharmazeutisch verträgliches Salz davon.

5. Verbindung nach Anspruch 1, nämlich N-[4-(N'-4-Acetylanilino-1-piperidinyl)-2-(3,4-dichlorphenyl)-butyl]-N-methylbenzamid oder ein pharmazeutisch verträgliches Salz davon.

6. Verbindung nach Anspruch 1, nämlich N-[4-(4-(1-Methyl-2-imidazolylthio)-1-piperidinyl)-2-naphthylbutyl]-2,4-dimethoxybenzamid oder ein pharmazeutisch verträgliches Salz davon.

7. Verfahren zur Herstellung der Verbindungen der Formel (I) nach Anspruch 1, dadurch gekennzeichnet, daß man

a) ein freies Amin der Formel

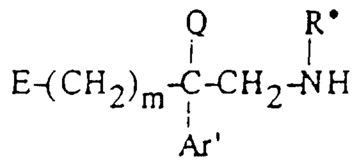

(II)

in der m, Ar' und Q die vorstehend in Anspruch 1 definierten Bedeutungen haben; $R^{\bullet}$ Wasserstoff, eine Methylgruppe oder eine $(CH_2)_n$-$L^{\bullet}$-Gruppe bedeutet, worin n die vorstehend in Anspruch 1 definierte Bedeutung hat und $L^{\bullet}$ Wasserstoff oder eine Aminogruppe, die durch eine in saurem Medium hydrolysierbare N-Schutzgruppe geschützt ist, bedeutet; und E eine Hydroxygruppe, Methansulfonyloxygruppe, Tetrahydropyranyl-2-oxygruppe oder eine Gruppe der Formel

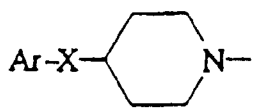

bedeutet oder Ar und X die in Anspruch 1 definierten Bedeutungen haben;

- entweder mit einem funktionellen Derivat einer Säure der Formel

HO-CO-Z (III)

in der Z die in Anspruch 1 definierte Bedeutung hat, falls eine Verbindung der Formel (I), in der T die Bedeutung -CO- hat, herzustellen ist,

- oder mit einem Iso(thio)cyanat der Formel

W=C=N-Z (III')

in der W und Z die in Anspruch 1 definierten Bedeutungen haben, wenn eine Verbindung der Formel (I), worin T die Bedeutung -C(W)-NH- hat, herzustellen ist, behandelt, wodurch eine Verbindung der Formel

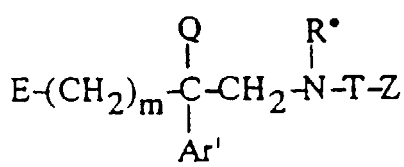

(IV)

entsteht,

b) sodann, wenn E Tetrahydropyranyloxy bedeutet, die Tetrahydropyranylgruppe durch saure Hydrolyse entfernt, wobei die Hydrolyse alternativ in der Stufe (a) am Ausgangsamin der Formel (II) stattfinden kann,

c) Wenn E eine Hydroxylgruppe oder eine Tetrahydropyranyloxygruppe, der hydrolysiert wurde, bedeutet, das auf diese Weise erhaltene N-substituierte Alkanolamin der Formel

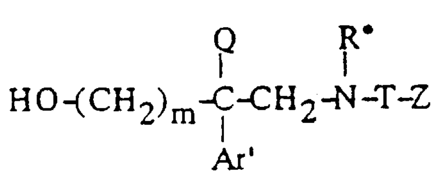

(V)

mit Methansulfonylchlorid behandelt,

d) das in der Stufe c) oder, wenn E eine Methanesulfonyloxygruppe bedeutet, direkt am Ende der Stufe a) erhaltene Mesylat der Formel

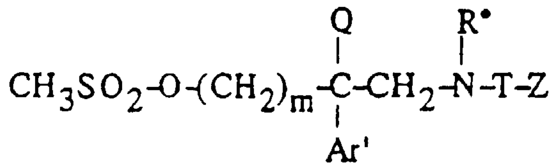

(VI)

mit einem sekundären Amin der Formel

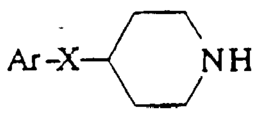

(VII)

in der Ar und X die in Anspruch 1 definierten Bedeutungen haben, umsetzt,

e) die gegebenenfalls vorhandenen N-Schutzgruppen entfernt und gegebenenfalls das in der Stufe d) oder, wenn E eine Gruppe

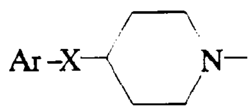

bedeutet, direkt am Ende der Stufe a) erhaltene Produkt in ein Salz überführt.

**8.** Stereoselektives Verfahren zur Herstellung der optisch reinen Verbindungen der Formel (I*) gemäß Anspruch 2, dadurch gekennzeichnet, daß man eine Verbindung der Formel

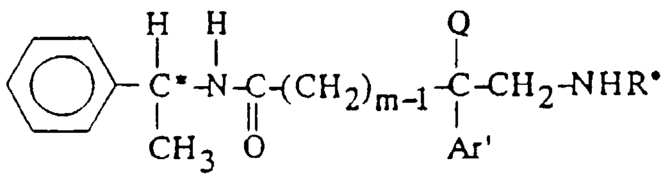

(XVII*)

in einem Lösungsmittel in saurem Medium behandelt, wodurch man eine Aminosäure der Formel

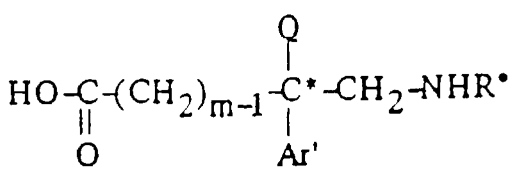

(XVIII*)

erhält, die in einem Alkanol AlkOH, in der Alk eine Alkylgruppe mit 1 bis 4 Kohlenstoffatomen bedeutet, in einem sauren Medium verestert wird, wonach man den entsprechenden Ester der Formel

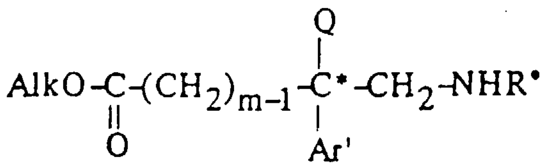
(XIX*)

in der Alk die vorstehend definierte Bedeutung hat; Q, Ar' und m die in Anspruch 1 definierten Bedeutungen haben und R• die in Anspruch 7 definierte Bedeutung hat,

- entweder mit einem funktionellen Derivat einer Säure der Formel

$$HO\text{-}CO\text{-}Z \qquad (III)$$

- oder mit einem Iso(thio)cyanat der Formel

$$W{=}C{=}N\text{-}Z \qquad (III')$$

worin Z und W die vorstehend in Anspruch 1 definierten Bedeutungen haben, behandelt,

- den auf diese Weise erhaltenen Ester der Formel

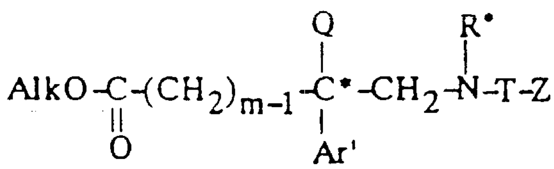
(XX*)

der Einwirkung eines Reduktionsmittels unterwirft und den erhaltenen Alkohol der Formel

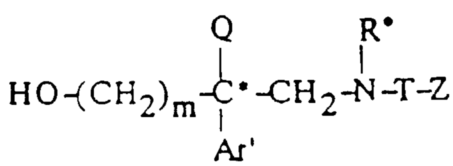
(V*)

in den Methansulfonatester der Formel

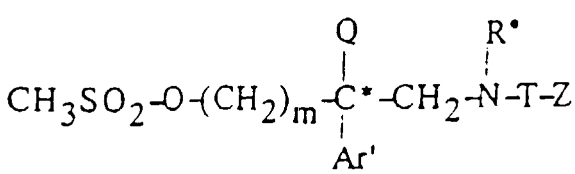
(VI*)

überführt, der dann durch Behandlung mit einem Amin der Formel

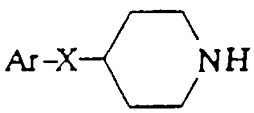

(VII)

in der Ar und X die in Anspruch 1 definierten Bedeutungen haben, die Verbindung der Formel (I*) ergibt.

**9.** Verbindung der Formel

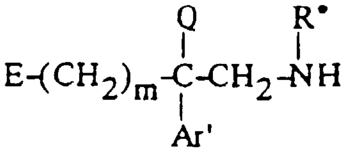

(II)

in der E eine Gruppe der folgenden Formel bedeutet

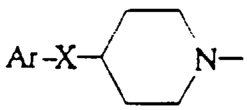

worin m, Q, Ar', Ar und X die in Anspruch 1 definierten Bedeutungen haben und R• Wasserstoff, eine Methylgruppe oder eine $(CH_2)_nL^{\bullet}$ -Gruppe bedeutet, worin n die in Anspruch 1 definierte Bedeutung hat und L• Wasserstoff oder eine geschützte Aminogruppe bedeutet.

**10.** Verbindungen der Formel (I) nach einem der Ansprüche 1 bis 3, in der Z eine Phenylgruppe, eine Benzylgruppe, eine Benzoylgruppe oder eine Phenylthioalkylgruppe mit einer $C_1$-$C_3$-Alkylgruppe bedeutet, wobei diese Gruppen unsubstituiert oder durch ein Halogen oder eine $C_1$-$C_4$-Alkoxygruppe mono- oder disubstituiert sein können.

**11.** Verbindungen der Formel (I) nach einem der Ansprüche 1 bis 3, worin:

- Ar eine ein- oder mehrfach durch Chlor- oder Fluoratome substituierte Phenylgruppe bedeutet und/oder
- Ar' eine Phenylgruppe, die durch ein Chlor- oder Fluoratom mono- oder disubstituiert ist; eine Imidazolylgruppe oder eine Benzothienylgruppe, die durch ein Chloroder Fluoratom substituiert sind; oder eine durch ein Fluoratom substituierte Naphthylgruppe bedeutet.

**12.** Verfahren zur Herstellung einer pharmazeutischen Zusammensetzung, dadurch gekennzeichnet, daß man als Wirkstoff eine Verbindung der Formel (I) oder (I*) nach einem der Ansprüche 1 bis 6 mit einem pharmazeutisch verträglichen Träger vermischt.

**Claims**

**Claims for the following Contracting States : AT, BE, CH, LI, DE, DK, FR, GB, IT, LU, NL, PT, SE.**

**1.** Compounds of formula

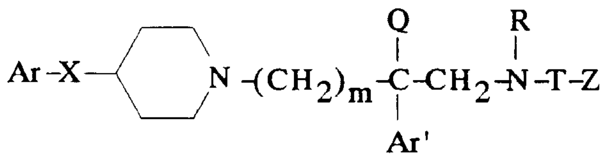 (I)

in which:

- m is equal to 2 or 3;
- Ar represents a phenyl, unsubstituted or substituted one or more times with a halogen atom, with a $C_1$-$C_3$ alkyl, with a trifluoromethyl, with an alkoxy in which the alkyl is a $C_1$-$C_3$ group, with a hydroxyl or with a methylenedioxy; a thienyl, pyridyl or imidazolyl group which is or is not substituted with a $C_1$-$C_3$ alkyl;
- Ar' represents a phenyl group, unsubstituted or mono- or disubstituted with a halogen atom, with a $C_1$-$C_3$ alkyl, with a trifluoromethyl, with an alkoxy in which the alkyl is a $C_1$-$C_3$ group, with a hydroxyl or with a methylenedioxy; a thienyl group; an imidazolyl group or a benzothienyl group, each of which is unsubstituted or substituted with a halogen; a naphthyl group unsubstituted or substituted with a halogen; a biphenyl group; an indolyl unsubstituted or substituted on the nitrogen with a benzyl group;
- X represents an oxygen atom, a sulphur atom, an -NH- group, an -N-CO-Alk group or an -N-Alk group in which Alk is a $C_1$-$C_3$ alkyl group;
- Q represents hydrogen, a $C_1$-$C_4$ alkyl group or an aminoalkyl group of formula $-(CH_2)_q$-Am', where q is 2 or 3 and Am' is a piperidino, 4-benzylpiperidino or dialkylamino group, it being possible for each alkyl to contain 1 to 4 carbon atoms;
- R represents hydrogen, a methyl group or a $(CH_2)_n$-L group, where n is an integer from 2 to 6 and L is hydrogen or an amino group;
- T represents a group chosen amongst

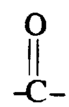 and 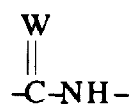

W being an oxygen or sulphur atom, and
- Z represents either M or OM when T represents the:

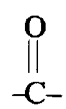 group,

or M when T represents the group

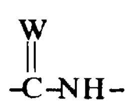 ;

M represents hydrogen or a linear or branched $C_1$-$C_6$ alkyl; an α-hydroxybenzyl, an α-alkylbenzyl or a phenylalkyl in which the alkyl group contains 1 to 3 carbon atoms, unsubstituted, mono- or polysubstituted on the aromatic ring with a halogen, a hydroxyl, an alkoxy of 1 to 4 carbon atoms, an alkyl of 1 to 4 carbon atoms; a pyridylalkyl in which the alkyl group contains 1 to 3 carbon atoms; a naphthylalkyl in which the alkyl group contains 1 to 3 carbon atoms; a pyridylthioalkyl in which the alkyl group contains 1 to 3 carbon atoms; a styryl; a 1-methyl-2-imidazolylthioalkyl in which the alkyl group contains 1 to 3 carbon atoms; a 1-oxophenyl-3-indan-2-yl; a phenyl group which is unsubstituted or substituted by one or more substituents chosen amongst halogen, CN, OH, $NH_2$, NH-CO-$NH_2$, $NO_2$, $CONH_2$, $CF_3$, $C_1$-$C_{10}$ alkyl, alkenyl containing 2 to 10 carbon atoms,

alkynyl containing 2 to 10 carbon atoms, cycloalkyl containing 3 to 8 carbon atoms, bicycloalkyl containing 4 to 11 carbon atoms, hydroxyalkyl containing 1 to 5 carbon atoms, alkoxy containing 1 to 10 carbon atoms, alkoxyalkyl containing 2 to 10 carbon atoms, alkoxyalkoxyalkyl containing 3 to 10 carbon atoms, alkoxyalkoxy containing 2 to 10 carbon atoms, alkenyloxy containing 2 to 10 carbon atoms, alkenyloxyalkyl containing 3 to 10 carbon atoms, alkynyloxy containing 2 to 10 carbon atoms, alkynyloxyalkyl containing 3 to 10 carbon atoms, cycloalkoxy containing 3 to 8 carbon atoms, alkylthio containing 1 to 10 carbon atoms, alkylthioalkyl containing 2 to 10 carbon atoms, acylamino containing 1 to 7 carbon atoms, acylaminoalkyl containing 2 to 8 carbon atoms, acyloxy containing 1 to 6 carbon atoms, alkoxycarbonyl containing 2 to 5 carbon atoms, cycloalkoxycarbonyl containing 4 to 8 carbon atoms, alkylaminocarbonylamino containing 2 to 4 carbon atoms, dialkylaminocarbonylamino containing 3 to 7 carbon atoms, cycloalkylaminocarbonylamino containing 4 to 8 carbon atoms, alkylaminocarbonylaminoalkyl containing 3 to 9 carbon atoms, dialkylaminocarbonylaminoalkyl containing 4 to 11 carbon atoms, cycloalkylaminocarbonylaminoalkyl containing 5 to 12 carbon atoms, alkoxycarbonylaminoalkyl containing 3 to 12 carbon atoms, cycloalkoxycarbonylaminoalkyl containing 5 to 12 carbon atoms, carbamoylalkyl containing 2 to 5 carbon atoms, alkylaminocarbonylalkyl containing 3 to 9 carbon atoms, dialkylaminocarbonylalkyl containing 4 to 11 carbon atoms, (1-pyrrolidono)carbonylmethyl, (1-piperidino)carbonylmethyl, (1-pipeddino)carbonylethyl, cycloalkylaminocarbonylalkyl containing 5 to 12 carbon atoms, alkylaminocarbonylalkoxy containing 3 to 10 carbon atoms, dialkylaminocarbonylalkoxy containing 4 to 10 carbon atoms, (piperidinyl-1)carbonylmethoxy, cycloalkylaminocarbonylalkoxy containing 5 to 11 carbon atoms, (1-pyrrolidino)-carbonylamino, (1-piperidino)-carbonylamino, (1-pyrrolidino)-carbonylaminoethyl, (1-piperidino)-carbonylaminoethyl;

a benzoyl group or a phenylthioalkyl group in which the alkyl is a $C_1$-$C_3$ group, it being possible for said groups to be unsubstituted or mono- or disubstituted with a halogen or with a $C_1$-$C_4$ alkoxy; a naphthyl or indenyl group, in which one or more bonds may be hydrogenated, it being possible for said groups to be unsubstituted or mono- or polysubstituted with a halogen, an alkyl, phenyl, cyano, hydroxyalkyl, hydoxyl, oxo, alkylcarbonylamino, alkoxycarbonyl, thioalkyl group in which the alkyl is a $C_1$-$C_4$ group;

a pyridyl, thiadiazolyl, indolyl, indazolyl, imidazolyl, benzimidazolyl, quinolyl, benzotriazolyl, benzofuranyl, benzothienyl, benzothiazolyl, benzisothiazolyl, isoquinolyl, benzoxazolyl, benzisoxazolyl, benzoxazinyl, benzodioxinyl, isoxazolyl, benzopyranyl, thiazolyl, thienyl, furyl, pyranyl, chromenyl, isobenzofuranyl, pyrrolyl, pyrazolyl, pyrazinyl, pyrimidinyl, pyridazinyl, indolizinyl, phthalazinyl, quinazolinyl, acridinyl, isothiazolyl, isochromanyl and chromanyl group, in which one or more double bonds may be hydrogenated, it being possible for said groups to be unsubstituted or mono- or polysubstituted with an alkyl, phenyl, cyano, hydroxyalkyl, hydroxy, alkylcarbonylamino, alkoxycarbonyl and thioalkyl groups, in which groups the alkyls are $C_1$-$C_4$ groups;

or one of their salts with inorganic or organic acids or one of their quaternary ammonium salts formed with the nitrogen of the piperidine.

**2.** Compounds according to claim 1, in the optically pure form, of formula:

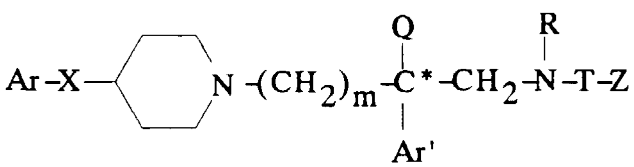

(I*)

in which :

- "*" denotes that the carbon atom thus marked has a determined (+) or (-) absolute configuration;
- m, Ar and Ar', X, Q, R, T and Z are as defined in claim 1.

or one of their salts with inorganic or organic acids or one of their ammonium salts formed with the nitrogen of the piperidine.

**3.** Compounds according to one of claims 1 or 2, characterised in that they are in the form of a quaternary ammium salt, the group of formula

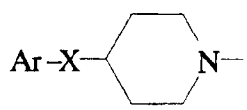

being then represented by the group:

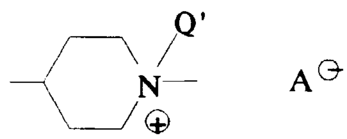

in which

- . Q' represents a $C_1$-$C_6$ alkyl group or a benzyl group and
- . $A^-$ represents an anion chosen amongst chloride, bromide, iodide, acetate, methanesulphonate or paratoluenesulphonate.

**4.** Compound according to claim 1, which is the N-[2-(3,4-Dichlorophenyl)-4-(4-(2-pyridylthio)-1-piperidinyl)butyl]-2,4-dichlorobenzamide or one of its pharmaceutically acceptable salts.

**5.** Compound according to claim 1, which is the N-[4-(N'-4-Acetylanilino-1-piperidinyl)-2-(3,4-dichlorophenyl)-butyl]-N-methylbenzamide or one of its pharmaceutically acceptable salts.

**6.** Compound according to claim 1, which is the N-[4-(4-(1-Methyl-2-imidazolylthio)-1-piperidinyl)-2-naphtylbutyl]-2,4-dimethoxybenzamide or one of its pharmaceutically acceptable salts.

**7.** Process for the preparation of compounds of formula (I) according to claim 1, characterised in that

a) a free amine of formula:

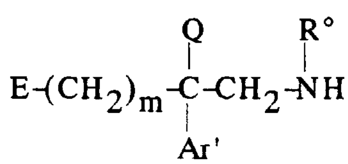

(II)

in which m, Ar' and Q are as defined above in claim 1; R° represents hydrogen, a methyl group or a $(CH_2)_n$-L° group, where n is as defined above in claim 1 and L° is hydrogen or an amino group protected by an N-protecting group hydrolysable in an acidic medium; and E represents a hydroxy, methanesulphonyloxy, tetrahydro-2-pyranyloxy group, or a group:

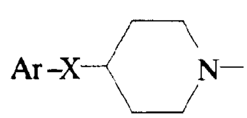

where Ar and X are as defined in claim 1, is treated

- either with a functional derivative of an acid of formula:

HO-CO-Z (III)

in which Z is as defined in claim 1, when a compound of formula (I) where T is -CO- is to be prepared,

- or with an iso(thio)cyanate of formula:

W = C = N-Z (III')

in which W and Z are as defined in claim 1, when a compound of formula (I) where T is -C(W)-NH- is to be prepared, to form the compound of formula:

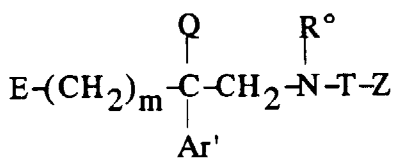
(IV)

b) then, when E represents tetrahydropyranyloxy, the tetrahydropyranyl group is removed by acid hydrolysis, it alternatively being possible for the hydrolysis to take place in step (a) on the starting amine of formula (II),
c) when E is a hydroxy group or a tetrahydropyranyloxy group which has been hydrolyzed, the N-substituted alkanolamine thereby obtained, of formula:

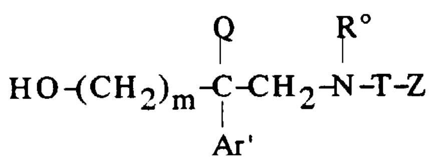
(V)

is treated with methanesulphonyl chloride,
d) the mesylate thereby obtained in step (c), or directly at the end of step (a) when E is a methanesulfonyloxy group, of formula:

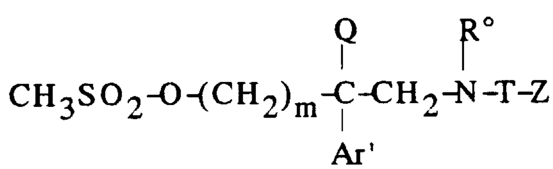
(VI)

is reacted with a secondary amine of formula:

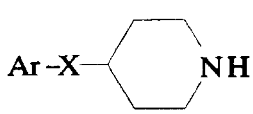
(VII)

in which Ar and X are as defined in claim 1,
e) the N-protecting groups, where appropriate, are removed and the product thereby obtained in step (d) or directly at the end of step (a) when E is a group

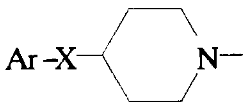

is optionally converted to one of its salts.

**8.** Stereoselective process for the preparation of optically pure compounds of formula (I*) according to claim 2, characterised in that a compound of formula:

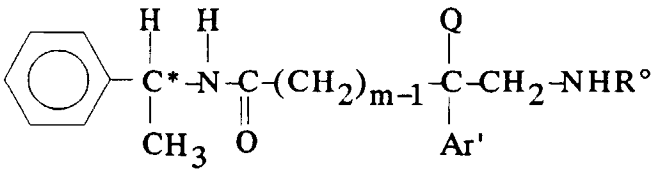

(XVII*)

is treated in a solvent, in acidic medium, to yield the amino acid of formula:

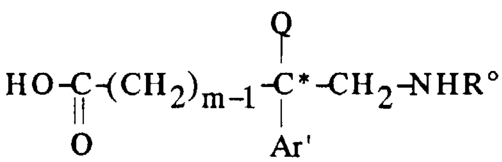

(XVIII*)

which is esterified in an alcohol AlkOH, in which Alk is an alkyl of 1 to 4 carbon atoms, in acidic medium, then the corresponding ester of formula:

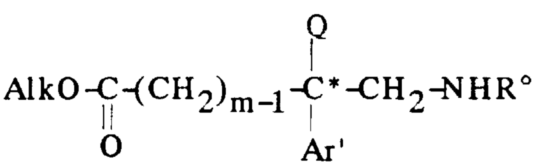

(XIX*)

in which Alk is as defined above; Q, Ar', and m are as defined in claim 1 and R° is as defined in claim 7, is treated

- either with a functional derivative of an acid of formula:

HO-CO-Z (III)

- or with an iso(thio)cyanate of formula:

W=C=N-Z (III')

Z and W being as defined in claim 1, the ester thus obtained of formula:

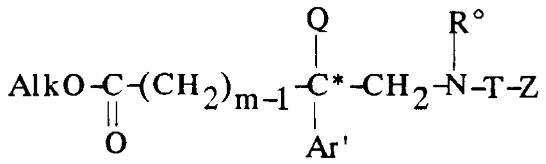 (XX*)

is subjected to the action of a reducing agent and the corresponding alcohol of formula:

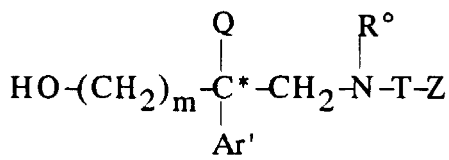 (V*)

is converted into its methanesulphonate ester of formula:

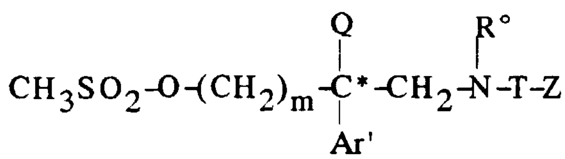 (VI*)

which, by treatment with an amine of formula:

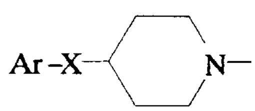 (VII)

in which Ar and X are as defined in claim 1, gives the compound of formula (I*).

**9.** Compound of formula:

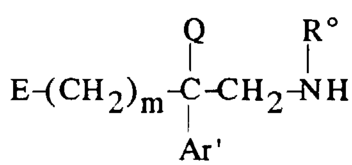 (II)

in which E represents the group of formula:

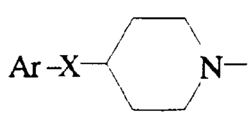

m, Q, Ar', Ar and X are as defined in claim 1 and R° represents hydrogen, a methyl group or a $(CH_2)_n$-L° group where n is as defined in claim 1 and L° is hydrogen or a protected amino group.

**10.** Pharmaceutical composition containing as active principle a compound of formula (I) or (I*) according to one of

claims 1 or 2.

**11.** Pharmaceutical composition according to claim 10, in the form of a dosage unit, in which the active principle is mixed with at least one pharmaceutical excipient.

**12.** Composition according to claim 11 containing from 2.5 to 1000 mg of active principle.

**13.** Compounds of formula (I) according to any one of claims 1 to 3, in which Z represents a phenyl group, a benzyl group, a benzoyl group, a phenylthioalkyl group in which the alkyl is a $C_1$-$C_3$ group, said groups being unsubstituted, mono- or disubstituted with a halogen or with a $C_1$-$C_4$ alkoxy.

**14.** Compounds of formula (I) according to any one of claims 1 to 3, in which:

- Ar represents a phenyl which is mono- or polysubstituted with a chlorine or fluorine atom, and/or
- Ar' represents a phenyl group which is mono- or disubstituted with a chlorine or fluorine atom; an imidazolyl group or a benzothienyl group substituted with a chlorine or fluorine atom; a naphthyl group substituted with a fluorine atom.

**Claims for the following Contracting State : ES**

**1.** Process for the preparation of compounds of formula

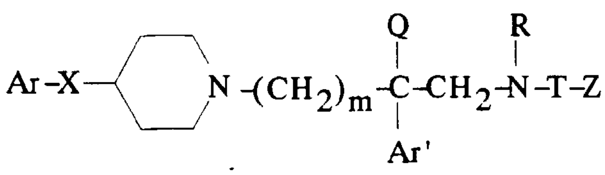

(I)

in which:

- m is equal to 2 or 3;
- Ar represents a phenyl, unsubstituted or substituted one or more times with a halogen atom, with a $C_1$-$C_3$ alkyl, with a trifluoromethyl, with an alkoxy in which the alkyl is a $C_1$-$C_3$ group, with a hydroxyl or with a methylenedioxy; a thienyl, pyridyl or imidazolyl group which is or is not substituted with a $C_1$-$C_3$ alkyl;
- Ar' represents a phenyl group, unsubstituted or mono- or disubstituted with a halogen atom, with a $C_1$-$C_3$ alkyl, with a trifluoromethyl, with an alkoxy in which the alkyl is a $C_1$-$C_3$ group, with a hydroxyl or with a methylenedioxy; a thienyl group; an imidazolyl group or a benzothienyl group, each of which is unsubstituted or substituted with a halogen; a naphthyl group unsubstituted or substituted with a halogen; a biphenyl group; an indolyl unsubstituted or substituted on the nitrogen with a benzyl group;
- X represents an oxygen atom, a sulphur atom, an -NH- group, an -N-CO-Alk group or an -N-Alk group in which Alk is a $C_1$-$C_3$ alkyl group;
- Q represents hydrogen, a $C_1$-$C_4$ alkyl group or an aminoalkyl group of formula $-(CH_2)_q$-Am', where q is 2 or 3 and Am' is a piperidino, 4-benzylpiperidino or dialkylamino group, it being possible for each alkyl to contain 1 to 4 carbon atoms;
- R represents hydrogen, a methyl group or a $(CH_2)_n$-L group, where n is an integer from 2 to 6 and L is hydrogen or an amino group;
- T represents a group chosen amongst

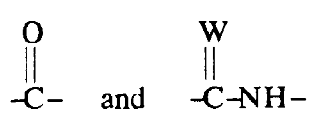
and

W being an oxygen or sulphur atom, and

- Z represents either M or OM when T represents the:

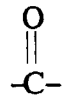 group,

or M when T represents the group

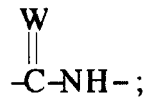

M represents hydrogen or a linear or branched $C_1$-$C_6$ alkyl; an $\alpha$-hydroxybenzyl, an $\alpha$-alkylbenzyl or a phenylalkyl in which the alkyl group contains 1 to 3 carbon atoms, unsubstituted, mono- or polysubstituted on the aromatic ring with a halogen, a hydroxyl, an alkoxy of 1 to 4 carbon atoms, an alkyl of 1 to 4 carbon atoms; a pyridylalkyl in which the alkyl group contains 1 to 3 carbon atoms; a naphthylalkyl in which the alkyl group contains 1 to 3 carbon atoms; a pyridylthioalkyl in which the alkyl group contains 1 to 3 carbon atoms; a styryl; a 1-methyl-2-imidazolylthioalkyl in which the alkyl group contains 1 to 3 carbon atoms; a 1-oxophenyl-3-indan-2-yl; a phenyl group which is unsubstituted or substituted by one or more substituents chosen amongst halogen, CN, OH, $NH_2$, NH-CO-$NH_2$, $NO_2$, $CONH_2$, $CF_3$, $C_1$-$C_{10}$ alkyl, alkenyl containing 2 to 10 carbon atoms, alkynyl containing 2 to 10 carbon atoms, cycloalkyl containing 3 to 8 carbon atoms, bicycloalkyl containing 4 to 11 carbon atoms, hydroxyalkyl containing 1 to 5 carbon atoms, alkoxy containing 1 to 10 carbon atoms, alkoxyalkyl containing 2 to 10 carbon atoms, alkoxyalkoxyalkyl containing 3 to 10 carbon atoms, alkoxyalkoxy containing 2 to 10 carbon atoms, alkenyloxy containing 2 to 10 carbon atoms, alkenyloxyalkyl containing 3 to 10 carbon atoms, alkynyloxy containing 2 to 10 carbon atoms, alkynyloxyalkyl containing 3 to 10 carbon atoms, cycloalkoxy containing 3 to 8 carbon atoms, alkylthio containing 1 to 10 carbon atoms, alkylthioalkyl containing 2 to 10 carbon atoms, acylamino containing 1 to 7 carbon atoms, acylaminoalkyl containing 2 to 8 carbon atoms, acyloxy containing 1 to 6 carbon atoms, alkoxycarbonyl containing 2 to 5 carbon atoms, cycloalkoxycarbonyl containing 4 to 8 carbon atoms, alkylaminocarbonylamino containing 2 to 4 carbon atoms, dialkylaminocarbonylamino containing 3 to 7 carbon atoms, cycloalkylaminocarbonylamino containing 4 to 8 carbon atoms, alkylaminocarbonylaminoalkyl containing 3 to 9 carbon atoms, dialkylaminocarbonylaminoalkyl containing 4 to 11 carbon atoms, cycloalkylaminocarbonylaminoalkyl containing 5 to 12 carbon atoms, alkoxycarbonylaminoalkyl containing 3 to 12 carbon atoms, cycloalkoxycarbonylaminoalkyl containing 5 to 12 carbon atoms, carbamoylalkyl containing 2 to 5 carbon atoms, alkylaminocarbonylalkyl containing 3 to 9 carbon atoms, dialkylaminocarbonylalkyl containing 4 to 11 carbon atoms, (1-pyrrolidono)carbonylmethyl, (1-piperidino)carbonylmethyl, (1-piperidino)carbonylethyl, cycloalkylaminocarbonylalkyl containing 5 to 12 carbon atoms, alkylaminocarbonylalkoxy containing 3 to 10 carbon atoms, dialkylaminocarbonylalkoxy containing 4 to 10 carbon atoms, (piperidinyl-1)carbonylmethoxy, cycloalkylaminocarbonylalkoxy containing 5 to 11 carbon atoms, (1-pyrrolidino)-carbonylamino, (1-piperidino)-carbonylarnino, (1-pyrrolidino)-carbonylaminoethyl, (1-piperidino)-carbonylaminoethyl;

a benzoyl group or a phenylthioalkyl group in which the alkyl is a $C_1$-$C_3$ group, it being possible for said groups to be unsubstituted or mono- or disubstituted with a halogen or with a $C_1$-$C_4$ alkoxy; a naphthyl or indenyl group, in which one or more bonds may be hydrogenated, it being possible for said groups to be unsubstituted or mono- or polysubstituted with a halogen, an alkyl, phenyl, cyano, hydroxyalkyl, hydoxyl, oxo, alkylcarbonylamino, alkoxycarbonyl, thioalkyl group in which the alkyl is a $C_1$-$C_4$ group;

a pyridyl, thiadiazolyl, indolyl, indazolyl, imidazolyl, benzimidazolyl, quinolyl, benzotriazolyl, benzofuranyl, benzothienyl, benzothiazolyl, benzisothiazolyl, isoquinolyl, benzoxazolyl, benzisoxazolyl, benzoxazinyl, benzodioxinyl, isoxazolyl, benzopyranyl, thiazolyl, thienyl, furyl, pyranyl, chromenyl, isobenzofuranyl, pyrrolyl, pyrazolyl, pyrazinyl, pyrimidinyl, pyridazinyl, indolizinyl, phthalazinyl, quinazolinyl, acridinyl, isothiazolyl, isochromanyl and chromanyl group, in which one or more double bonds may be hydrogenated, it being possible for said groups to be unsubstituted or mono- or polysubstituted with an alkyl, phenyl, cyano, hydroxyalkyl, hydroxy, alkylcarbonylamino, alkoxycarbonyl and thioalkyl groups, in which groups the alkyls are $C_1$-$C_4$ groups;

or one of their salts with inorganic or organic acids or one of their quaternary ammonium salts formed with the nitrogen of the piperidine,

characterised in that

a) a free amine of formula:

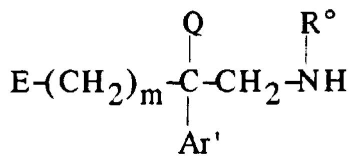

(II)

in which m, Ar' and Q are as defined above; R° represents hydrogen, a methyl group or a $(CH_2)_n$-L° group, where n is as defined above in claim 1 and L° is hydrogen or an amino group protected by an N-protecting group hydrolysable in an acidic medium; and E represents a hydroxy, methanesulphonyloxy, tetrahydro-2-pyranyloxy group, or a group:

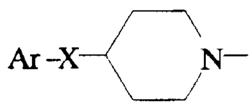

where Ar and X are as defined above, is treated

- either with a functional derivative of an acid of formula:

HO-CO-Z (III)

  in which Z is as defined above, when a compound of formula (I) where T is -CO- is to be prepared,
- or with an iso(thio)cyanate of formula:

W = C = N-Z (III')

  in which W and Z are as defined above, when a compound of formula (I) where T is -C(W)-NH- is to be prepared,
  to form the compound of formula:

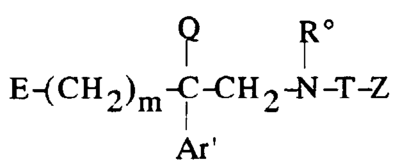

(IV)

b) then, when E represents tetrahydropyranyloxy, the tetrahydropyranyl group is removed by acid hydrolysis, it alternatively being possible for the hydrolysis to take place in step (a) on the starting amine of formula (II),
c) when E is a hydroxy group or a tetrahydropyranyloxy group which has been hydrolyzed, the N-substituted alkanolamine thereby obtained, of formula:

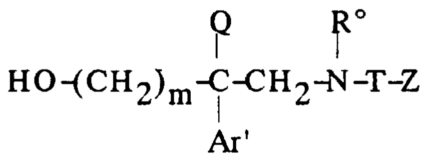

(V)

is treated with methanesulphonyl chloride,

d) the mesylate thereby obtained in step (c), or directly at the end of step (a) when E is a methanesulfonyloxy group, of formula:

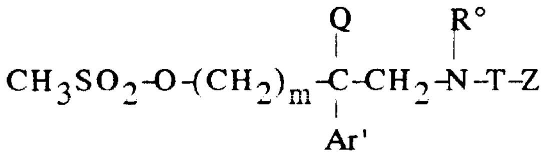

(VI)

is reacted with a secondary amine of formula:

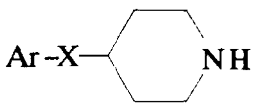

(VII)

in which Ar and X are as defined above,

e) the N-protecting groups, where appropriate, are removed and the product thereby obtained in step (d) or directly at the end of step (a) when E is a group

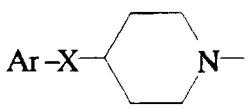

is optionally converted to one of its salts.

**2.** Stereoselective process for the preparation of optically pure compounds of formula (I*)

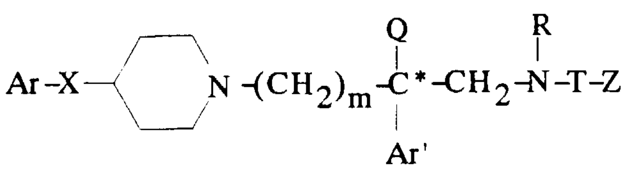

(I*)

in which :

- "*" denotes that the carbon atom thus marked has a determined (+) or (-) absolute configuration;
- m, Ar and Ar', X, Q, R, T and Z are as defined in claim 1;

or one of their salts with inorganic or organic acids or one of their ammonium salts formed with the nitrogen of the piperidine,

characterised in that a compound of formula:

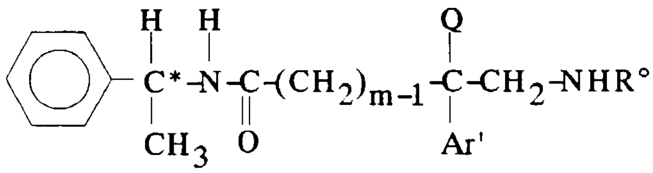 (XVII*)

in which Ar' and R° and m are as defined in claim 1, is treated in a solvent, in acidic medium, to yield the amino acid of formula:

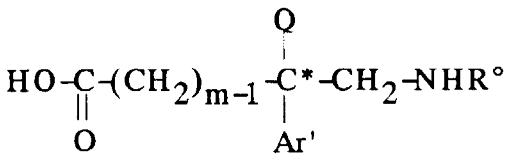 (XVIII*)

which is esterified in an alcohol AlkOH, in which Alk is an alkyl of 1 to 4 carbon atoms, in acidic medium, then the corresponding ester of formula:

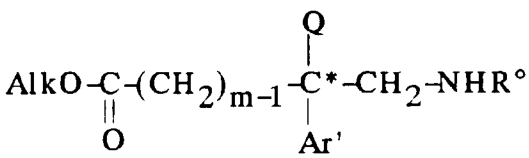 (XIX*)

in which Alk is as defined above, Q, Ar', R° and m are as defined in claim 1, is treated

- either with a functional derivative of an acid of formula:

HO-CO-Z (III)

- or with an iso(thio)cyanate of formula:

W=C=N-Z (III')

Z and W being as defined in claim 1, the ester thus obtained of formula:

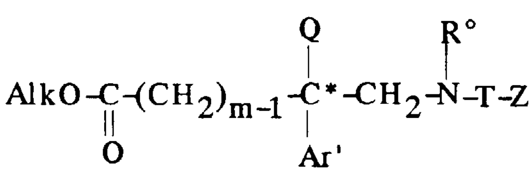 (XX*)

is subjected to the action of a reducing agent and the corresponding alcohol of formula:

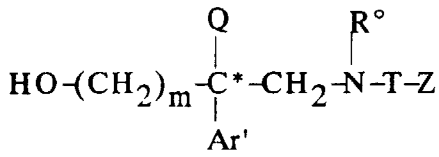 (V*)

is converted into its methanesulphonate ester of formula:

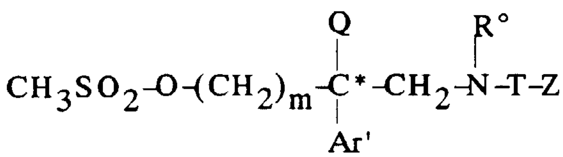 (VI*)

which, by treatment with an amine of formula:

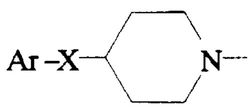 (VII)

in which Ar and X are as defined in claim 1, gives the compound of formula (I*);
and the resulting compound of formula (I*) is optionally converted into one of its salts.

**3.** Process according to one of claims 1 or 2, characterised in that the conversion step of compounds of formula (I) or formula (I*) into their quaternary ammium salts consists in reacting the free bases of compounds (I) or (I*), in which the other amino functions optionally present are N-protected by a customary N-protecting group, with an excess of alkylating agent of formula:

A - Q'

in which A represents a leaving group and is such as defined hereinafter and the reaction mixture is heated in a solvent, for example chosen amongst dichloromethane, chloroform, acetone or acetonitrile, at a temperature between room temperature and reflux for one to several hours to obtain, after treatment according to the customary methods and after deprotection if necessary, a mixture of axial and equatorial conformers of the quaternary ammonium salts of the compounds of formula (I) or formula (I*) in which the group

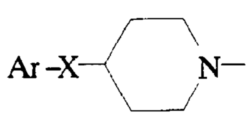

is represented by the group:

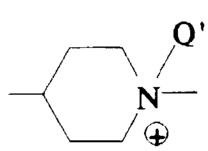 $A^{\ominus}$

in which

- Q' represents a $C_1$-$C_6$ alkyl group or a benzyl group and
- $A^-$ represents an anion chosen amongst chloride, bromide, iodide, acetate, methanesulphonate or paratoluenesulphonate.

**4.** Process according to claim 1, characterised in that a compound of formula (II) in which:

E = THP-O; m = 2;
Ar' = 3,4-diclorophenyl;
R° = $CH_3$ and Q = H;
is reacted with a compound of formula (III) in which Z = phenyl and in that step d) uses an amine of formula (VII) in which Ar-X is the acetylanilino group to form the compound:
N-[4-(N'-4-Acetylanilino-1-piperidinyl)-2-(3,4-dichlorophenyl)-butyl]-N-methylbenzamide or one of its pharmaceutically acceptable salts.

**5.** Process according to claim 1, characterised in that a compound of formula (II) in which:

E = THP-O; m = 2;
Ar' = naphthyl;
R° = H and Q = H;
is reacted with a compound of formula (III) in which Z = 2,4-dimethoxyphenyl and in that step d) uses an amine of formula (VII) in which Ar-X is the 1-methyl-2-imidazolylthio group to form the compound: N-[4-(4-(1-Methyl-2-imidazolylthio)-1-piperidinyl)-2-naphtylbutyl]-2,4-dimethoxybenzamide or one of its pharmaceutically acceptable salts.

**6.** Process according to claim 1, characterised in that a compound of formula (II) in which:

E = THP-O; m = 2;
Ar' = 3,4-dichlorophenyl;
R° = H and Q = H;
is reacted with a compound of formula (III) in which Z = 2,4-dichlorophenyl and in that step d) uses an amine of formula (VII) in which Ar-X is the 2-pyridylthio group to form the compound:
N-[2-(3,4-Dichlorophenyl)-4-(4-(2-pyridylthio)-1-piperidinyl)butyl]-2,4-dichlorobenzamide or one of its pharmaceutically acceptable salts.

**7.** Process for obtaining compounds of formula:

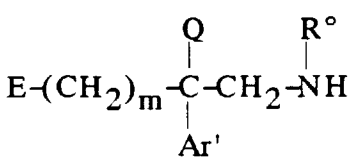

(II)

in which E represents the group of formula:

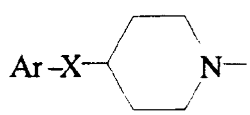

in which m, Q, Ar', Ar and X are as defined in claim 1 and R° represents hydrogen, a methyl group or a $(CH_2)_n$-L° group where n is as defined above in claim 1 and L° is hydrogen or a protected amino group; characterised in that it consists in reducing the nitrile of formula:

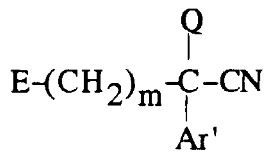

(VIII)

in which m, E, Q and Ar' are as defined above and in optionally sujecting the resulting amine to an alkylation.

**8.** Process according to claim 1 or 3, characterised in that in step a), a compound of formula (II) in which E, m, Q, Ar' and R° are as defined in claim 1 is reacted with a compound of formula (III) or (III') in which Z represents a phenyl group, a benzyl group, a benzoyl group, a phenylthioalkyl group in which the alkyl is a $C_1$-$C_3$ group, said groups being unsubstituted, mono- or disubstituted with a halogen or with a $C_1$-$C_4$ alkoxy, to obtain a compound of formula (I) in which Z is as defined above.

**9.** Stereoselective process according to claim 2 or 3, characterised in that step a) uses a compound of formula (III) or (III') in which Z represents a phenyl group, a benzyl group, a benzoyl group, a phenylthioalkyl group in which the alkyl is a $C_1$-$C_3$ group, said groups being unsubstituted, mono-or disubstituted with a halogen or with a $C_1$-$C_4$ alkoxy, to obtain a compound of formula (I*) in which Z is as defined above.

**10.** Process according to claim 1 or 3, characterised in that the Ar group of the compounds of formula (II) or (VII) represents a phenyl which is mono- or polysubstituted with a chlorine or fluorine atom, and/or the Ar' group of the compound of formula (II) represents a phenyl group which is mono- or disubstituted with a chlorine or fluorine atom, an imidazolyl group or a benzothienyl group substituted with a chlorine or fluorine atom; a naphthyl group substituted with a fluorine atom.

**11.** Stereoselective process according to claim 2 or 3, characterised in that the Ar' group of the compound (XVII*) represents a phenyl group which is mono- or disubstituted with a chlorine or fluorine atom, an imidazolyl group or a benzothienyl group substituted with a chlorine or fluorine atom; a naphthyl group substituted with a fluorine atom and/or the Ar group of the compound (VII) a phenyl which is mono- or polysubstituted with a chlorine or fluorine atom.

**12.** Process for the preparation of a pharmaceutical composition, characterised in that a compound of formula (I) or (I*) prepared by the process according to any one of claims 1 to 6, is mixed as the active principle with a pharmaceutically acceptable vehicle.

**Claims for the following Contracting State : GR**

**1.** Compounds of formula

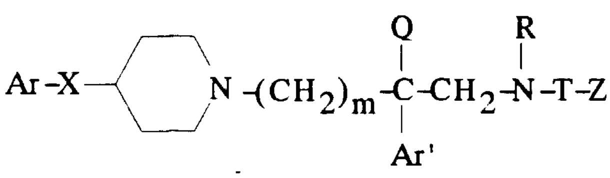

(I)

in which:

- m is equal to 2 or 3;
- Ar represents a phenyl, unsubstituted or substituted one or more times with a halogen atom, with a $C_1$-$C_3$ alkyl, with a trifluoromethyl, with an alkoxy in which the alkyl is a $C_1$-$C_3$ group, with a hydroxyl or with a methylenedioxy; a thienyl, pyridyl or imidazolyl group which is or is not substituted with a $C_1$-$C_3$ alkyl;
- Ar' represents a phenyl group, unsubstituted or mono- or disubstituted with a halogen atom, with a $C_1$-$C_3$ alkyl, with a trifluoromethyl, with an alkoxy in which the alkyl is a $C_1$-$C_3$ group, with a hydroxyl or with a methylenedioxy; a thienyl group; an imidazolyl group or a benzothienyl group, each of which is unsubstituted or substituted with a halogen; a naphthyl group unsubstituted or substituted with a halogen; a biphenyl group; an indolyl unsubstituted or substituted on the nitrogen with a benzyl group;

- X represents an oxygen atom, a sulphur atom, an -NH- group, an -N-CO-Alk group or an -N-Alk group in which Alk is a $C_1$-$C_3$ alkyl group;
- Q represents hydrogen, a $C_1$-$C_4$ alkyl group or an aminoalkyl group of formula $-(CH_2)_q$-Am', where q is 2 or 3 and Am' is a piperidino, 4-benzylpiperidino or dialkylamino group, it being possible for each alkyl to contain 1 to 4 carbon atoms;
- R represents hydrogen, a methyl group or a $(CH_2)_n$-L group, where n is an integer from 2 to 6 and L is hydrogen or an amino group;
- T represents a group chosen amongst

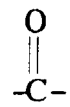 and 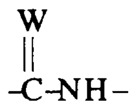

W being an oxygen or sulphur atom, and
- Z represents either M or OM when T represents the:

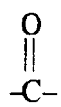 group,

or M when T represents the group

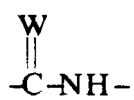 ;

M represents hydrogen or a linear or branched $C_1$-$C_6$ alkyl; an $\alpha$-hydroxybenzyl, an $\alpha$-alkylbenzyl or a phenylalkyl in which the alkyl group contains 1 to 3 carbon atoms, unsubstituted, mono- or polysubstituted on the aromatic ring with a halogen, a hydroxyl, an alkoxy of 1 to 4 carbon atoms, an alkyl of 1 to 4 carbon atoms; a pyridylalkyl in which the alkyl group contains 1 to 3 carbon atoms; a naphthylalkyl in which the alkyl group contains 1 to 3 carbon atoms; a pyridylthioalkyl in which the alkyl group contains 1 to 3 carbon atoms; a styryl; a 1-methyl-2-imidazolylthioalkyl in which the alkyl group contains 1 to 3 carbon atoms; a 1-oxophenyl-3-indan-2-yl; a phenyl group which is unsubstituted or substituted by one or more substituents chosen amongst halogen, CN, OH, $NH_2$, NH-CO-$NH_2$, $NO_2$, $CONH_2$, $CF_3$, $C_1$-$C_{10}$ alkyl, alkenyl containing 2 to 10 carbon atoms, alkynyl containing 2 to 10 carbon atoms, cycloalkyl containing 3 to 8 carbon atoms, bicycloalkyl containing 4 to 11 carbon atoms, hydroxyalkyl containing 1 to 5 carbon atoms, alkoxy containing 1 to 10 carbon atoms, alkoxyalkyl containing 2 to 10 carbon atoms, alkoxyalkoxyalkyl containing 3 to 10 carbon atoms, alkoxyalkoxy containing 2 to 10 carbon atoms, alkenyloxy containing 2 to 10 carbon atoms, alkenyloxyalkyl containing 3 to 10 carbon atoms, alkynyloxy containing 2 to 10 carbon atoms, alkynyloxyalkyl containing 3 to 10 carbon atoms, cycloalkoxy containing 3 to 8 carbon atoms, alkylthio containing 1 to 10 carbon atoms, alkylthioalkyl containing 2 to 10 carbon atoms, acylamino containing 1 to 7 carbon atoms, acylaminoalkyl containing 2 to 8 carbon atoms, acyloxy containing 1 to 6 carbon atoms, alkoxycarbonyl containing 2 to 5 carbon atoms, cycloalkoxycarbonyl containing 4 to 8 carbon atoms, alkylaminocarbonylamino containing 2 to 4 carbon atoms, dialkylaminocarbonylamino containing 3 to 7 carbon atoms, cycloalkylaminocarbonylamino containing 4 to 8 carbon atoms, alkylaminocarbonylaminoalkyl containing 3 to 9 carbon atoms, dialkylaminocarbonylaminoalkyl containing 4 to 11 carbon atoms, cycloalkylaminocarbonylaminoalkyl containing 5 to 12 carbon atoms, alkoxycarbonylaminoalkyl containing 3 to 12 carbon atoms, cycloalkoxycarbonylaminoalkyl containing 5 to 12 carbon atoms, carbamoylalkyl containing 2 to 5 carbon atoms, alkylaminocarbonylalkyl containing 3 to 9 carbon atoms, dialkylaminocarbonylalkyl containing 4 to 11 carbon atoms, (1-pyrrolidono)carbonylmethyl, (1-piperidino)carbonylmethyl, (1-piperidino)carbonylethyl, cycloalkylaminocarbonylalkyl containing 5 to 12 carbon atoms, alkylaminocarbonylalkoxy containing 3 to 10 carbon atoms, dialkylaminocarbonylalkoxy containing 4 to 10 carbon atoms, (piperidinyl-1)carbonylmethoxy, cycloalkylaminocarbonylalkoxy containing 5 to 11 carbon atoms, (1-pyrrolidino)-carbonylamino, (1-piperidino)-carbonylamino, (1-pyrrolidino)-carbonylaminoethyl, (1-piperidino)-carbonylaminoethyl;

a benzoyl group or a phenylthioalkyl group in which the alkyl is a $C_1$-$C_3$ group, it being possible for said groups to be unsubstituted or mono- or disubstituted with a halogen or with a $C_1$-$C_4$ alkoxy; a naphthyl or indenyl group, in which one or more bonds may be hydrogenated, it being possible for said groups to be unsubstituted or mono- or polysubstituted with a halogen, an alkyl, phenyl, cyano, hydroxyalkyl, hydoxyl, oxo, alkylcarbonylamino, alkoxycarbonyl, thioalkyl group in which the alkyl is a $C_1$-$C_4$ group;

a pyridyl, thiadiazolyl, indolyl, indazolyl, imidazolyl, benzimidazolyl, quinolyl, benzotriazolyl, benzofuranyl, benzothienyl, benzothiazolyl, benzisothiazolyl, isoquinolyl, benzoxazolyl, benzisoxazolyl, benzoxazinyl, benzodioxinyl, isoxazolyl, benzopyranyl, thiazolyl, thienyl, furyl, pyranyl, chromenyl, isobenzofuranyl, pyrrolyl, pyrazolyl, pyrazinyl, pyrimidinyl, pyridazinyl, indolizinyl, phthalazinyl, quinazolinyl, acridinyl, isothiazolyl, isochromanyl and chromanyl group, in which one or more double bonds may be hydrogenated, it being possible for said groups to be unsubstituted or mono- or polysubstituted with an alkyl, phenyl, cyano, hydroxyalkyl, hydroxy, alkylcarbonylamino, alkoxycarbonyl and thioalkyl groups, in which groups the alkyls are $C_1$-$C_4$ groups;

or one of their salts with inorganic or organic acids or one of their quaternary ammonium salts formed with the nitrogen of the piperidine.

**2.** Compounds according to claim 1, in the optically pure form, of formula:

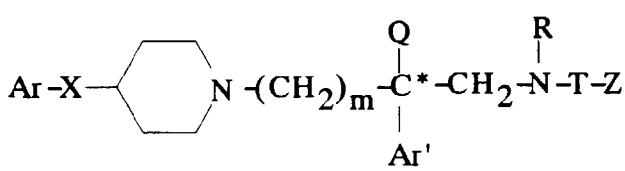

(I*)

in which :

- "*" denotes that the carbon atom thus marked has a determined (+) or (-) absolute configuration;
- m, Ar and Ar', X, Q, R, T and Z are as defined in claim 1.

or one of their salts with inorganic or organic acids or one of their ammonium salts formed with the nitrogen of the piperidine.

**3.** Compounds according to one of claims 1 or 2, characterised in that they are in the form of a quaternary ammium salt, the group of formula

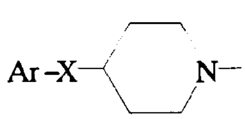

being then represented by the group:

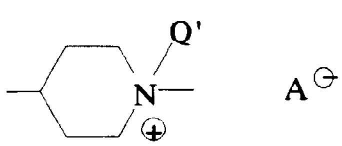

in which

- Q' represents a $C_1$-$C_6$ alkyl group or a benzyl group and
- $A^-$ represents an anion chosen amongst chloride, bromide, iodide, acetate, methanesulphonate or paratoluenesulphonate.

**4.** Compound according to claim 1, which is the N-[2-(3,4-Dichlorophenyl)-4-(4-(2-pyridylthio)-1-piperidinyl)butyl]-2,4-dichlorobenzamide or one of its pharmaceutically acceptable salts.

**5.** Compound according to claim 1, which is the N-[4-(N'-4-Acetylanilino-1-piperidinyl)-2-(3,4-dichlorophenyl)-butyl]-N-methylbenzamide or one of its pharmaceutically acceptable salts.

**6.** Compound according to claim 1, which is the N-[4-(4-(1-Methyl-2-imidazolylthio)-1-piperidinyl)-2-naphtylbutyl]-2,4-dimethoxybenzamide or one of its pharmaceutically acceptable salts.

**7.** Process for the preparation of compounds of formula (I) according to claim 1, characterised in that

a) a free amine of formula:

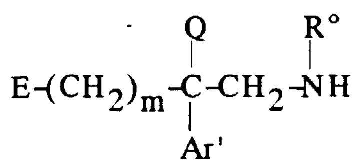

(II)

in which m, Ar' and Q are as defined above in claim 1; R° represents hydrogen, a methyl group or a $(CH_2)_n$-L° group, where n is as defined above in claim 1 and L° is hydrogen or an amino group protected by an N-protecting group hydrolysable in an acidic medium; and E represents a hydroxy, methanesulphonyloxy, tetrahydro-2-pyranyloxy group, or a group:

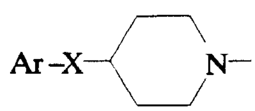

where Ar and X are as defined in claim 1, is treated

- either with a functional derivative of an acid of formula:

$$HO\text{-}CO\text{-}Z \qquad (III)$$

in which Z is as defined in claim 1, when a compound of formula (I) where T is -CO- is to be prepared,
- or with an iso(thio)cyanate of formula:

$$W = C = N\text{-}Z \qquad (III')$$

in which W and Z are as defined in claim 1, when a compound of formula (I) where T is -C(W)-NH- is to be prepared,
to form the compound of formula:

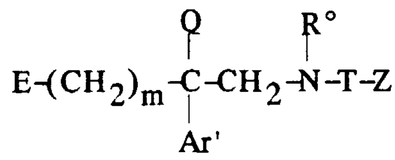

(IV)

b) then, when E represents tetrahydropyranyloxy, the tetrahydropyranyl group is removed by acid hydrolysis, it alternatively being possible for the hydrolysis to take place in step (a) on the starting amine of formula (II),
c) when E is a hydroxy group or a tetrahydropyranyloxy group which has been hydrolyzed, the N-substituted alkanolamine thereby obtained, of formula:

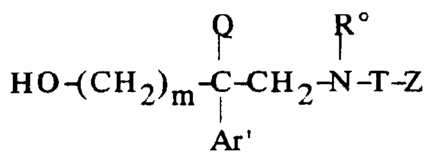

(V)

is treated with methanesulphonyl chloride,
d) the mesylate thereby obtained in step (c), or directly at the end of step (a) when E is a methanesulfonyloxy group, of formula:

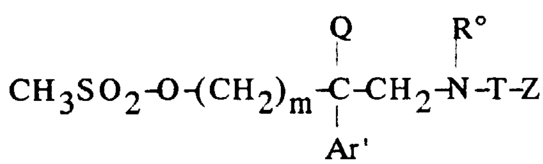

(VI)

is reacted with a secondary amine of formula:

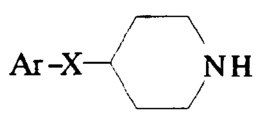

(VII)

in which Ar and X are as defined in claim 1,
e) the N-protecting groups, where appropriate, are removed and the product thereby obtained in step (d) or directly at the end of step (a) when E is a group

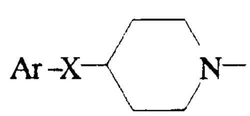

is optionally converted to one of its salts.

**8.** Stereoselective process for the preparation of optically pure compounds of formula (I*) according to claim 2, characterised in that a compound of formula:

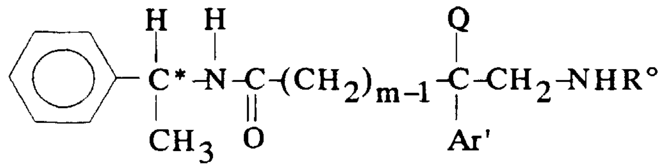 (XVII*)

is treated in a solvent, in acidic medium, to yield the amino acid of formula:

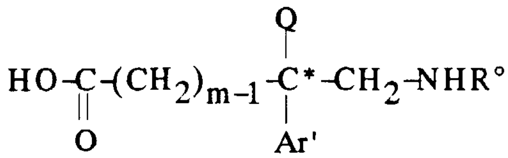 (XVIII*)

which is esterified in an alcohol AlkOH, in which Alk is an alkyl of 1 to 4 carbon atoms, in acidic medium, then the corresponding ester of formula:

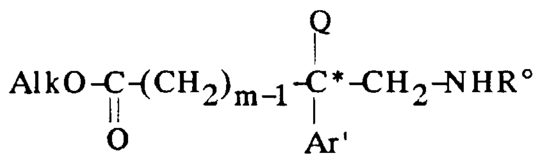 (XIX*)

in which Alk is as defined above; Q, Ar', and m are as defined in claim 1 and R° is as defined in claim 7, is treated

- either with a functional derivative of an acid of formula:

HO-CO-Z (III)

- or with an iso(thio)cyanate of formula:

W=C=N-Z (III')

Z and W being as defined in claim 1, the ester thus obtained of formula:

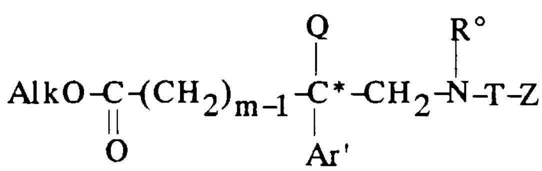 (XX*)

is subjected to the action of a reducing agent and the corresponding alcohol of formula:

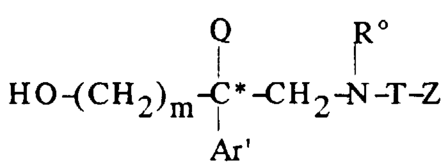 (V*)

is converted into its methanesulphonate ester of formula:

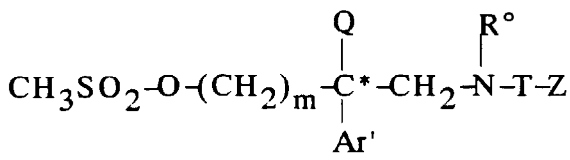

(VI*)

which, by treatment with an amine of formula:

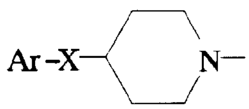

(VII)

in which Ar and X are as defined in claim 1, gives the compound of formula (I*).

**9.** Compound of formula:

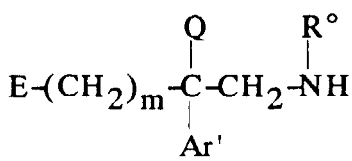

(II)

in which E represents the group of formula:

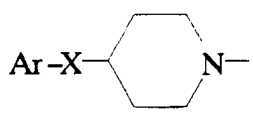

m, Q, Ar', Ar and X are as defined in claim 1 and R° represents hydrogen, a methyl group or a $(CH_2)_n$-L° group where n is as defined in claim 1 and L° is hydrogen or a protected amino group.

**10.** Compounds of formula (I) according to any one of claims 1 to 3, in which Z represents a phenyl group, a benzyl group, a benzoyl group, a phenylthioalkyl group in which the alkyl is a $C_1$-$C_3$ group, said groups being unsubstituted, mono- or disubstituted with a halogen or with a $C_1$-$C_4$ alkoxy.

**11.** Compounds of formula (I) according to any one of claims 1 to 3, in which:

- Ar represents a phenyl which is mono- or polysubstituted with a chlorine or fluorine atom, and/or
- Ar' represents a phenyl group which is mono- or disubstituted with a chlorine or fluorine atom; an imidazolyl group or a benzothienyl group substituted with a chlorine or fluorine atom; a naphthyl group substituted with a fluorine atom.

**12.** Process for the preparation of a pharmaceutical composition, characterised in that a compound of formula (I) or (I*) according to any one of claims 1 to 6, is mixed as the active principie with a pharmaceutically acceptable vehicle.